# FASCICULE DE BREVET EUROPEEN

(11) **EP 2 285 366 B1**
(45) Date de publication et mention de la délivrance du brevet: **25.02.2015**
(21) Numéro de dépôt: 09738328.5
(22) Date de dépôt: 08.04.2009
(51) Int. Cl.: A61K 31/167, A61K 31/18, A61K 31/343, A61K 31/351, A61K 31/4015, A61K 31/4025, A61K 31/4035, A61K 31/404, A61K 31/41, A61K 31/4155, A61K 31/4162, A61K 31/4178, A61K 31/4196, A61K 31/427, A61K 31/435, A61K 31/445, A61K 31/451, A61K 31/4525, A61K 31/4704, A61K 31/495, A61P 37/00

(54) **MOLECULES INHIBANT UNE VOIE METABOLIQUE IMPLIQUANT LA PROTEINE TYROSINE KINASE SYK ET PROCEDE D'IDENTIFICATION DE CES MOLECULES**
MOLEKÜLE ALS HEMMER EINES STOFFWECHSELWEGES UMFASSEND DIE SYK-PROTEINTYROSINKINASE UND VERFAHREN ZUR IDENTIFIZIERUNG DERARTIGER MOLEKÜLE
MOLECULES INHIBITING A METABOLIC PATHWAY INVOLVING THE SYK PROTEIN TYROSINE KINASE AND METHOD FOR IDENTIFYING SAID MOLECULES

(30) Priorité: 09.04.2008 FR 0801959
(43) Date de publication de la demande: 23.02.2011
(73) Titulaire: Centre National de la Recherche Scientifique, 75794 Paris Cedex 16 (FR)
(72) Inventeur: DARIAVACH, Piona, F-34090 Montpellier (FR); MARTINEAU, Pierre, Emile, Ulysse, F-34980 Saint Gely du Fesc (FR); VILLOUTREIX, Bruno, F-75006 Paris (FR)
(74) Mandataire: Almond-Martin, Carol
(86) Numéro de dépôt international: PCT/FR2009/000414
(87) Numéro de publication internationale: WO 2009/133294

(56) Documents cités:
- WO-A-01/09134
- WO-A-03/030897
- WO-A-03/057695
- WO-A-2004/089415
- WO-A-2008/033798
- MAZUC E ET AL: "A novel druglike spleen tyrosine kinase binder prevents anaphylactic shock when administered orally" JOURNAL OF ALLERGY AND CLINICAL IMMUNOLOGY, MOSBY - YEARLY BOOK, INC, US, vol. 122, no. 1, 1 juillet 2008 (2008-07-01), pages 188-194.E3, XP022831406 ISSN: 0091-6749 [extrait le 2008-06-09]

## Description

La présente invention est relative à des molécules organiques capables d'inhiber la liaison d'un anticorps ou fragment d'anticorps avec la protéine tyrosine kinase Syk humaine, à l'utilisation de ces molécules pour la fabrication de médicaments pour la prévention ou le traitement de pathologies dépendantes de voies métaboliques impliquant Syk, ainsi qu'à un procédé d'identification de telles molécules.

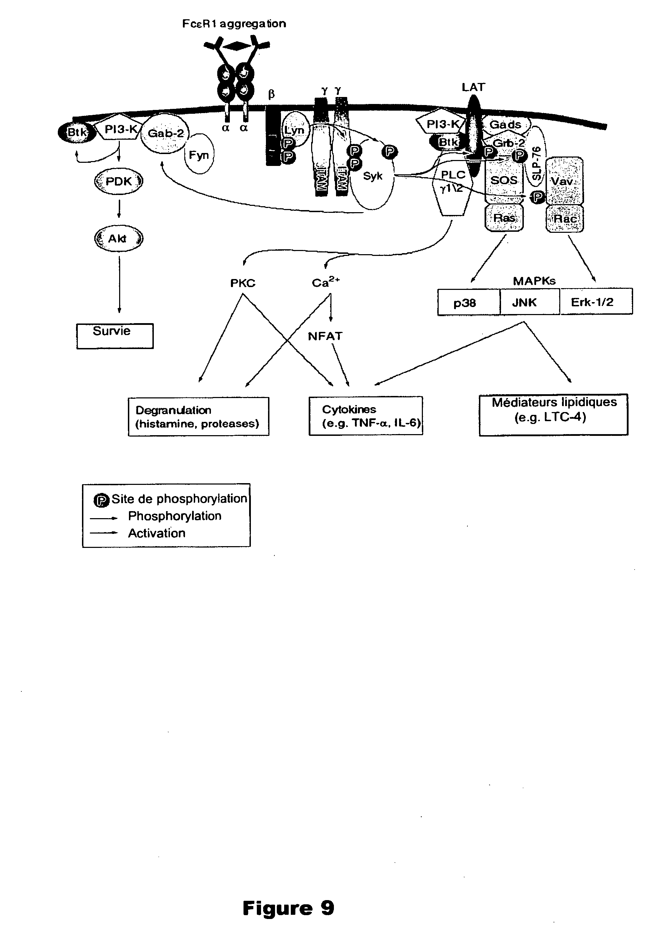

La Protéine Tyrosine Kinase (PKT) Syk (« Spleen tyrosine kinase ») est une protéine cytoplasmique qui est un médiateur clé de la signalisation dépendante d'immunorécepteurs dans des cellules impliquées dans l'inflammation telles que les lymphocytes B, les mastocytes, les macrophages et les neutrophiles. Au niveau des mastocytes et des basophiles, la réticulation du récepteur FcεRI (récepteur à haute affinité des Immunoglobulines E) avec les IgE et les antigènes induit la phosphorylation des motifs ITAM (« Immunoreceptor Tyrosine-based Activation Motif ») de FcεRI de manière à former un site de liaison pour Syk qui est alors activé. La protéine Syk activé phosphoryle à son tour de nombreux substrats, parmi lesquels les protéines adaptatrices LAT (« linker for activation of T cells »), SLP-76 (« Src homology 2 (SH2) domain-containing leukocyte protein of 76 kD ») et Vav, conduisant à l'activation de plusieurs cascades de signalisation, telles que celles de PLC-γ (phospholipase Cγ), PI3K (« phosphatidylinositol 3-kinase »), Erk (« extracellular signal-regulated kinase »), JNK (« c-jun N-terminal kinase ») et p38 (voir figure 9). Ces cascades mènent finalement à la dégranulation, la synthèse et la libération de médiateurs lipidiques, et à la production et à la sécrétion de cytokines, chimiokines et facteurs de croissance par les mastocytes et les basophiles^{1,2}.

La protéine Syk est par conséquent reconnue comme une cible pharmaceutique potentielle, notamment pour le traitement de réactions d'hypersensibilité de type I incluant la rhinite allergique, l'urticaire, l'asthme et l'anaphylaxie en raison de sa position critique en amont des complexes immuno-récepteurs de signalisation qui régulent la réponse inflammatoire dans les leucocytes. Le fait que la protéine Syk régule positivement la signalisation FcεRI³, suggère en particulier qu'elle pourrait être une excellente cible pour le traitement des troubles allergiques. De plus, en raison de son rôle central dans la signalisation FcεRI-dépendante, le fait d'interagir pharmaceutiquement avec Syk pourrait s'avérer plus avantageux que l'utilisation classique d'antihistaminiques ou d'agonistes du récepteur du leucotriène qui inhibent une seule étape avale des cascades complexes qui contribuent aux symptômes aigus et chroniques associés avec les affections allergiques.

Des inhibiteurs pharmacologiques de l'activité kinase de Syk présentant un potentiel thérapeutique, tels que notamment des oligonucléotides anti-sens spécifiques de Syk sous forme d'aérosol et des petites molécules qui interfèrent avec l'activité de Syk telles que ER-27139, BAY-613606, le picéatannol et R112 ont déjà été développés^{1,4}.

Les publications de demande de brevet international WO2008/033798, WO03/057695 et WO01/09134 décrivent également différents composés hétérocycliques utilisés comme inhibiteur de la protéine Syk.

De plus, la publication de demande de brevet international WO2004/089415 décrit l'utilisation d'inhibiteurs de 11β-hydroxystéroide déshydrogenase de type 1 (HSD-1) en association avec un agoniste de récepteur aux glucocorticoïdes, pour le traitement de différents troubles inflammatoires. Parmi les inhibiteurs de HSD-1, WO2004/089415 décrit divers sulfonamides et dérivés de triazole. Cependant, ces composés sont toujours administrés en association avec un agoniste de récepteur aux glucocorticoïdes, et leur effet sur la protéine Syk n'est pas divulgué.

Cependant, si l'on considère les multiples types de cellules qui expriment Syk, des effets secondaires potentiels liés à une exposition systémique du système immunitaire à des médicaments ciblant le domaine kinase de Syk doivent être pris en considération. En effet, la protéine Syk est largement distribuée dans différents types cellulaires, il est par conséquent essentiel de prendre en considération les effets indésirables de son inhibition sur des fonctions physiologiques variées telles que la différenciation, l'adhésion et la prolifération cellulaire^{5,6}.

Les inventeurs ont identifié des inhibiteurs de la protéine Syk qui agissent en empêchant son interaction avec ses partenaires cellulaires naturels plutôt qu'en ciblant son site catalytique, en particulier le composé C-13 et les composés 1 à 87 présentés dans le tableau 1.

Un objet de la présente invention est la molécule C-13 (méthyl 2-{5-[(3-benzyl-4-oxo-2-thioxo-1,3-thiazolidin-5-ylidene)méthyl]-2-furyl}benzoate) ayant pour formule 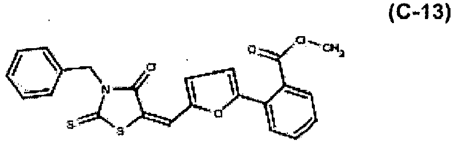 en tant que médicament pour la prévention ou le traitement d'une pathologie dépendante d'une voie métabolique impliquant Syk chez l'homme ou l'animal.

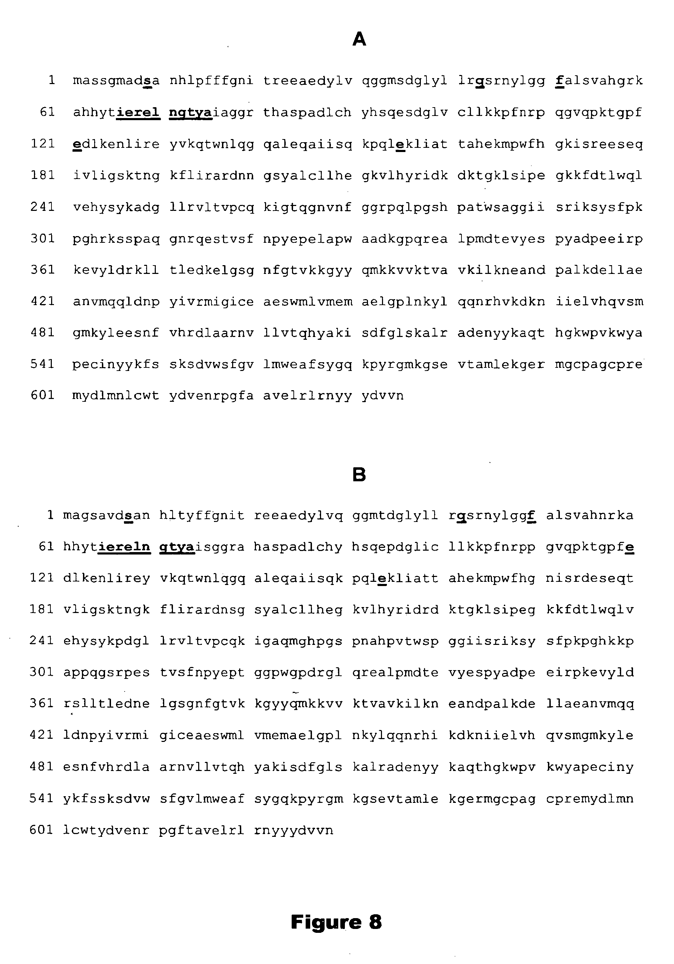

La présente invention a également pour objet des molécules organiques équivalentes sur le plan fonctionnel à la molécule C-13, choisies parmi les formules (I) à (IV) et les molécules n°1 à 87 du tableau 1, se liant à la protéine tyrosine kinase Syk et en particulier des molécules capable d'inhiber d'au moins 5%, de préférence d'au moins 10%, par exemple d'au moins 15, 20, 25, 30, 35. 40, 45, 50, 55, 60, 65, 70, 75, 80 ou 85 % *in vitro* la liaison
(i) du fragment d'anticorps G4G11 (SEQ ID N°2), ou
(ii) du fragment d'anticorps G4E4 (SEQ ID N°3), ou
(iii) d'un anticorps ou fragment d'anticorps se liant à la protéine tyrosine kinase Syk humaine au niveau d'un épitope comprenant au moins l'un des résidus 65 à 74 de la séquence en acides aminés de la protéine tyrosine kinase Syk humaine représentée par la séquence SEQ ID N°1, ou
(iv) d'un anticorps ou fragment d'anticorps se liant à la protéine tyrosine kinase Syk humaine et inhibant d'au moins 10% la liaison des fragments d'anticorps G4G11 ou G4E4 avec la protéine tyrosine kinase Syk humaine (SEQ ID N°1)
à la protéine tyrosine kinase Syk humaine ou à l'une de ses variantes chez l'animal, par exemple la protéine tyrosine kinase Syk murine dont la séquence est illustrée à la figure 8B (SEQ ID N°4), en tant que médicament pour la prévention ou le traitement d'une pathologie dépendante d'une voie métabolique impliquant Syk chez l'homme ou l'animal.

**Par « molécule équivalente sur le plan fonctionnel** » on entend une molécule, par exemple une molécule organique de poids moléculaire compris entre 50 et 2500 Da, capable de conduire au même effet *in vitro* en milieu intra ou extra cellulaire ou *in vivo,* éventuellement avec une intensité différente, qu'une molécule donnée. En particulier dans le cadre de la présente invention, on entend par « molécule équivalente sur le plan fonctionnel à la molécule C-13 », une molécule capable de produire le même effet *in vitro* en milieu intra ou extra cellulaire ou *in vivo,* éventuellement avec une intensité différente, sur la protéine tyrosine kinase humaine telle que représentée par séquence SEQ ID N°1 (figure 8A) que la molécule C-13. En particulier il s'agit de molécules inhibant la liaison de Syk avec une autre protéine se produisant au niveau de la région de Syk comprenant ses domaines SH2. Plus particulièrement encore ces molécules n'affectent pas l'activité enzymatique kinase de Syk. Il s'agit par exemple de molécules capables d'inhiber l'interaction de la protéine tyrosine kinase Syk avec le fragment d'anticorps G4G11 (SEQ ID N°2), le fragment d'anticorps G4E4 (SEQ ID N°3), ou un anticorps ou fragment d'anticorps se liant au même épitope que le fragment d'anticorps G4G11 ou G4E4 sur la protéine Syk humaine (SEQ ID N°1).

Par « **pourcentage d'inhibition** » de la liaison d'un anticorps ou fragment d'anticorps à la protéine Syk, on entend en particulier le ratio [(A - B) / (A x 100)], où A correspond à l'intensité d'un signal proportionnel à la quantité d'un anticorps ou d'un fragment d'anticorps lié à la protéine Syk en l'absence d'une molécule selon l'invention et B à l'intensité du même signal en présence d'une molécule selon l'invention dans les mêmes conditions. L'inhibition de la liaison d'un anticorps ou fragment d'anticorps à la protéine Syk peut notamment être mise en évidence *in vitro* par un essai de déplacement de l'anticorps basé sur la technique ELISA tel que décrit par exemple dans la demande internationale WO 2005106481. Cet essai peut être réalisé par exemple selon le protocole décrit dans l'exemple 3-2) ci-après.

Par « **variantes de Syk chez l'animal** » on entend des gènes de différentes espèces animales, par exemple de la souris, du rat, du chien, du chat ou d'un autre mammifère, codant pour une protéine présentant une forte homologie ou identité de séquence et de fonction avec la protéine Syk humaine telle que représentée par la séquence SEQ ID N°1 (voir figure 8A), par exemple une protéine présentant une homologie ou une identité de séquence d'au moins 70, 75, 80, 85, 90 ou 95% avec la séquence SEO ID N°1 de la protéine Syk humaine, présentant la même activité tyrosine kinase et impliquée dans les mêmes cascades fonctionnelles que cette dernière, en particulier dans la cascade fonctionnelle conduisant à la dégranulation des mastocytes. Il peut notamment s'agir de gènes orthologues c'est-à-dire de gènes présents dans des organismes différents, ayant évolué à partir d'un même gène ancestral suite à des événements de spéciation. La présente invention a également pour objet les sels pharmaceutiquement acceptables, et le cas échéant, les stéréoisomères et racémates de C-13 ou des molécules équivalentes selon l'invention choisies parmi les formules (I) à (IV) et les molécules n°1 à 87 du tableau 1.

Par « sels pharmaceutiquement acceptables » on entend les sels d'addition acides ou de bases, inorganiques et organiques, relativement non toxiques qui conservent l'activité biologique des molécules selon l'invention. Des exemples de sels pharmaceutiquement acceptables sont notamment décrits dans S. M. Berge et al., « Pharmaceutical Salts », J. Pharm. Sci, 1977, 66:p.1-1940. Les sels d'addition pharmaceutiquement acceptables de molécules selon l'invention peuvent être par exemple des sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, méthanesulfonates, éthane-sulfonates, benzènesulfonates, p-toluènesulfonates, cyclohexyl sulfamates et quinateslaurylsulfonate, et analogues. D'autres sels pharmaceutiquement acceptables pouvant être appropriés incluent des sels de métaux, par exemple les sels pharmaceutiquement acceptables de métaux alcalins ou alcalino-terreux, tels que les sels de sodium, potassium, calcium ou magnésium.

Ces sels pharmaceutiquement acceptables peuvent être préparés *in situ* pendant l'isolement final et la purification des molécules. Alternativement, les sels d'addition acides ou de base peuvent être préparés en faisant réagir séparément la molécule purifiée sous sa forme acide ou basique avec respectivement une base ou un acide organique ou inorganique et en isolant le sel ainsi formé. Par exemple, un sel d'addition acide pharmaceutiquement acceptable peut être préparé en faisant réagir une molécule selon l'invention avec un acide organique ou inorganique convenable (tel que par exemple l'acide hydrobromique, hydrochlorique, sulfurique, nitrique, phosphorique, succinique, maléique, formique, acétique, propionique, fumarique, citrique, tartarique, lactique, benzoïque, salicylique, glutamique, aspartique, p-toluène-sulfonique, benzène-sulfonique, méthane-sulfonique, éthane-sulfonique, hexanoïque ou naphthalène-sulfonique tel que l'acide 2-naphthalène sulfonique), éventuellement dans un solvant approprié tel qu'un solvant organique. Un sel d'addition de base peut, lorsqu'un groupement acide approprié est présent, être préparé en faisant réagir une molécule selon l'invention avec une base organique ou inorganique appropriée (par exemple le triéthyl-amine, l'éthanol-amine, le triéthanol-amine, la choline, l'arginine, la lysine ou l'histidine), éventuellement dans un solvant approprié tel qu'un solvant organique. Les sels ainsi générés peuvent ensuite être isolés par cristallisation et filtration.

Dans certaines mises en oeuvre, les sels pharmaceutiquement acceptables sont préférés dans la mesure où ils confèrent une meilleure stabilité ou solubilité aux molécules selon l'invention facilitant leur formulation.

Selon une mise en oeuvre particulièrement préférée, les molécules organiques selon l'invention se lient à la protéine tyrosine kinase Syk au niveau d'un site localisé en dehors de son domaine catalytique.

De préférence également, les molécules organiques selon l'invention ont un poids moléculaire compris entre 50 et 2500 Dalton, par exemple entre 50 et 2000 Da, entre 50 et 1500 Da ou entre 50 et 1000 Da.

Selon une mise en oeuvre particulière, les molécules selon l'invention sont capables d'inhiber d'au moins 5%, de préférence d'au moins 10%, la liaison d'un anticorps ou fragment d'anticorps se liant à la protéine tyrosine kinase Syk humaine au niveau d'un épitope comprenant au moins deux, de préférence au moins 3, 4 ou 5, par exemple 5, 6, 7, 8, 9 ou 10 des résidus 65 à 74 de la séquence en acides aminés de la protéine tyrosine kinase Syk humaine (SEQ ID N°1), à la protéine tyrosine kinase Syk humaine. Selon une mise en oeuvre préférée, l'anticorps ou fragment d'anticorps se lie à la protéine tyrosine kinase Syk humaine au niveau du même épitope que le fragment d'anticorps G4G11 ou que le fragment d'anticorps G4E4 sur la protéine tyrosine kinase Syk humaine dont la séquence est illustrée par la SEQ ID N°1.

Selon une autre mise en oeuvre particulière, les molécules selon l'invention sont capables d'inhiber d'au moins 5%, de préférence d'au moins 10%, la liaison d'un anticorps ou fragment d'anticorps se liant à la protéine tyrosine kinase Syk et inhibant d'au moins 15%, de préférence d'au moins 20, 30, 40, 50, 60, 70 ou 80 % la liaison des fragments d'anticorps G4G11 ou G4E4 avec la protéine tyrosine kinase Syk humaine (SEQ ID N°1), à la protéine tyrosine kinase Syk humaine.

De préférence, les molécules selon l'invention se lient à la protéine Syk humaine au niveau d'une poche tridimensionnelle comprenant le résidu Arginine situé en position 68 et les deux résidus acide glutamique situés en positions 121 et 155 de la protéine Syk dont la séquence est illustrée par la SEQ ID N° 1. De préférence encore, la poche tridimensionnelle comprend également le résidu Sérine situé en position 9, le résidu Glutamine situé en position 43, le résidu Phénylalanine situé en position 51, le résidu Isoleucine situé en position 66, les résidus Glutamate situés en position 67 et 69, le résidu Leucine situé en position 70, le résidu Asparagine situé en position 71, le résidu Glycine situé en position 72, le résidu Thréonine situé en position 73, le résidu Tyrosine situé en position 74 et le résidu Alanine situé en position 75 de la protéine Syk humaine dont la séquence est illustrée par la SEQ ID N°1.

De préférence encore, l'affinité *in vitro,* mesurée par la constante de dissociation (ou Kd), des molécules selon l'invention pour la protéine Syk, est inférieure à 100 µM, de préférence encore, inférieure à 50 µM, et de manière particulièrement préférée, inférieure à 25 µM. L'affinité des molécules selon l'invention pour la protéine Syk est par exemple comprise entre 0,01 et 100 µM, entre 0,1 et 50 µM ou entre 0,5 et 25 µM. La constante de dissociation des molécules selon l'invention vis-à-vis de la protéine Syk peut notamment être mesurée *in vitro* par spectroscopie de fluorescence (ou spectroflurométrie).

La présente invention a également pour objet l'utilisation d'une molécule selon l'invention choisies parmi celles de la revendication 1 pour la fabrication d'un médicament pour la prévention ou le traitement d'une pathologie dépendante d'une voie métabolique impliquant Syk chez l'homme ou l'animal, choisies parmi les maladies citées à la revendication 1.

Selon une mise en oeuvre particulièrement préférée, les molécules ou sels selon l'invention sont utilisées pour la fabrication de médicament pour la prévention ou le traitement de réactions d'hypersensibilité de type I.

Par « **hypersensibilité** » on entend une réponse immunitaire inappropriée et excessive à un allergène, par exemple le pollen, la poussière, des poils d'animaux ou certains aliments, les effets allant de la réaction allergique modérée (éruption cutanée, rhinite, conjonctivite...) à des réactions systémiques sévères pouvant mener au choc anaphylactique et pouvant dans certains cas être fatales. Les réactions d'hypersensibilité à effet immédiat et retardé sont classifiées dans les types I et IV respectivement de la classification établie par Gell et Coombs (Gell PGH, Coombs RRA, eds.

Clinical Aspects of Immunology. 1st ed. Oxford, England: Blackwell; 1963³⁹). Selon cette classification l' « **hypersensibilité de type I** » **(ou « atopique** » **ou « anaphylactique »)** est une réaction allergique immédiate liée à l'exposition à un antigène spécifique ou allergène, par exemple par ingestion, inhalation, injection ou contact direct, et au déclenchement de la sécrétion d'Immunoglobulines E (IgE) par les cellules plasmatiques. Les IgE se lient aux récepteurs Fc présent à la surface des mastocytes tissulaires et des basophiles sanguins. Une exposition ultérieure des mastocytes et basophiles ainsi sensibilisés au même allergène conduit à la dégranulation des cellules présentant l'IgE correspondante et à la libération de médiateurs tels que l'histamine, le leukotriène ou les prostaglandines qui agissent sur les tissus environnant, conduisant en particulier à une vasodilatation et à la contraction des muscles lisses. Les réactions peuvent être locales ou systémiques et les symptômes varient de l'irritation modérée à la mort soudaine par choc anaphylactique. A titre d'exemple de pathologies causées par l'hypersensibilité de type I, on peut citer l'asthme allergique, la conjonctivite allergique, la rhinite allergique (ou rhume des foins), l'anaphylaxie, l'angioedème, l'urticaire, l'éosinophilie, des allergies aux antibiotiques tels que la pénicilline ou la céphalosporine. L' « hypersensibilité de type II » ou « réponse immunitaire dépendante des anticorps » est une réaction nécessitant généralement de quelques heures à une journée, liée aux interactions entre des anticorps (IgG, IgM) et un antigène au niveau de la surface des cellules du patient présentant cet antigène, conduisant à la destruction de ces cellules et à la prolifération des lymphocytes B produisant les anticorps contre l'antigène. L' « hypersensibilité de type III » ou « maladie du complexe immun » est une réaction se développant sur plusieurs heures, jours ou semaines, liée à la présence en quantités similaires d'anticorps et d'antigènes conduisant à la formation de complexes immuns ne pouvant être évacués circulant dans les vaisseaux, à leur dépôt au niveau des parois de ces derniers et conduisant à des réponses inflammatoires locales ou générales. L'« hypersensibilité de type IV » ou « immunité à médiation cellulaire » ou « réaction d'hypersensibilité retardée » est une réaction immunitaire nécessitant généralement deux à trois jours pour se développer et non liée à une réponse anticorps mais à la formation d'un complexe entre des cellules présentant un antigène du complexe majeur d'histocompatibilité I ou II et des lymphocytes T conduisant à la libération de lymphokines et/ou à la cytotoxicité médiée par les lymphocytes T.

De préférence, les molécules ou sels selon l'invention sont utilisées pour la fabrication de médicaments pour la prévention ou le traitement de réactions d'hypersensibilité de type I qui inhibent la dégranulation des mastocytes dépendante des IgE. De préférence encore, les molécules selon l'invention sont capables d'inhiber de 50% *in vitro* la dégranulation des mastocytes à une concentration (CI50) comprise entre 1ng/ml et 1 mg/ml, par exemple à une concentration comprise entre 1 ng/ml et 500 µg/ml, entre 1 ng/ml et 250 µg/ml, entre 1 ng/ml et 100 µg/ml, entre 1 ng/ml et 50 µg/ml, entre 1 ng/ml et 10 µg/ml, entre 1 ng/ml et 5 µg/ml ou entre 1 ng/ml et 2 µg/ml. De préférence également, une quantité comprise entre 1 nM et 1 mM, par exemple entre 1 nM et 100 nM, entre 10 nM et 100 nM ou entre 1 nM et 10 nM, d'une molécule selon l'invention est capable d'inhiber de 50% *in vitro* la dégranulation des mastocytes.

De préférence encore, la voie métabolique impliquant Syk sur laquelle agissent les molécules ou sels selon l'invention est une voie d'activation des mastocytes ou des basophiles.

De préférence encore, la pathologie sur laquelle agissent les molécules ou sels selon l'invention est l'asthme allergique, la conjonctivite allergique, la rhinite allergique, l'anaphylaxie, l'angioedème, l'urticaire, l'éosinophilie ou une allergie à un antibiotique.

Selon une mise en oeuvre préférée, les molécules ou sels selon l'invention n'ont pas d'effet sur les voies métaboliques impliquant la protéine Syk humaine (SEQ ID N° 1) autres que celles conduisant à la dégranulation des mastocytes et/ou aux réactions d'hypersensibilité de type I. De préférence encore, les molécules ou sels selon l'invention n'ont pas d'effet sur la réponse anticorps faisant suite à une immunisation par un antigène thymo-dépendant ou sur le recrutement Syk-dépendant des neutrophiles.

La protéine tyrosine kinase Syk est également présente à la surface de lymphocytes B, lymphocytes T, neutrophiles, éosinophiles, cellules NK, plaquettes, érythrocytes, ostéoclastes, cellules épithéliales ou de cellules cancéreuses. Selon une mise en oeuvre alternative, la voie métabolique impliquant Syk sur laquelle agissent les molécules ou sels selon l'invention est une voie d'activation des lymphocytes B, des lymphocytes T, des neutrophiles, des éosinophiles, des cellules NK, des plaquettes, des érythrocytes, des ostéoclastes, des cellules épithéliales ou de cellules cancéreuses. Selon cette mise en oeuvre, la pathologie sur laquelle agissent les molécules ou sels selon l'invention peut alors être la polyarthrite rhumatoïde, une maladie auto-immune, une inflammation ou un cancer.

Selon une mise en oeuvre particulière, les molécules ou sels selon l'invention peuvent être utilisés en combinaison avec une autre molécule thérapeutique. Il peut s'agir par exemple d'une molécule thérapeutique également utilisée pour la prévention ou le traitement d'une pathologie dépendante d'une voie métabolique impliquant Syk ou au contraire d'une molécule thérapeutique utilisée pour la prévention ou le traitement d'une pathologie non dépendante d'une voie métabolique impliquant Syk. Selon une mise en oeuvre préférée, les molécules ou sels selon l'invention sont utilisés en combinaison avec une molécule utilisée pour le traitement de l'allergie ou de l'hypersensibilité de type I ou pour le traitement des symptômes y étant associés. De préférence encore, les molécules ou sels selon l'invention sont utilisés en combinaison avec l'épinéphrine (ou adrénaline), un antihistaminique H1, par exemple diphénhydramine, méclizine, fluphénazine, perphénazine, prochlorpérazine, trifluopérazine, acrivastine, astémizole, cétirizine, lévocétirizine, fexofénadine, loratadine, desloratadine, mizolastine, azélastine, lévocabastine, olopatadine, cromoglicate, nédocromil, un anti-inflammatoire non stéroïdien (AINS) ou un anti-inflammatoire stéroïdien, par exemple cortisone, hydrocortisone (ou cortisol), acétate de cortisone, prednisone, prednisolone, méthylprednisolone, dexaméthasone, bétaméthasone, triamcinolone, béclométasone, fludrocortisone, acétate de désoxycorticostérone ou aldostérone. Selon une autre mise en oeuvre préférée, les molécules ou sels selon l'invention sont utilisés en combinaison avec un traitement de désensibilisation allergique (ou vaccination anti-allergique), c'est-à-dire un traitement basé sur l'administration régulière à doses croissantes d'un allergène. Selon une mise en oeuvre particulière, la molécule selon l'invention n'est pas utilisée en combinaison avec un agoniste d'un récepteur de glucocorticoïde.

Les molécules ou sels selon l'invention peuvent être administrées par n'importe quelle voie d'administration, en particulier par les voies orale, sublinguale, nasale, oculaire, locale, intraveineuse, intrapéritonéale, sous-cutanée, par aérosol ou par inhalation.

Les molécules ou sels selon l'invention peuvent notamment être administrées à des patients humains adultes, enfants ou nouveau-nés. Les molécules ou sels selon l'invention peuvent également être administrées à des patients animaux, en particulier à des mammifères tels que le chien, le chat, le rat, la souris.

En particulier, les molécules ou sels selon l'invention sont administrées à un patient humain à des doses qui sont déterminées notamment en fonction de la pathologie du patient, de ses antécédents et de son âge, par exemple des doses comprises entre 0,1 mg/kg et 200mg/kg.

La présente invention a également pour objet une méthode thérapeutique pour le traitement ou la prévention d'une pathologie dépendante d'une voie métabolique impliquant Syk chez un patient humain ou animal comprenant l'administration d'une molécule selon l'invention au patient à des doses, intervalles et durées déterminées notamment en fonction de la pathologie du patient, de ses antécédents et de son âge.

Les molécules selon l'invention sont choisies parmi l'ensemble de molécules constitué par C-13, les molécules N°1 à 87 présentées dans le tableau N°1 et les molécules ayant l'une des formules (I), (II), (III) ou (IV) suivantes : 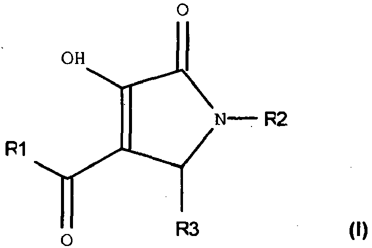 où
- R1 est un groupement aromatique substitué ou non, ou un hétérocycle substitué ou non comprenant au moins un atome S, O ou N ;
- R2 est un groupement aromatique substitué ou non, un hétérocycle substitué ou non, une chaîne carbonée saturée ou non, comprenant un groupement amine, une chaîne carbonée saturée ou non comprenant un groupement aromatique substitué ou non ou une chaîne carbonée saturée ou non comprenant un hétérocycle substitué ou non comprenant au moins un atome S, O ou N ;
- R3 est un groupement phényle, 2-pyridinyle, 3-pyridinyle ou 4-pyridinyle, substitué ou non ; 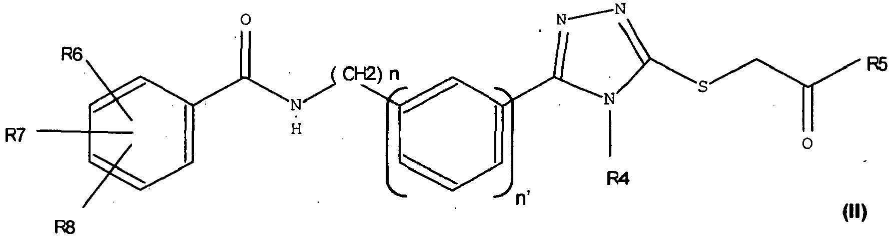 où
- n = 0 ou 1 ; n'= 0 ou 1 ;
- R4 est une chaîne carbonée saturée ou non comprenant 1 à 5 atomes de carbone, substituée ou non par un groupement aromatique ;
- R5 est un groupement aromatique substitué ou non ou un groupement amine substitué ou non ;
- R6 est un atome d'hydrogène, un groupement alkoxy, un groupement alkyle ou un halogène ;
- R7 est un atome d'hydrogène, un groupement alkoxy, un groupement alkyle ou un halogène ;
- R8 est un atome d'hydrogène, un groupement alkoxy, un groupement alkyle ou un halogène ; 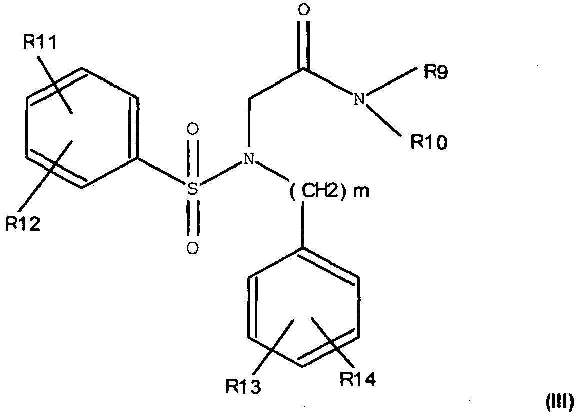 où
- m = 0, 1 ou 2;
- R9 est un atome d'hydrogène et R10 est un groupement phényle substitué ou non, ou R9 et R10 font partie d'un méme hétérocycle substitué ou non, ou R9 et R10 font partie d'un même groupement aromatique substitué ou non;
- R11 est un atome hydrogène, un groupement alkoxy ou un groupement alkyle ;
- R12 est un atome hydrogène, un groupement alkoxy ou un groupement alkyle ;
- R13 est un atome hydrogène ou un groupement alkyle ou alkoxy;
- R14 est un atome hydrogène ou un groupement alkyle ou alkoxy ; 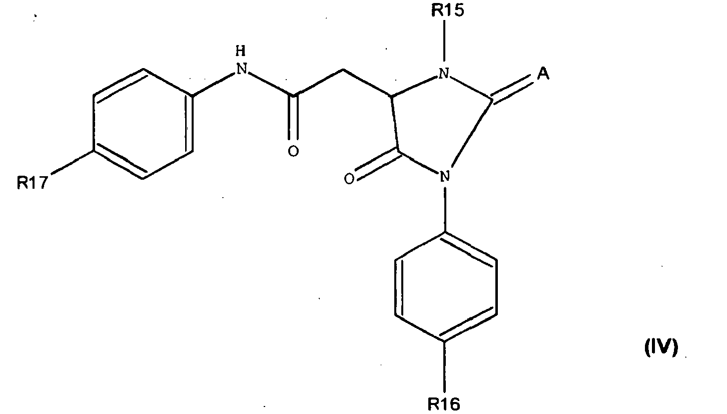 ou
- A est un atome d'oxygène ou de Soufre ;
- R15 est une chaîne carbonée saturée ou non comprenant 1, 2 ou 3 atomes de carbone, substituée ou non par un groupement aromatique substitué ou non, par un hétérocycle substitué ou non ou par un groupement amine faisant partie d'un hétérocycle substitué ou non ;
- R16 est un atome d'hydrogène, un halogène ou un groupement alkoxy ;
- R17 est un atome d'hydrogène, un groupement alkoxy ou un groupement acétoxy
où si ladite molécule est sélectionnée parmi les molécules de formules suivantes 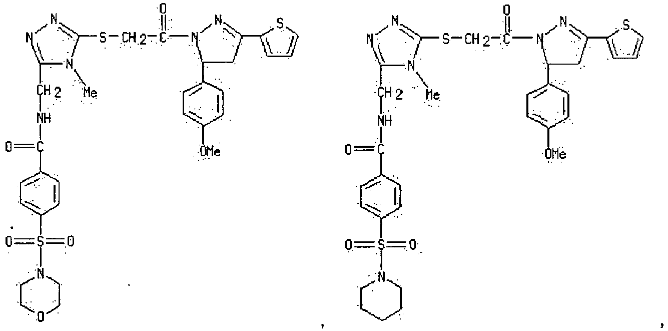 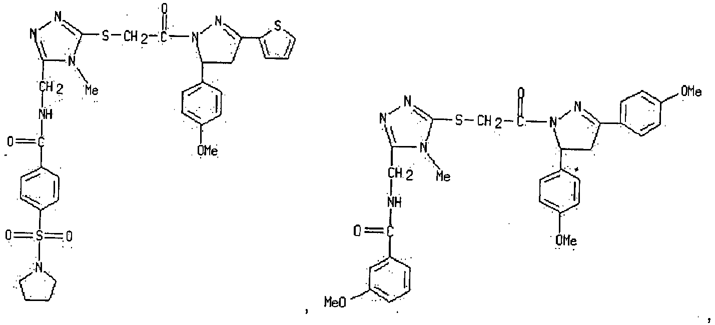 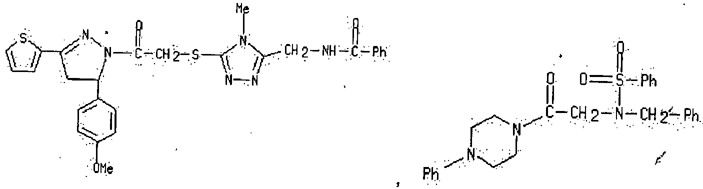 et 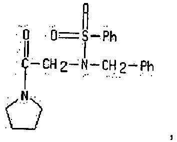 elle n'est pas utilisée en combinaison avec un agoniste d'un récepteur de glucocorticoïde.

Selon une mise en oeuvre particulière, le groupement R1 des molécules de formule (I) est choisi parmi les groupements suivants :
∘ un groupement phényle non substitué ou substitué par un atome F ou Cl, par un groupement méthyle ou éthyle, par un groupement N,N-diméthyl-sulfonamide ou par deux groupements choisis parmi les groupements méthyle, éthyle, hydroxy, méthoxy et éthoxy,
∘ un groupement 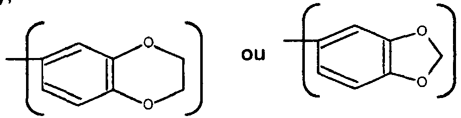 ou 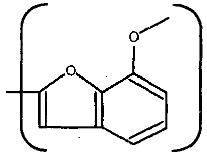
∘ un groupement furane non substitué ou substitué par un groupement méthyle, éthyle, hydroxy, méthoxy ou éthoxy,
∘ un groupement thiophène non substitué ou substitué par un groupement méthyle, éthyle, hydroxy, méthoxy ou éthoxy; le groupement R2 des molécules de formules (I) est choisi parmi les groupements suivants :
∘ un groupement 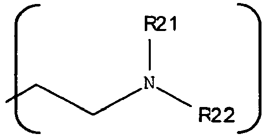 où R21 et R22 sont des atomes de carbone faisant chacun partie d'une chaîne alkyle comprenant 1, 2 ou 3 atomes de carbone, ou bien faisant tous deux partie avec l'atome d'azote auquel ils sont liés d'un même hétérocycle saturé ou non comprenant également un atome d'oxygène ou un second atome d'azote,
∘ ou un groupement 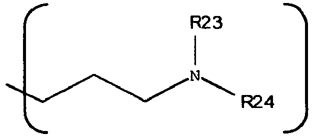 où R23 et R24 sont des atomes de carbone faisant chacun partie d'une chaîne alkyle comprenant 1, 2 ou 3 atomes de carbone, ou bien faisant tous deux partie avec l'atome d'azote auquel ils sont liés d'un même hétérocycle saturé ou non comprenant également un atome d'oxygène ou un second atome d'azote,
∘ ou un groupement 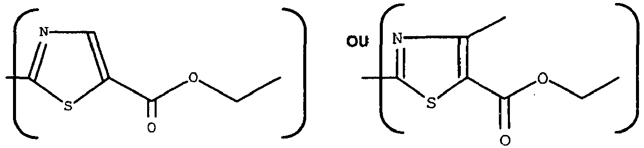 ou 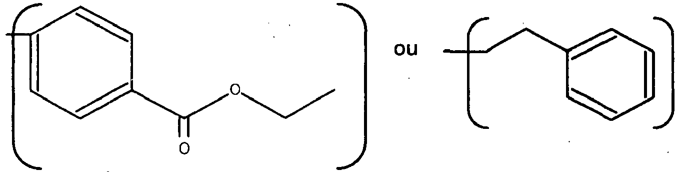 ou 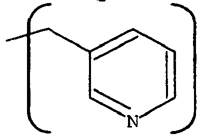 et le groupement R3 des molécules de formules (I) est choisi parmi les groupements suivants :
∘ un groupement 2-pyridinyle, 3-pyridinyle ou 4-pyridinyle non substitué,
∘ un groupement phényle non substitué ou substitué par un groupement benzoxy, et/ou par un groupement hydroxy, et/ou par un groupement méthyle, et/ou par un groupement éthyle, et/ou par un groupement propyle, et/ou par un ou deux atomes Br, F ou Cl, et/ou par un à trois groupements hydroxy, méthoxy ou éthoxy.

Selon une mise en oeuvre particulière, lorsque le groupement R3 des molécules de formule **(I)** est un groupement phényle, le groupement R2 des molécules de formule **(I)** n'est pas un groupement aromatique ou un hétérocycle.

Selon une autre mise en oeuvre particulière, le groupement R4 des molécules de formule **(II)** est une chaîne carbonée saturée ou insaturée comprenant 1, 2 ou 3 atomes de carbone; le groupement R5 est un groupement phényle ou un groupement amine secondaire substitué par un groupement phényle substitué ou non, ou par un groupement 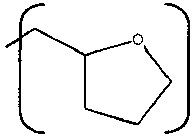 le groupement R6 des molécules de formule **(II)** est un atome d'hydrogène ou de chlore ou un groupement méthyle, éthyle, hydroxy, méthoxy ou éthoxy; le groupement R7 des molécules de formule **(II)** est un atome d'hydrogène ou de chlore ou un groupement méthyle, éthyle, hydroxy, méthoxy ou éthoxy ; et le groupement R8 des molécules de formule **(II)** est un atome d'hydrogène ou de chlore ou un groupement méthyle, éthyle, hydroxy, méthoxy ou éthoxy.

Selon une autre mise en oeuvre particulière,
- le groupement R9 des molécules de formule **(III)** est un atome d'hydrogène et le groupement R10 est un groupement phényle substitué ou non, ou les groupements R9 et R10 font partie d'un même hétérocycle substitué ou non comprenant 2 atomes d'azote et 4 atomes de carbone ;
- le groupement R11 des molécules de formule **(III)** est un atome hydrogène ou un groupement méthyle, éthyle, hydroxy, méthoxy ou éthoxy ;
- le groupement R12 des molécules de formule **(III)** est un atome hydrogène ou un groupement méthyle, éthyle, hydroxy, méthoxy ou éthoxy;
- le groupement R13 des molécules de formule **(III)** est un atome hydrogène ou un groupement méthyle, éthyle, hydroxy, méthoxy ou éthoxy ;
- et le groupement R14 des molécules de formule **(III)** est un atome hydrogène ou un groupement méthyle, éthyle, hydroxy, méthoxy ou éthoxy.

Selon une autre mise en oeuvre particulière, le groupement R15 des molécules de formule (IV) est un groupement 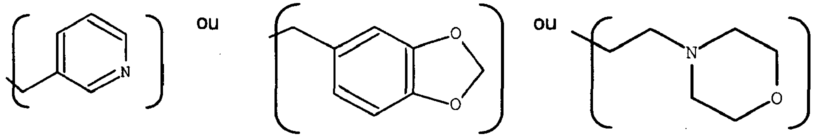 le groupement R16 des molécules de formule **(IV)** est un atome d'hydrogène ou de chlore ou un groupement méthyle, éthyle, hydroxy, méthoxy ou éthoxy et le groupement R17 des molécules de formule **(IV)** est un groupement méthyle, éthyle, hydroxy, méthoxy, éthoxy, acétoxy, méthoxycarbonyle ou éthoxycarbonyle.

Selon une mise en oeuvre particulière, la molécule selon l'invention n'est pas 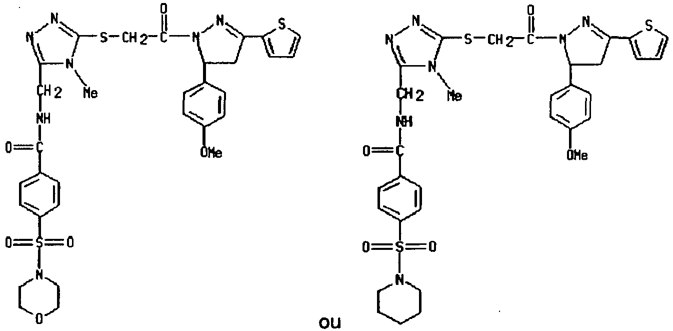 ou 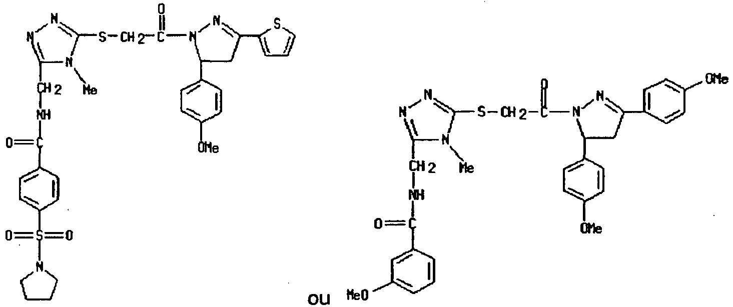 ou 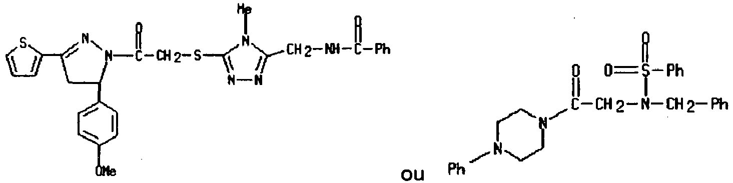 ou

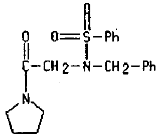

Par **chaîne carbonée,** on entend une chaîne organique ayant pour squelette un enchaînement linéaire ou cyclique, ramifié ou non, d'atomes de carbone contigus, reliés par des liaisons covalentes. Une chaîne carbonée au sens de la présente invention peut par exemple être un enchaînement linéaire d'un à vingt, de préférence de 1 à 12, de 1 à 10, de 1 à 6, de 1 à 5 ou de 1 à 4 atomes de carbone. Il peut notamment s'agir d'un groupement alkyle, c'est-à-dire dérivé d'un alcane (molécule hydrocarbonée saturée linéaire ou ramifiée) par perte d'un atome d'hydrogène, par exemple un groupement méthyle, un groupement éthyle, un groupement propyle linéaire ou ramifié ou un groupement butyle linéaire ou ramifié ou d'une chaîne hydrocarbonée insaturée linéaire ou ramifiée, par exemple un groupement éthényle ou éthynyle. Il est entendu que les électrons de la couche externe (au nombre de 4) de chaque atome de carbone formant le squelette de la chaîne carbonée ne participant pas à une liaison covalente avec un autre atome de carbone ou avec un hétéroatome, participent à une liaison covalente avec un atome d'hydrogène.

Par **hétéroatome,** on entend un atome différent du carbone ou de l'hydrogène et non métallique, par exemple l'oxygène, l'azote, le soufre, le phosphore ou les halogènes.

Par **groupement aromatique** ou **aryle**, on entend un système cyclique insaturé respectant la règle d'aromaticité de Hückel. Il peut s'agir par exemple d'un groupement phényle (groupement dérivé d'un noyau benzénique).

Par **hétérocycle** on entend un système cyclique dans lequel un ou plusieurs atomes de carbone sont remplacés par un hétéroatome comme par exemple l'oxygène, l'azote ou le soufre. Il peut notamment s'agir hétérocycles aromatiques, tels que le pyrrole, le thiophène, le furane et la pyridine ou d'hétérocycles saturés, tels que les sucres, ou oses. Un hétérocycle selon l'invention comprend par exemple 2 à 8 atomes de carbone et 1 à 4 hétéroatomes, de préférence il comprend 2, 3, 4 ou 5 atomes de carbone et 1, 2, 3 ou 4 hétéroatomes.

Chaque atome faisant partie d'une chaîne carbonée, d'un groupement aromatique, d'un cycle ou d'un hétérocycle au sens de la présente invention peut être substitué par le biais d'une liaison covalente par un ou plusieurs halogènes, par exemple le Fluor, le Chlore, l'Iode ou le Brome, et/ou par un ou plusieurs groupements organiques, par exemple un ou plusieurs groupements aromatiques (tel qu'un groupement phényle), cycliques, hétérocycliques (tel qu'un groupement furane ou thiophène), alkyles (tel qu'un groupement méthyle, un groupement éthyle, un groupement propyle linéaire ou ramifié ou un groupement butyle linéaire ou ramifié), alkoxy (tel qu'un groupement méthoxy (OCH₃) ou éthoxy (OCH₂CH₃)), carboxyle (tel qu'un groupement carboxy (COOH)), carbonyle (tel qu'un groupement acétoxy (OCOCH₃) ou méthoxycarbonyle (COOCH₃) ou éthoxycarbonyle (COOCH₂CH₃)), amines primaires, secondaires ou tertiaires, amide (tel qu'un groupement acétamide) ou sulfonamide (tel qu'un groupement N,N-diméthyl-sulfonamide). Il est entendu qu'un cycle ou hétérocycle saturé, insaturé ou aromatique peut être fusionné avec un autre cycle, par exemple par l'intermédiaire d'une liaison simple ou double entre deux atomes de carbone.

La présente invention a également pour objet une composition pharmaceutique comprenant une molécule selon l'invention ainsi qu'un excipient acceptable sur le plan pharmacologique.

Selon une mise en oeuvre la composition pharmaceutique selon l'invention comprend également une autre molécule thérapeutique. Il peut s'agir par exemple d'une molécule thérapeutique également utilisée pour la prévention ou le traitement d'une pathologie dépendante d'une voie métabolique impliquant Syk ou au contraire d'une molécule thérapeutique utilisée pour la prévention ou le traitement d'une pathologie non dépendante d'une voie métabolique impliquant Syk. Selon une mise en oeuvre particulière, la composition pharmaceutique selon l'invention ne comprend pas un agoniste d'un récepteur de glucocorticoïde.

Selon une autre mise en oeuvre particulière, la composition pharmaceutique selon l'invention peut être utilisée en combinaison avec une ou plusieurs autres compositions pharmaceutiques. Il peut s'agir par exemple de compositions pharmaceutiques également utilisées pour la prévention ou le traitement d'une pathologie dépendante d'une voie métabolique impliquant Syk ou au contraire de compositions pharmaceutiques utilisées pour la prévention ou le traitement d'une pathologie non dépendante d'une voie métabolique impliquant Syk. Selon cette mise en oeuvre, la composition pharmaceutique selon l'invention et la ou les autres compositions pharmaceutiques peuvent être administrée simultanément ou en alternance, par la même voie d'administration ou par des voies différentes. Selon une mise en oeuvre particulière, la composition pharmaceutique selon l'invention n'est pas utilisée en combinaison avec un agoniste d'un récepteur de glucocorticoïde.

La présente invention a également pour objet un procédé pour l'identification d'une molécule organique de poids moléculaire compris entre 50 et 2500 Dalton se liant à la protéine tyrosine kinase Syk et capable d'inhiber d'au moins 10%, par exemple d'au moins 15, 20, 25, 30, 35, 40, 45, 50, 55, 60, 65, 70, 75, 80 ou 85 % *in vitro* la liaison (i) du fragment d'anticorps G4G11 (SEQ ID N°2), ou (ii) d'un anticorps ou fragment d'anticorps se liant à la protéine tyrosine kinase Syk humaine au niveau d'un épitope comprenant au moins l'un des résidus 65 à 74 de la séquence en acides aminés de la protéine tyrosine kinase Syk humaine représentée par la séquence SEQ ID N°1, ou (iii) d'un anticorps ou fragment d'anticorps se liant à la protéine tyrosine kinase Syk humaine et inhibant d'au moins 10% la liaison du fragment d'anticorps G4G11 avec la protéine tyrosine kinase Syk humaine (SEQ ID N°1), à la protéine tyrosine kinase Syk humaine ou à l'une de ses variantes chez l'animal, comprenant au moins les étapes suivantes :
a) le criblage à partir d'une banque de molécules organiques candidates de poids moléculaire compris entre 50 et 2500 Da de celles susceptibles de se lier à la protéine Syk au niveau de la poche tridimensionnelle de liaison sur la protéine Syk d'une molécule choisies parmi les molécules de formule C-13, I, II, III, IV ou 1 à 87 telles qu'illustrées ci-dessus;
b) la sélection parmi les molécules identifiées en a) de celles capables d'inhiber d'au moins 10%, par exemple d'au moins 15, 20, 25, 30, 35, 40, 45, 50, 55, 60, 65, 70, 75, 80 ou 85 % *in vitro* la liaison de l'anticorps ou fragment d'anticorps (i), (ii) ou (iii) à la protéine Syk.

Selon une mise en oeuvre particulièrement préférée, la molécule de l'étape a) choisies parmi les molécules de formule C-13, I, II, III, IV ou 1 à 87 est la molécule **C-13**, la molécule (**59**) ou la molécule (**61**).

Selon une mise en oeuvre préférée, le procédé selon l'invention comprend une étape supplémentaire préalable à l'étape a) d'identification de la poche tridimensionnelle de liaison sur la protéine Syk de la molécule choisies parmi les molécules de formule C-13, I, II, III, IV ou 1 à 87. Cette étape préalable peut notamment être réalisée par un « docking in silico ».

Par « **docking *in silico*** » ou « **amarrage moléculaire** » ou « **docking virtuel** », on entend le recours à un outil de bioinformatique pour prédire et modéliser le positionnement d'un ligand au sein d'une macromolécule. En particulier, les outils de Docking *in silico* permettent de calculer la probabilité qu'un composé chimique donné puisse s'amarrer avec une protéine active cible, par exemple au niveau d'une poche de liaison tridimensionnelle préalablement identifiée.

Selon une mise en oeuvre préférée, le procédé selon l'invention comprend une étape supplémentaire c) de sélection parmi les molécules identifiées en b) de celles capables d'inhiber de 50% *in vitro* la dégranulation des mastocytes à une concentration (CI50) comprise entre 1ng/ml et 1mg/ml, par exemple à une concentration comprise entre 1 ng/ml et 500 µg/ml, entre 1 ng/ml et 250 µg/ml, entre 1 ng/ml et 100 µg/ml, entre 1 ng/ml et 50 µg/ml, entre 1 ng/ml et 10 µg/ml, entre 1 ng/ml et 5 µg/ml ou entre 1 ng/ml et 2 µg/ml.

Selon une mise en oeuvre particulièrement préférée des utilisations et procédés décrits ci-dessus, la molécule selon l'invention est choisie parmi le groupe constitué par la molécule C-13 et les molécules N°1 à 87 présentées dans le tableau 1.

Ces molécules ont été identifiées par les inventeurs dans le cadre d'un projet faisant suite à une précédente étude (Dauvillier et al., 2002⁷ ), au cours de laquelle ils ont exprimé des fragments d'anticorps scFv (« single chain variable domain ») (ou « anticorps intracellulaires » ou « intracorps »), G4G11 (SEQ ID N° 2) et G4E4 (SEQ ID N° 3) dans une lignée mastocytaire. Cette étude a démontré les effets inhibiteurs de ces « anticorps intracellulaires » ou « intracorps » sur la libération de médiateurs allergiques induite par la stimulation du FcεRI au niveau de la membrane des mastocytes. Les fragments d'anticorps scFv G4G11 et G4E4 ont été isolés à partir d'une banque combinatoire criblée contre une protéine recombinante contenant les domaines SH2 de Syk et la région inter-domaine A qui les sépare, c'est-à-dire une portion de la protéine Syk ne comprenant pas le domaine kinase de Syk⁸.

Le **procédé ADA** (« **antibody displacement assay »)** est un procédé développé par les inventeurs et décrit dans WO2005106481, qui permet notamment l'identification d'un ligand capable de moduler sélectivement une cascade fonctionnelle impliquant une cible, comprenant une première étape d'identification d'un anticorps intracellulaire capable de se lier à la cible et de moduler la cascade fonctionnelle en question, une seconde étape de criblage à partir d'une banque de petites molécules organiques, des ligands modulateurs de la liaison entre la cible et l'anticorps intracellulaire pouvant être effectuée *in vitro* dans un essai extracellulaire, et une troisième étape d'identification à partir des ligands modulateurs obtenus à l'étape 2, de ceux capables de moduler la cascade fonctionnelle dans la cellule.

Les inventeurs ont émis l'hypothèse selon laquelle les fragments d'anticorps G4G11 et G4E4 se lient au niveau d'une région de Syk interagissant avec un ou des partenaires essentiels de la cascade fonctionnelle conduisant à la dégranulation. Prenant en considération les limites de l'utilisation d'anticorps intracellulaires en thérapie, telles que le transfert efficace du gène encodant l'anticorps dans les cellules cibles⁹, les inventeurs ont cherché à isoler des molécules organiques qui agissent comme des mimes fonctionnels de l'intracorps G4G11 et pouvant être utilisées plus aisément en thérapie. Pour ce faire, ils ont utilisé le procédé ADA pour cribler une banque de 3000 petites molécules organiques et identifier des inhibiteurs potentiels de la réponse allergique.

Parmi les 3000 petites molécules organiques testées, les inventeurs ont identifié la petite molécule C-13 (méthyl 2-{5-[(3-benzyl-4-oxo-2-thioxo-1,3-thiazolidin-5-ylidene)méthyl]-2-furyl}benzoate) et ont mis en évidence sa capacité de modulation de l'interaction du fragment d'anticorps G4G11 ou G4E4 avec Syk *in vitro* et d'inhibition de la dégranulation des mastocytes induite par FcεRI *in vitro.*

Les inventeurs ont notamment mis en évidence le fait que le composé C-13 inhibe le choc anaphylactique lorsqu'il est administré oralement et possède des propriétés anti-allergiques prometteuses, illustrant le fort potentiel thérapeutique de candidats médicaments isolés en utilisant l'approche décrite ici.

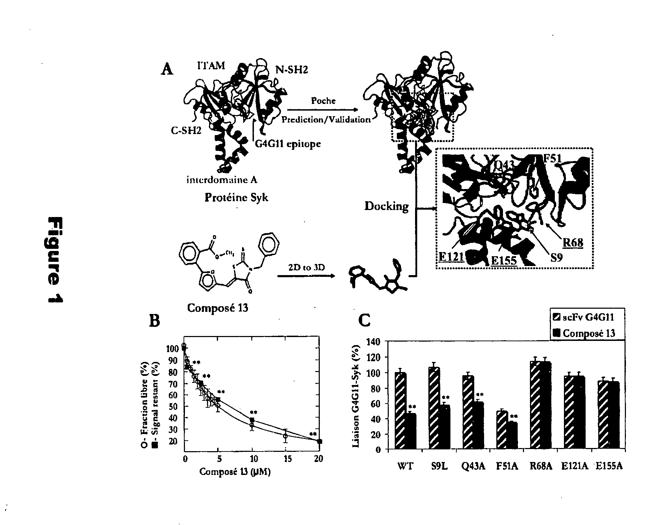

Les inventeurs ont également mis en évidence le fait que C-13 se lie à Syk au niveau d'une cavité nouvellement identifiée située entre les deux domaines SH2 et l'interdomaine A de Syk (Fig.1). La poche de liaison de C-13 forme une zone d'interaction unique qui est spécifique à Syk, et ne correspond pas à un site de liaison connu de ligands physiologiques de Syk tels que le peptide ITAM doublement phosphorylé au niveau de résidus tyrosine (Fig. 1A). Les résultats obtenus suggèrent que C-13 inhibe l'interaction de Syk avec certains de ses substrats macromoléculaires, soit directement de part le fait que C-13 occupe une surface au niveau de laquelle un partenaire de Syk pourrait établir un contact direct, et/ou par un effet allostérique.

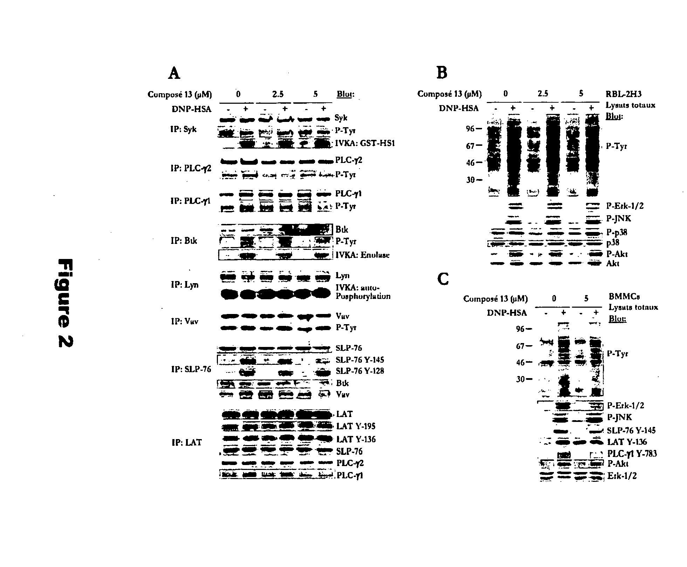

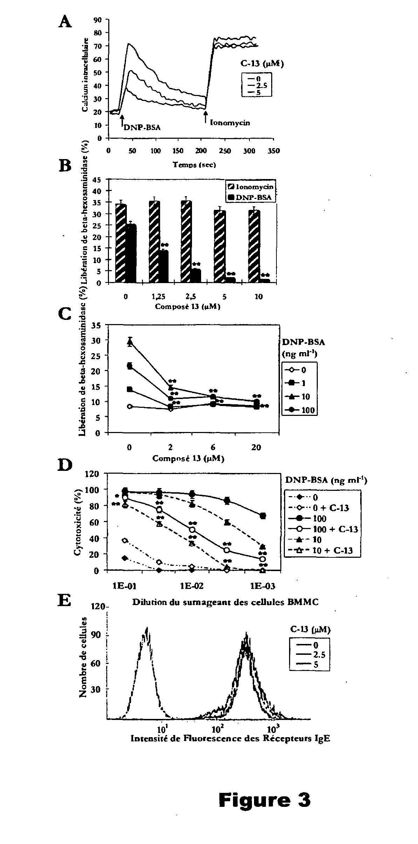

Les études biochimiques réalisées sur des mastocytes ont en effet montré que C-13 inhibe la phosphorylation FcεRI-dépendante du substrat SLP-76 au niveau de résidus tyrosine qui contribue à sa fonction d'adaptateur pour la liaison et/ou la stabilisation de Btk, PLC-γ et Vav au complexe de signalisation macromoléculaire formé avec LAT^{22, 28-30} (Fig.2). Ceci affecte la phosphorylation et l'activité catalytique de Btk et le renouvellement de PLC-γ à proximité de Syk et/ou Btk pour sa phosphorylation complète qui est requise pour le maintient du flux de calcium et de l'exocytose ³¹⁻³⁵ . En effet, C-13 a inhibé les réponses mastocytaires précoce (libération de β-hexosaminidase) et tardive (sécrétion de TNF-α) induites par l'aggrégation au niveau des récepteurs FcεRI avec une CI50 estimée à 2 µM (Fig. 3).

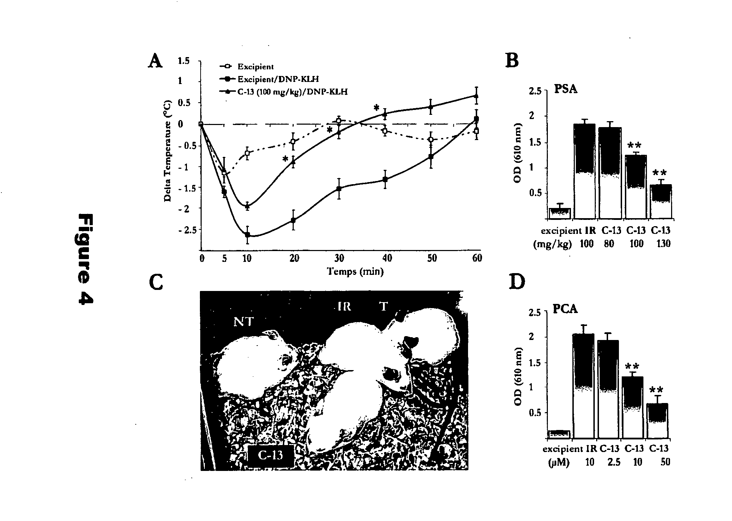

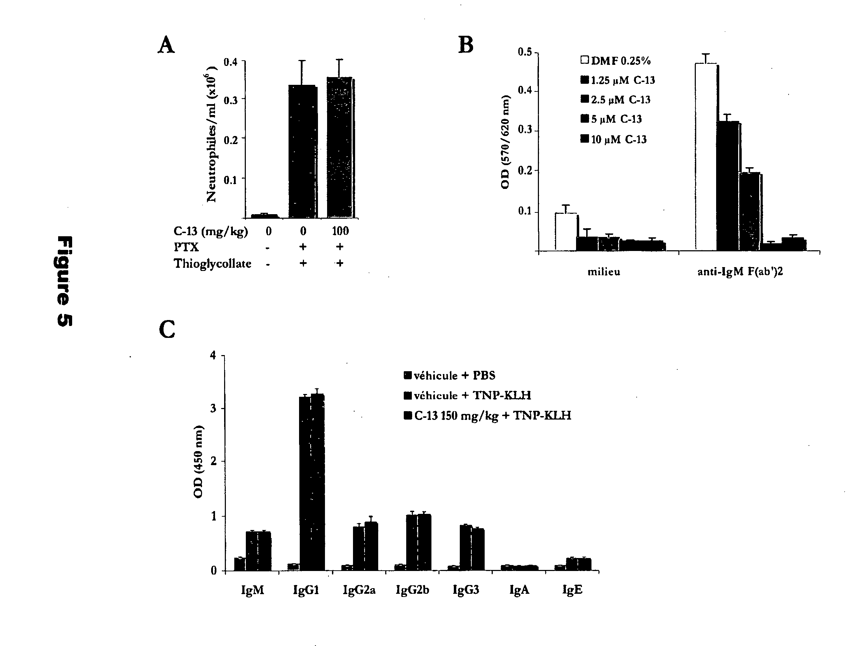

De façon importante, l'administration orale d'une dose unique de C-13 a inhibé l'anaphylaxie passive systémique (PSA) induite par IgE avec une CI50 estimée à 110 mg/kg (Fig. 4), confirmant les propriété anti-allergiques prometteuses de ce composé. En revanche, une administration orale unique de 100 mg/kg de C-13 n'a pas affecté le recrutement Syk-dépendant des neutrophiles induit par le thioglycolatte dans la cavité péritonéale en présence de la toxine de *Bordetella pertussis* (Fig. 5A). De plus, les inventeurs ont montré que malgré le fait que la prolifération *in vitro* BCR-dépendante des lymphocytes B était inhibée de façon dose-dépendante par C-13 (Fig. 5B), les réponses anticorps de souris immunisées avec un antigène thymo-dépendant n'ont pas été affectées par l'administration orale de 150mg/kg de C-13 (Fig. 5C). Par conséquent, la molécule C-13 n'affecte pas certaines réponses dépendantes de Syk mais non dépendantes des mastocytes *in vivo* à des doses et durées d'administration auxquelles elle peut inhiber une réponse allergique sévère.

Prenant en considération l'absence d'effet toxique apparent suivant l'administration orale ou locale de C-13 sur une période allant d'une heure à 12 jours, C-13 peut être considéré comme le potentiel premier membre d'une nouvelle famille d'inhibiteurs de Syk pouvant être administrés oralement et de molécules pharmacologiquement actives ayant un effet anti-inflammatoire. L'approche de criblage de molécules pharmaceutiques décrite ici constitue une plateforme générique dans laquelle l'utilisation initiale d'anticorps permet de mettre en évidence les domaines de la molécule cible ayant un potentiel thérapeutique, facilitant ainsi la conception de molécules chimiques (à travers un criblage *in silico* et/ou *in vitro*) capable d'agir en tant que mimes fonctionnels de l'anticorps, et en tant qu'inhibiteurs potentiels d'interactions protéines-protéines. De plus, les inventeurs ont mis en évidence le fait que ces petites molécules peuvent induire la réponse souhaitée dans des modèles cellulaires et animaux, ceci venant en appui du concept en faveur du remplacement de larges macromolécules difficiles à administrer par des petites molécules organiques pouvant être administrées oralement.

Le site probable de liaison à Syk a été prédit *in silico,* guidé par la localisation de l'épitope de G4G11. Une poche candidate située à côté de l'épitope de G4G11, au niveau de l'interface située entre les deux domaines SH2 et le lieur inter-domaine de Syk et comprenant les résidus Ser 9, Gln 43, Phe 51, Ile 66, Glu 67, Arg 68, Glu 69, Leu 70, Asn 71, Gly 72, Thr 73, Tyr 74, Ala 75, Glu 121 et Glu 155 a ainsi été identifiée (Fig. 1 C). Des expériences de mutagenèse dirigée ont permis de confirmer que les résidus Arg 68, Glu 121 et Glu 155 de la protéine Syk humaine (SEQ ID N° 1) contribuent significativement à l'interaction avec C-13, la mutation de ces résidus supprimant l'inhibition causée par C-13, tandis que la liaison du scFv G4G11 est maintenue (Fig. 1A, 1C). Ces résultats tendent à confirmer l'hypothèse selon laquelle la cavité (ou poche) de liaison de C-13 sur Syk est localisée dans un voisinage proche du site de liaison de l'intracorps G4G11.

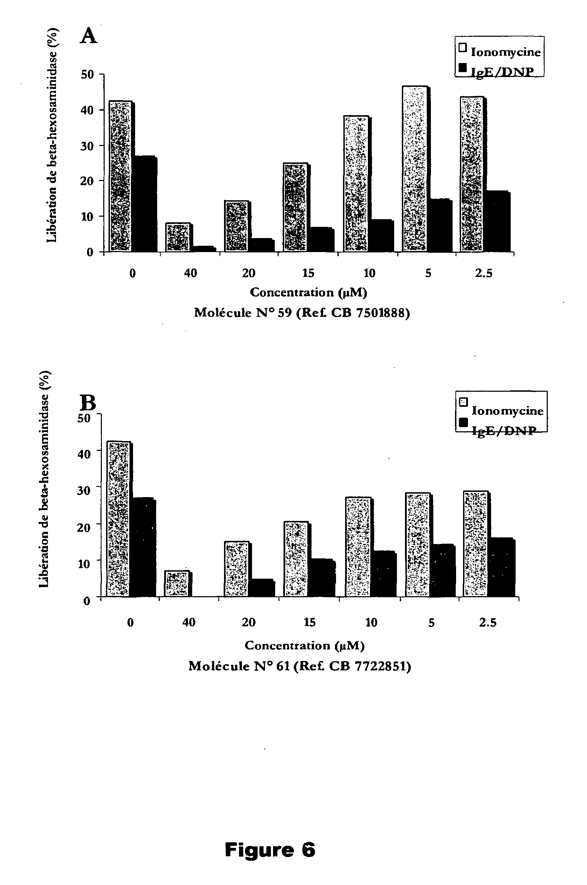

Les inventeurs ont ensuite effectué un « docking » virtuel sur une banque de molécules afin d'identifier des molécules candidates présentant les meilleures propriétés de liaison au niveau de cette poche tridimensionnelle, et ont testé la capacité de ces molécules candidates à inhiber la liaison du scFv G4G11 avec Syk. Ces molécules sont présentées dans le tableau 1 (voir exemple 2). La capacité de ces molécules à inhiber la dégranulation des mastocytes *in vitro* a également été testée. Avec les molécules N° 59 et 61 en particulier la concentration inhibant de 50% in vitro la dégranulation des mastocytes est de l'ordre de 5µM (voir figure 6).

Les résultats présentés dans la partie expérimentale et en particulier dans le tableau 1 mettent en évidence le fait que les molécules ou sels selon l'invention inhibent les réactions d'hypersensibilité de type I, et en particulier la dégranulation des mastocytes dépendante des IgE, et sont aussi capables d'interférer *in vivo* avec l'anaphylaxie passive cutanée et systémique chez des souris BALB/c.

### Brève description des figures

**Figure 1**. Liaison de C-13 à Syk *in vitro.* **A.** Structure chimique de C-13. Structure 3D de la poche prédite et validée pour C-13 (ou « Compound 13 ») comprenant les résidus Ser 9, Gln 43, Phe 51, Arg 68, Glu 121 et Glu 155 (représentation mesh); l'épitope G4G11 comprend les résidus 65 à 74 de la protéine Syk humaine représentée par SEQ ID N°1. **B.** Liaison du fragment scFv G4G11 à Syk mesurée en utilisant la méthodologie ADA en présence de C-13 (■). Liaison de C-13 à Syk mesurée en utilisant la spectroscopie par fluorescence (○) (Kd = 4,8 ± 0.2 µM). C. Liaison de G4G11 aux mutants Syk avec la méthodologie ADA. Inhibition significative avec C-13 *versus* DMF : **P<.01.
**Figure 2****.** C-13 inhibe l'activation de mastocytes induite par FcεRI. **A**. Les immunoblots réalisées sur des lysats de cellules RBL-2H3 ont été analysées par des immunoblots avec les anticorps indiqués. Les activités kinase *in vitro* des immunoblots Syk, Btk et Lyn ont été examinées. **B.** Les lysats de cellules RBL-2H3 et C. BMMCs ont été soumis à une électrophorèse et les protéines ont été analysées par immunoblot. Ces données sont représentatives d'au moins deux expériences.
**Figure 3****.** C-13 inhibe la libération de calcium et la dégranulation FcεRI-dépendantes. **A.** Analyse par FACS du flux de calcium IgE-dépendant dans des cellules RBL-2H3. **B.** Libération de β-hexosaminidase dans des cellules RBL-2H3 sensibilisées par IgE/DNP. **C.** Libération de β-hexosaminidase et, **D.** titration de TNF-α dans des cellules BMMCs sensibilisées par IgE/DNP; C-13: 3 µM. **E.** Analyse par FACS de l'expression de surface de FcεRI dans des cellules RBL-2H3. (0=0,25% DMF). Inhibition significative avec C-13 *versus* DMF : **P<.01 et *P<.05.
**Figure 4****.** Etudes *in vivo* chez des souris BALB/c. **A-C.** Réponse PSA. **A.** Evolution de la température, **B.** quantification de l'extravasation de bleu d'Evans, **C** Photographie représentant l'extravasation du bleu d'Evans suivant une administration orale de 130 mg/kg de C-13, d'un composé non pertinent (IR) ou du vecteur (T) ; Animal non traité (NT). Les oreilles et la queue des souris ayant reçu le composé non pertinent (IR) ou le vecteur seul (T) ont pris une coloration bleue prononcée, celles des souris traitées avec C-13 ont pris une légère coloration bleue et celles des souris non traitées sont de couleur normales **D.** Réponse PCA : quantification de l'extravasation du bleu d'Evans. Inhibition significative avec C-13 *versus* un composé non pertinent: **P < .01 ; *versus* le véhicule +DNP-KLH : *P < .05.
**Figure 5****.** Effets *in vivo* et *in vitro* de C-13 sur les neutrophiles et les lymphocytes B. **A.** Recrutement de neutrophiles dans la cavité péritonéale après injection de thioglycollate ou du véhicule en présence de la toxine *Bordetella Pertussis* (lorsqu'indiqué) (n=4). **B.** Prolifération *in vitro* de lymphocytes B induite par anti-IgM. **C.** Concentration du sérum en immunoglobulines 12 jours après immunisation par TNP-KLH (n=4).
**Figure 6****.** Effet des molécules N°59 (ref. Chembridge 7501888) et 61 (ref. Chembridge 7722851) sur la dégranulation des cellules RBL-2H3. Le niveau de libération de la β-hexosaminidase dans des cellules RBL-2H3 sensibilisées par IgE/DNP a été mesurée en présence **A.** de la molécule N°59 et **B.** de la Molécule N°61 à des concentrations allant de 0 à 40 µM.

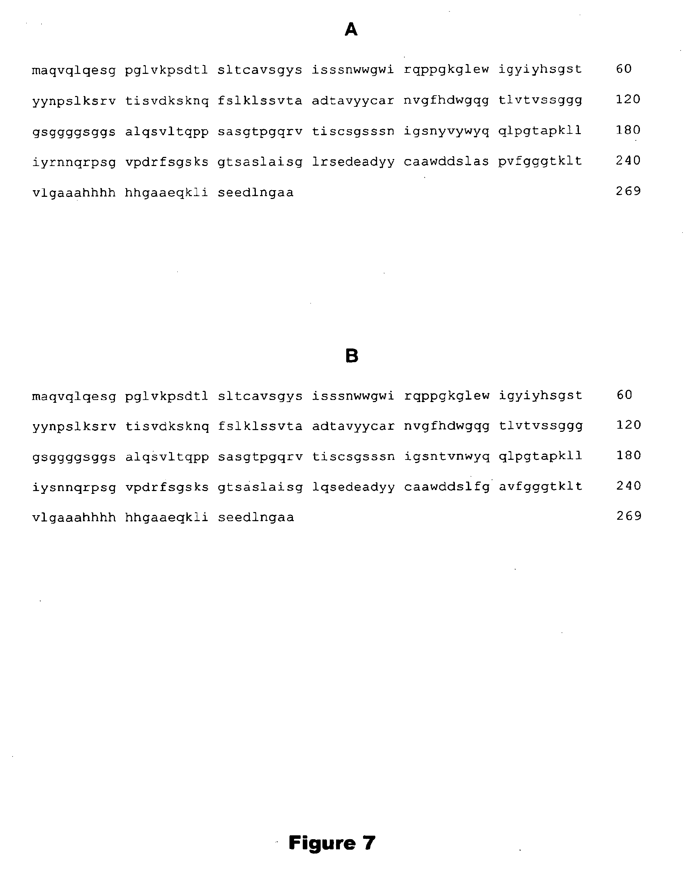

**Figure 7****.** Séquences en acides aminés des intracorps scFv **A.** G4G11 (SEQ ID N° 2) et ***B**. G4E4 (SEQ ID N° 3).*
**Figure 8****.** Séquence en acides aminés de la protéine Syk **A.** humaine (SEQ ID N° 1) correspondant au n° accession NP_003168 (NCBI) et **B.** murine correspondant au numéro d'accession NP_035648 (NCBI). Les résidus identifiés comme faisant partie de la poche tridimensionnelle de liaison de C-13 sur la protéine Syk humaine (**A**) et leurs équivalents sur la protéine Syk murine (**B**) sont indiqué en gras et soulignés.
**Figure 9****.** Représentation schématique des voies métaboliques impliquant la protéine tyrosine kinase Syk au niveau des mastocytes.

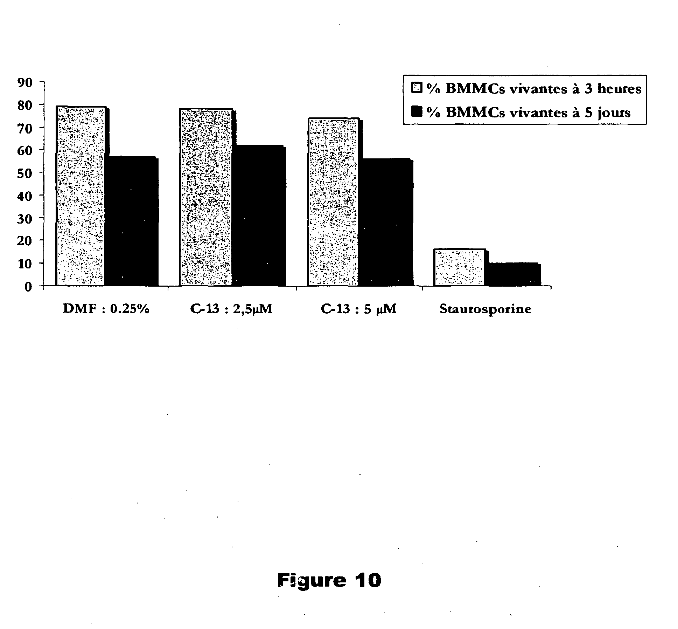

**Figure 10****.** Test de toxicité de C-13 sur les cellules BMMC. Les cellules BMMC ont été incubées à 37°C en présence de 2.5 µM ou 5 µM de C-13, de DMF à 0.25% ou de Staurosporine. Les pourcentages de cellules BMMC vivantes après 3 heures et après 5jours ont été mis en évidence par un double marquage à l'Annexine-V et Iodure de Propidium. La viabilité des cellules BMMC incubées en présence de 2.5 µM ou 5 µM de C-13 ou de DMF à 0.25% n'a pas été affectée de façon notoire après 3 heures (78%, 74% et 79% respectivement) ou cinq jours (62, 56 et 57%, respectivement). En revanche, la viabilité des cellules of BMMC traitées dans les mêmes conditions avec la Staurosporine était réduite à un niveau de 16% après seulement 3 heures.

### Exemples

### Exemple 1 - Identification de la molécule C-13 pouvant potentiellement être administrée oralement pour prévenir le choc anaphylactique.

### 1) Identification du composé 13 (C-13) et de sa cavité de liaison sur Syk

Les inventeurs ont développé la méthodologie ADA (Antigen Displacement Assay) pour identifier des petites molécules capables de déplacer l'association du scFv G4G11 avec Syk. Parmi les membres d'une banque de 3000 molécules chimiques, 15 petites molécules se sont montrées capables de rentrer en compétition avec la liaison du scFv G4G11 à Syk, et parmi ces composés, celui dénommé ci-après C-13 (Fig. 1A) fit preuve du meilleur potentiel d'inhibition avec une CI50 estimée à 4 µM (Fig. 1B). Ce résultat est en accord avec la valeur de la constante de dissociation (ou Kd) égale à 4,8 µM obtenue par la mesure par spectroscopie par fluorescence (ou spectrofluorométrie) de l'affinité *in vitro* de C-13 pour Syk (Fig. 1B). Pour comprendre le mécanisme d'action de C-13, les inventeurs ont dans un premier temps identifié le site de liaison de G4G11 en utilisant la méthode SPOT¹⁶. Un épitope localisé au niveau du domaine SH2 N-terminal de Syk, comprenant les acides aminés 65-74 et conservé à 100% dans les séquences de souris, de rat et humaines, a été identifié.

Sur la base de ces informations et de la structure 3D connue du complexe peptidique formé par les domaines SH2 de Syk et les motifs ITAM¹⁷, les inventeurs ont utilisé des approches informatiques pour chercher un site de liaison putatif pour C-13. Une poche candidate comprenant les résidus Ser 9, Gln 43, Phe 51, Ile 66, Glu 67, Arg 68, Glu 69, Leu 70, Asn 71, Gly 72, Thr 73, Tyr 74, Ala 75, Glu 121 et Glu 155 de la protéine Syk humaine (voir Figure 8A, SEQ ID N°1) et située à proximité de l'épitope de G4G11 a été identifiée (Fig. 1C). Une analyse structurelle a indiqué que les résidus Ser 9, Gln 43, Phe 51, Arg 68, Glu 121 et Glu 155 pourraient être impliqués dans la liaison du ligand, et pouvaient être mutés sans nuire à la structure 3D de la protéine. Pour valider plus avant la poche, ces six acides aminés ont été individuellement mutés et les mutants Syk ont été soumis à l'essai ADA. Les résidus Arg 68, Glu 121 et Glu 155 se sont montrés comme contribuant significativement à l'interaction avec la petite molécule, étant donné que leur mutation abrogeait l'inhibition causée par C-13, tandis que la liaison du scFv G4G11 était maintenue (Fig. 1C). Ces données ont confirmé le fait que la cavité de liaison de C-13 sur Syk est localisée dans un voisinage proche du site de liaison de G4G11.

### 2) Activation des mastocytes induite par FcεRI

Afin d'examiner les similarités fonctionnelles avec G4G11, les inventeurs ont exploré les effets biologiques de C-13 sur l'activation des mastocytes. L'incubation de cellules RBL-2H3 avec C-13 n'a pas affecté la phosphorylation et l'activité kinase induite par FcεRI de Syk (Fig. 2A) et, en conséquence, le niveau global de phosphorylation tyrosine de l'ensemble des protéines cellulaires connues comme étant principalement dépendantes de Syk était normal (Fig. 2B, C). Similairement à l'intracorps G4G11, C-13 a inhibé la phosphorylation et l'activité kinase induite par FcεRI de Btk (Fig. 2A) et la phosphorylation de PLC-γ1 et PLC-γ2, les deux isoformes de PLC-γ exprimés dans les mastocytes (Fig. 2A, C). La PTK Lyn phosphoryle à la fois Syk et Btk menant à leur activation complète et à la phosphorylation subséquente de PLC-γ¹⁸. Etant donné que C-13 n'a pas affecté l'activation de Lyn FcεRI-dépendante (Fig. 2A), il peut être conclut que la réduction du niveau de phosphorylation de Btk et PLC-γ pourrait être due à un défaut par rapport à leur localisation correcte au voisinage des PTK amont.

### 3) Analyse des cascades de signalisation dépendantes de Fyn et Lyn

Dans les mastocytes, la cascade de signalisation Fyn/Gab2/PI3K conduit à l'activation de PI3K et à la génération de PI-3,4,5-P3 qui recrute un certain nombre de protéines contenant un domaine d'homologie de la pleckstrine (PH), incluant Btk et PLC-γ au niveau de la membrane plasmique ¹⁹. L'analyse de la phosphorylation d'Akt, un marqueur de l'activité PI3K, a indiqué que C-13 n'affectait pas la cascade dépendante de Fyn (Fig. 2B, C), suggérant que le niveau de phosphorylation réduit de Btk et PLC-γ n'était pas du à un défaut au niveau de leur localisation membranaire, connue comme étant un facteur essentiel pour l'augmentation des flux de calcium²⁰.

Le recrutement de Btk et PLC-γ au niveau de la membrane requiert également la cascade de signalisation canonique Lyn/Syk/LAT/SLP-76. La phosphorylation de LAT par Syk conduit à la translocation de SLP-76 vers le complexe organisé par LAT²¹, où SLP-76 se co-localise avec Syk ²² . Cette localisation permet à Syk de phosphoryler des tyrosines N-terminales de SLP-76²³ qui deviennent des sites de liaison pour Vav, Nck et Btk. LAT et SLP-76 (au travers de son domaine riche en proline recrutant PLC-γ) coopèrent pour localiser PLC-γ au niveau de ce complexe membranaire, permettant la phosphorylation et l'activation de PLC-γ par Btk²⁴ et/ou Syk²⁵. L'utilisation d'anticorps phospho-spécifiques a montré que C-13 inhibe la phosphorylation de SLP-76, mais accroît la phosphorylation de LAT de manière dose-dépendante (Fig. 2A). Les inventeurs ont émis l'hypothèse selon laquelle l'inhibition de la phosphorylation de SLP-76 pourrait permette à une plus grande quantité de LAT d'interagir avec Syk, causant ainsi une augmentation de son niveau de phosphorylation. Ces résultats démontrent que la réduction de la phosphorylation de SLP-76 n'était pas due à un défaut au niveau de son recrutement vers LAT, et a résulté en un défaut de la co-localisation de Btk et dans une moindre mesure de celle de Vav avec SLP-76 (Fig. 2A). Néanmoins, la phosphorylation de Vav connu comme étant indépendante de son recrutement vers SLP-76²⁶ n'a pas été inhibée (Fig. 2A).

### 4) Activation de MAPK

L'association de SLP-76 avec Vav et/ou Nck contribue à une activation optimale des MAP kinases dans les mastocytes²⁷. Les inventeurs ont mis en évidence le fait que C-13 affecte légèrement l'activation des MAP kinases (évaluée au travers de leur niveau de phosphorylation) : une forte concentration de C-13 diminue le niveau de phosphorylation de ERK1/2, tandis que les niveaux de phosphorylation de p38 et JNK restent normaux (Fig. 2B, C).

### 5) Flux de calcium et dégranulation

La liaison de Btk et Vav à SLP-76 est critique pour la régulation de l'activité de PLC-γ au niveau de la membrane, la mobilisation du calcium et l'exocytose des granules^{27, 28}. Les inventeurs ont mis en évidence le fait que l'association de PLC-γ avec LAT était inhibée par C-13 de façon dose-dépendante (Fig. 2A). De façon cohérente avec le défaut au niveau de la phosphorylation de PLC-γ1 et PLC-γ2, les mastocytes ont montré une amplitude du flux de calcium réduite en réponse à la liaison de FcεRI (Fig. 3A), et les réponses allergiques précoce et tardive induite par FcεRI dans des cellules BMMC (« bone marrow derived mast cell ») et dans la lignées cellulaire RBL-2H3 sont également affaiblies de façon dose-dépendante, basée sur la mesure de la libération de β-hexosaminidase et la sécrétion de TNF-α (Fig. 3B, C, D). Les résultats ont également montré que C-13 n'a pas d'effet toxique sur les mastocytes. En effet, ni la dégranulation induite par ionomycine (fig. 3B), ni la viabilité des cellules BMMC (voir figure 10) n'ont été affectées de façon détectable par le traitement avec C-13. De plus, les défauts observés au niveau de l'activation des mastocytes ne sont pas dus à un niveau réduit d'expression de surface de FcεRI, l'analyse par cytométrie de flux ayant indiqué que les cellules incubées avec C-13 expriment des niveaux de FcεRI similaires à ceux des cellules contrôles (Fig. 3E).

### 6) Anaphylaxie passive systémique (PSA) et cutanée (PCA)

Pour finir, pour étendre ces observations sur les fonctions des mastocytes *in vivo,* les inventeurs ont testé les effets de C-13 sur l'anaphylaxie passive systémique (PSA) et cutanée (PCA) induite chez des souris BALB/c par l'administration de molécules IgE DNP-spécifiques suivi d'une stimulation intraveineuse avec l'haptène DNP-KLH. Cela mime l'anaphylaxie systémique comme démontré par les changements immédiats au niveau cardio-pulmonaire et l'augmentation de la perméabilité vasculaire. L'intensité de l'anaphylaxie systémique a été déterminée par la mesure à la fois de la baisse de la température corporelle et de l'augmentation de la perméabilité vasculaire suivant l'administration de l'antigène. Après l'administration orale de C-13 (et avant la stimulation avec l'antigène), les animaux sont apparus sains avec aucun signe manifeste de toxicité. L'administration d'une dose orale unique de 100 mg/kg de C-13 a inhibé l'hypothermie et accéléré le rétablissement des animaux (Fig. 4A). A partir de la quantification de l'extravasation du bleu d'Evans, il a été déterminé que C-13 inhibe l'augmentation de la perméabilité vasculaire avec une CI50 estimée à 110 mg/kg (Fig. 4B et 4C). C-13 a également montré un effet inhibiteur sur la PCA avec une CI50 estimée à 25 µM (Fig. 4D).

### Exemple 2 - Identification à partir de la poche de liaison de C-13 d'autres inhibiteurs potentiels de la dégranulation des mastocytes.

En partant de la poche de liaison identifiée dans l'exemple 1-1), un crible par « docking » virtuel a été conduit sur un ensemble de 350000 molécules présentes dans la chimiothèque de ChemBridge Corporation (San Diego, USA) afin d'identifier les 1000 molécules présentant les meilleures propriétés de liaison dans cette poche.

Le procédé ADA a ensuite été appliqué à chacune de ces 1000 molécules pour mesurer leur capacité d'inhibition de la liaison du scFv G4G11 avec Syk. Les 87 molécules présentant le meilleur taux d'inhibition (entre 11 et 86,5 %) sont présentées dans le tableau 1.

Ces 87 molécules ont également été testées in vitro pour évaluer leur capacité à inhiber la dégranulation de cellules RBL-2H3 (voir tableau 1). Les deux molécules présentant le meilleur potentiel (molécules N° 59 et 61) ont été testées à différentes concentrations sur des cellules RBL-2H3 afin d'évaluer plus finement la concentration inhibant de 50% in vitro la dégranulation (voir figure 6).

Enfin, l'affinité *in vitro* de la molécule C-13 et de certaines des 87 molécules mentionnées ci-dessus pour la protéine Syk a été mesurée par spectroscopie de fluorescence (ou spectrofluorométrie) et est exprimée par la constante de dissociation (ou Kd) en µmole/litre (µM).

**Tableau 1 : C-13 et les 87 molécules identifiées à partir de C-13 et du fragment d'anticorps G4G11**

| **Structure** | **Ref. CB** | **Nom** | **Rang** | **inhib. In vit.** | **IC50 degran.** | **Kd** | **N°** | **Gp** |
|---|---|---|---|---|---|---|---|---|
| 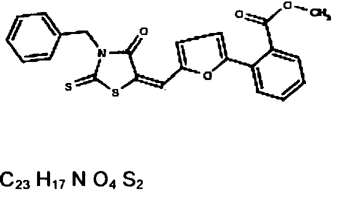 | **6197 026** | **méthyl 2-{5-[(3-benzyl-4-oxo-2-thioxo-1,3-thiazolidin-5-ylidene) méthyle]-2-furyl} benzoate** | - | **81%** | **1µg/ml** | **4,8 µM** | **C-13** | - |
| 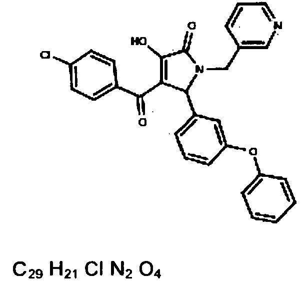 | 6752 784 | 4-(4-chloro benzoyl)-3-hydroxy-5-(3-phénoxy phényl)-1-(3-pyridinyl méthyl)-1,5-dihydro-2H-pyrrol-2-one | 611 | 86,5 | > 10µg/ml | 5 µM | 1 | 1 |
| 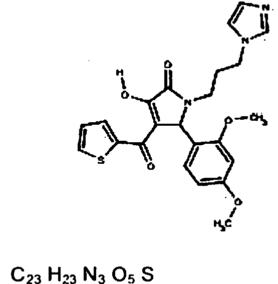 | 6670 340 | 5-(2,4-diméthoxy phényl)-3-hydroxy-1-[3-(1 H-imidazol-1-yl)propyl]-4-(2-thiényl carbonyl)-1,5-dihydro-2H-pyrrol-2-one | 792 | 81 | >10µg/ml | 16,3 µM | 2 | I |
| 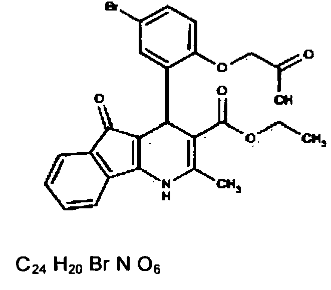 | 6422 575 | acide {4-bromo-2-[3-(éthoxy carbonyl)-2-méthyl-5-oxo-4,5-dihydro-1H-indéno[1,2-b]pyridin-4-yl]phénoxy} acétique | 706 | 81 | >10µg/ml | 6,2 µM | 3 | - |
| 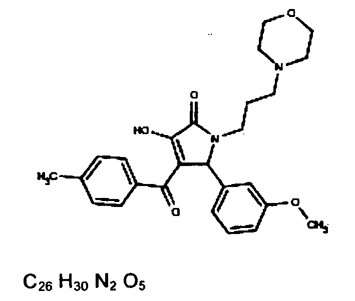 | 6882 059 | 3-hydroxy-5-(3-méthoxy phényl)-4-(4-méthyl benzoyl)-1-[3-(4-morpholinyl)pr opyl]-1,5-dihydro-2H-pyrrol-2-one | 243 | 79,5 | >10µg/ml | 9,4 µM | 4 | I |
| 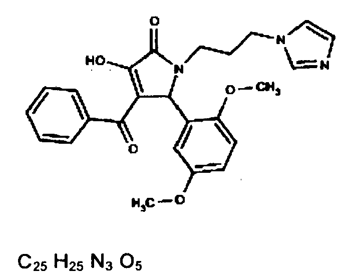 | 7111 786 | 4-benzoyl-5-(2,5-diméthoxy phényl)-3-hydroxy-1-[3-(1H-imidazol-1-yl)propyl]-1,5-dihydro-2H-pyrrol-2-one | 557 | 77,5 | >10µg/ml | - | 5 | I |
| 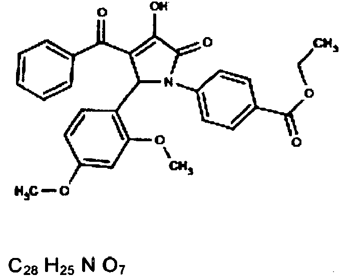 | 6203 863 | éthyl 4-[3-benzoyl-2-(2,4-diméthoxy phényl)-4-hydroxy-5-oxo-2,5-dihydro-1H-pyrrol-1-yl] benzoate | 423 | 73,5 | >10µg/ml | 6,1 µM | 6 | I |
| 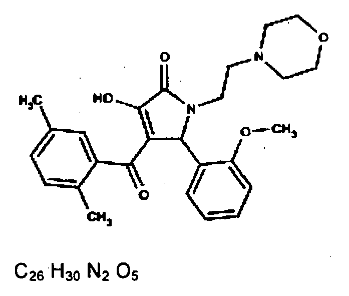 | 7347 627 | 4-(2,5-diméthyl benzoyl)-3-hydroxy-5-(2-méthoxy phényl)-1-[2-(4-morpholinyl)ét hyl]-1,5-dihydro-2H-pyrrol-2-one | 775 | 72 | >10µg/ml | - | 7 | I |
| 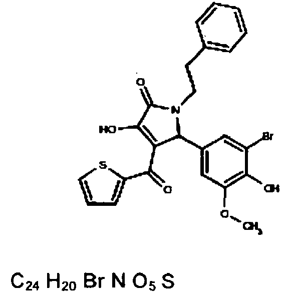 | 7489 416 | 5-(3-bromo-4-hydroxy-5-méthoxy phényl)-3-hydroxy-1-(2-phényléthyl)-4-(2-thiényl carbonyl)-1,5-dihydro-2H-pyrrol-2-one | 648 | 71 | >10µq/ml | - | 8 | I |
| 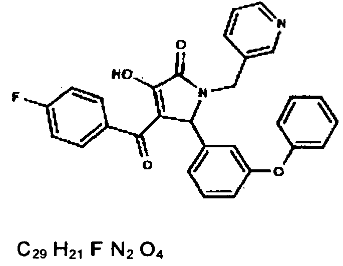 | 6719 738 | 4-(4-fluoro benzoyl)-3-hydroxy-5-(3-phénoxy phényl)-1-(3-pyridinyl méthyl)-1,5-dihydro-2H-pyrrol-2-one | 977 | 71 | >10µg/ml | - | 9 | I |
| 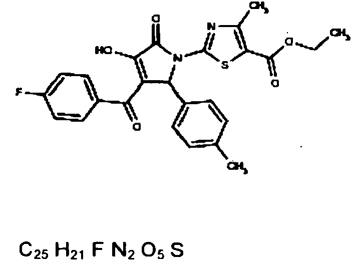 | 6650 234 | éthyl 2-[3-(4-fluorobenzoyl) -4-hydroxy-2-(4-méthyl phényl)-5-oxo-2,5-dihydro-1H-pyrrol-1-yl]-4-méthyl-1,3-thiazole-5-carboxylate | 946 | 71 | ~10µg/ml | 6,3 µM | 10 | I |
| 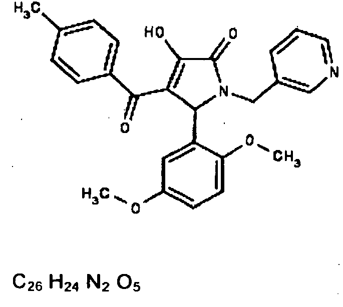 | 6652 639 | 5-(2,5-diméthoxy phényl)-3-hydroxy-4-(4-méthyl benzoyl)-1-(3-pyridinyl méthyl)-1,5-dihydro-2H-pyrrol-2-one | 829 | 70,5 | >10µg/ml | - | 11 | I |
| 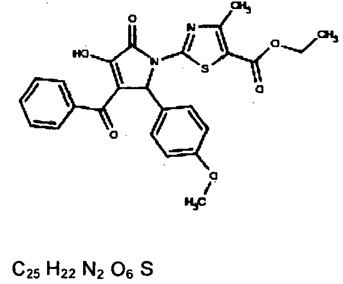 | 6673 225 | éthyl 2-[3-benzoyl-4-hydroxy-2-(4-méthoxy phényl)-5-oxo-2,5-dihydro-1H-pyrrol-1-yl]-4-méthyl-1,3-thiazole-5-carboxylate | 301 | 69 | >10µg/ml | - | 12 | I |
| 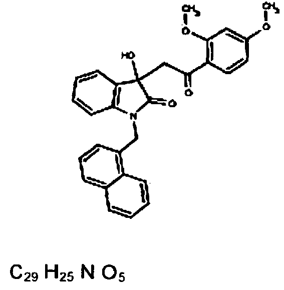 | 6800 873 | 3-[2-(2,4-diméthoxy phényl)-2-oxoéthyl]-3-hydroxy-1-(1-naphthyl méthyl)-1,3-dihydro-2H-indol-2-one | 250 | 67,5 | >10µg/ml | 4,6 µM | 13 | - |
| 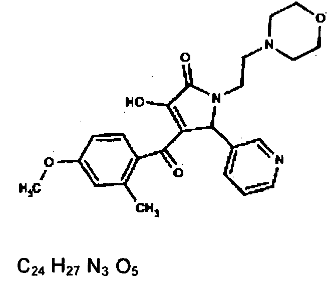 | 6879 058 | 3-hydroxy-4-(4-méthoxy-2-méthyl benzoyl)-1-[2-(4-morpholinyl)ét hyl]-5-(3-pyridinyl)-1,5-dihydro-2H-pyrrol-2-one | 758 | 67,5 | >10µg/ml | - | 14 | I |
| 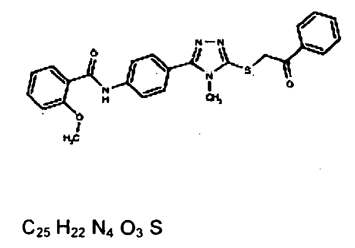 | 6282 824 | 2-méthoxy-N-(4-{4-méthyl-5-[(2-oxo-2-phényléthyl) thio]-4H-1,2,4-triazol-3-yl}phényl) benzamide | 905 | 67,5 | ~2,5 µg /ml | 5,6 µM | 15 | II |
| 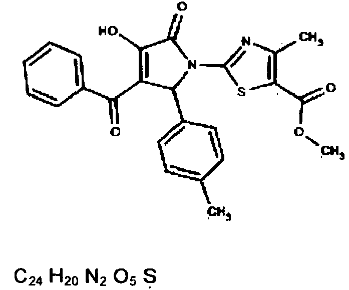 | 6750 319 | méthyl 2-[3-benzoyl-4-hydroxy-2-(4-méthyl phényl)-5-oxo-2,5-dihydro-1 H-pyrrol-1-yl]-4-méthyl-1,3-thiazole-5-carboxylate | 850 | 67 | >10µg/ml | - | 16 | I |
| 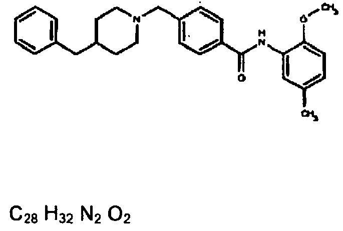 | 6474 819 | 4-[(4-benzyl-1-pipéridinyl) méthyl]-N-(2-méthoxy-5-méthylphényl) benzamide | 954 | 63 | >10µg/ml | 17,9 µM | 17 | - |
| 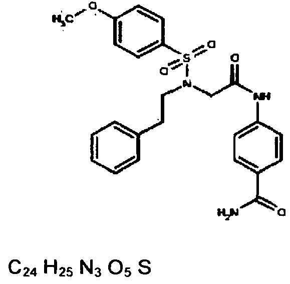 | 6498 669 | 4-{[N-[(4-méthoxy phényl) sulfonyl]-N-(2-phényl éthyl)glycyl] amino} benzamide | 808 | 63 | >10µg/ml | 0,8 µM | 18 | III |
| 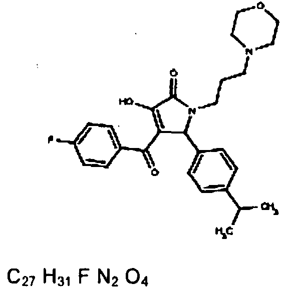 | 6651 552 | 4-(4-fluoro benzoyl)-3-hydroxy-5-(4-isopropyl phényl)-1-[3-(4-morpholinyl)pr opyl]-1,5-dihydro-2H-pyrrol-2-one | 100 | 60 | >10µg/ml | - | 19 | I |
| 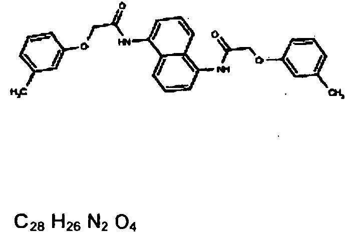 | 6453 860 | N,N'-1,5-naphthalènedi ylbis[2-(3-méthyl phénoxy) acétamide] | 145 | 60 | >10µg/ml | - | 20 | - |
| 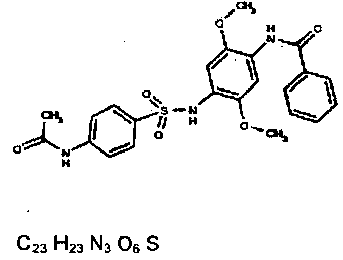 | 6853 966 | N-[4-({[4-(acétyl amino) phényl] sulfonyl} amino)-2,5-diméthoxy phényl] benzamide | 11 | 58 | >11µg/ml | - | 21 | - |
| 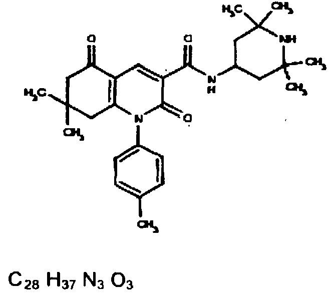 | 6866 968 | 7,7-diméthyl-1-(4-méthyl phényl)-2,5-dioxo-N-(2,2,6,6-tetraméthyl-4-pipéridinyl)-1,2,5,6,7,8-hexahydro-3-quinoline carboxamide | 255 | 57,5 | >10µg/ml | - | 22 | - |
| 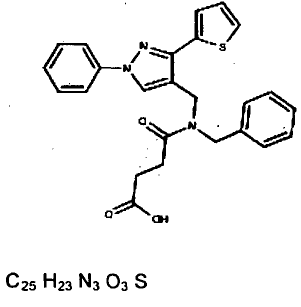 | 7938 324 | acide 4-(benzyl{[1-phényl-3-(2-thiényl)-1H-pyrazol-4-yl]méthyl} amino)-4-oxo butanoïque | 795 | 57 | > 10µg/ml | - | 23 | - |
| 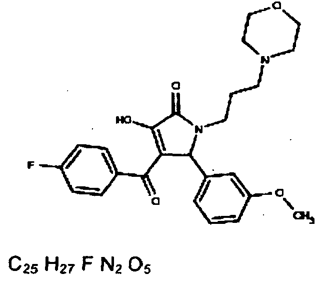 | 6905 988 | 4-(4-fluoro benzoyl)-3-hydroxy-5-(3-méthoxy phényl)-1-[3-(4-morpholinyl)pr opyl]-1,5-dihydro-2H-pyrrol-2-one | 843 | 57 | >10µg/ml | - | 24 | I |
| 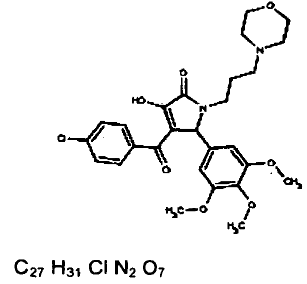 | 6885 782 | 4-(4-chloro benzoyl)-3-hydroxy-1-[3-(4-morpholinyl)pr opyl]-5-(3,4,5-triméthoxy phényl)-1,5-dihydro-2H-pyrrol-2-one | 249 | 56,5 | >10µg/ml | - | 25 | I |
| 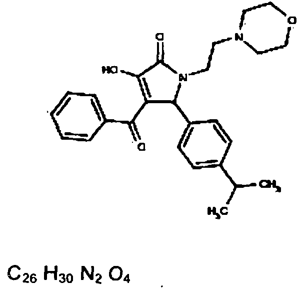 | 6663 684 | 4-benzoyl-3-hydroxy-5-(4-isopropyl phényl)-1-[2-(4-morpholinyl)ét hyl]-1,5-dihydro-2H-pyrrol-2-one | 530 | 56,5 | >10µg/ml | - | 26 | I |
| 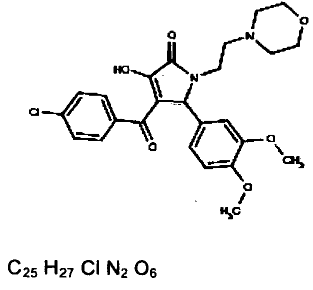 | 6672 500 | 4-(4-chloro benzoyl)-5-(3,4-diméthoxy phényl)-3-hydroxy-1-[2-(4-morpholinyl)ét hyl]-1,5-dihydro-2H-pyrrol-2-one | 194 | 55 | >10µg/ml | - | 27 | I |
| 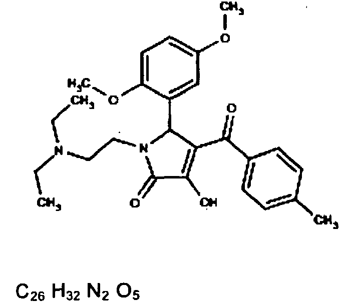 | 7721 949 | 1-[2-(diméthyl amino)éthyl]-5-(2,5-diméthoxy phényl)-3-hydroxy-4-(4-méthyl benzoyl)-1,5-dihydro-2H-pyrrol-2-one | 139 | 54 | >10µg/ml | - | 28 | I |
| 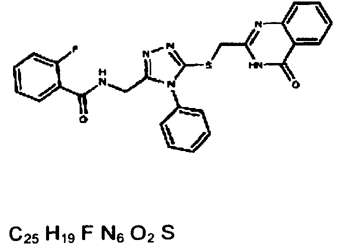 | 7966 545 | 2-fluoro-N-[(5-{[(4-oxo-3,4-dihydro-2-quinazolinyl)m éthyl]thio}-4-phényl-4H-1,2,4-triazol-3-yl)méthyl] benzamide | 773 | 53,5 | ~10µg/ml | 6,7 µM | 29 | - |
| 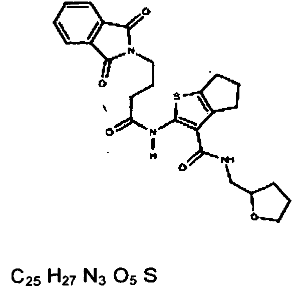 | 7437 580 | 2-{[4-(1,3-dioxo-1,3-dihydro-2H-isoindol-2-yl)butanoyl] amino}-N-(tetrahydro-2-furanyl méthyl)-5,6-dihydro-4H-cyclopenta[b]t hiophène-3-carboxamide | 222 | 51 | >10µg/ml | - | 30 | - |
| 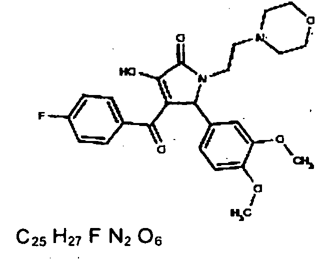 | 6654 239 | 5-(3,4-diméthoxy phényl)-4-(4-fluoro benzoyl)-3-hydroxy-1-[2-(4-morpholinyl)ét hyl]-1,5-dihydro-2H-pyrrol-2-one | 343 | 50 | >10µg/ml | - | 31 | I |
| 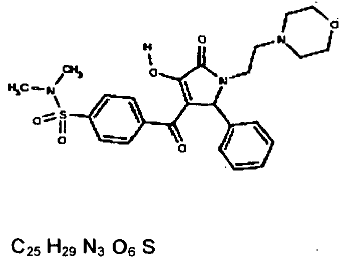 | 6670 570 | 4-({4-hydroxy-1-[2-(4-morpholinyl)ét hyl]-5-oxo-2-phényl-2,5-dihydro-1H-pyrrol-3-yl} carbonyl)-N,N-diméthyl benzène sulfonamide | 926 | 50 | >10µg/ml | - | 32 | I |
| 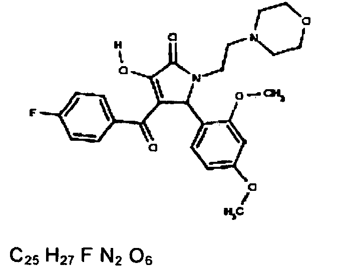 | 6670 673 | 5-(2,4-diméthoxy phényl)-4-(4-fluorobenzoyl) -3-hydroxy-1-[2-(4-morpholinyl)ét hyl]-1,5-dihydro-2H-pyrrol-2-one | 742 | 50 | >10µg/ml | - | 33 | I |
| 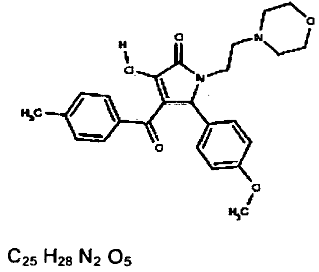 | 6670 747 | 3-hydroxy-5-(4-méthoxy phényl)-4-(4-méthyl benzoyl)-1-[2-(4-morpholinyl)ét hyl]-1,5-dihydro-2H-pyrrol-2-one | 413 | 50 | >10µg/ml | - | 34 | I |
| 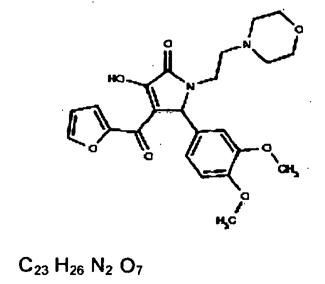 | 6671 401 | 5-(3,4-diméthoxy phényl)-4-(2-furoyl)-3-hydroxy-1-[2-(4-morpholinyl)ét hyl]-1,5-dihydro-2H-pyrrol-2-one | 954 | 50 | >10µg/ml | - | 35 | I |
| 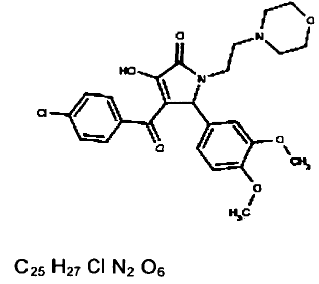 | 6672 500 | 4-(4-chloro benzoyl)-5-(3,4-diméthoxy phényl)-3-hydroxy-1-[2-(4-morpholinyl)ét hyl]-1,5-dihydro-2H-pyrrol-2-one | 194 | 50 | >10µg/ml | - | 36 | I |
| 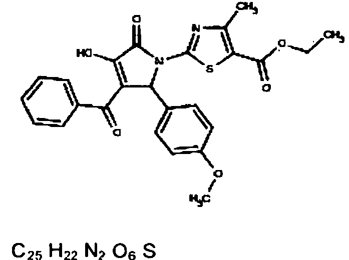 | 6673 225 | éthyl 2-[3-benzoyl-4-hydroxy-2-(4-méthoxy phényl)-5-oxo-2,5-dihydro-1H-pyrrol-1-yl]-4-méthyl-1,3-thiazole-5-carboxylate | 124 | 50 | >10µg/ml | - | 37 | I |
| 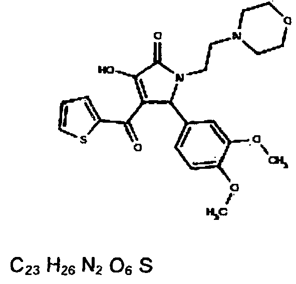 | 6677 533 | 5-(3,4-diméthoxy phényl)-3-hydroxy-1-[2-(4-morpholinyl)ét hyl]-4-(2-thiényl carbonyl)-1,5-dihydro-2H-pyrrol-2-one | 409 | 50 | >10µg/ml | - | 38 | I |
| 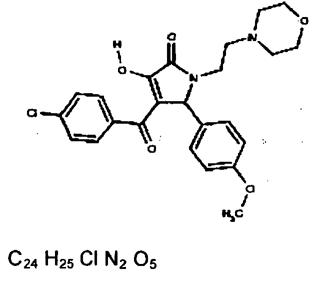 | 6683 618 | 4-(4-chloro benzoyl)-3-hydroxy-5-(4-méthoxy phényl)-1-[2-(4-morpholinyl)ét hyl]-1,5-dihydro-2H-pyrrol-2-one | 808 | 50 | >10µg/ml | - | 39 | I |
| 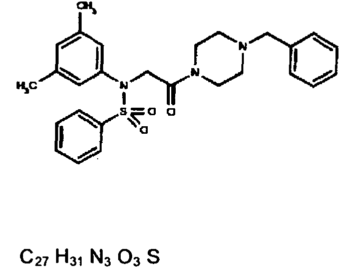 | 6417 902 | N-[2-(4-benzyl-1-piperazinyl)-2-oxoéthyl]-N-(3,5-diméthyl phényl) benzène sulfonamide | 897 | 49,5 | >10µg/ml | - | 40 | III |
| 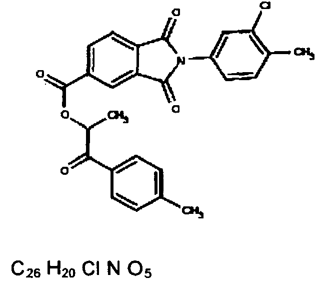 | 6437 157 | 1-méthyl-2-(4-méthyl phényl)-2-oxoéthyl 2-(3-chloro-4-méthyl phényl)-1,3-dioxo-5-isoindoline carboxylate | 871 | 49,5 | 10µg/ml | - | 41 | - |
| 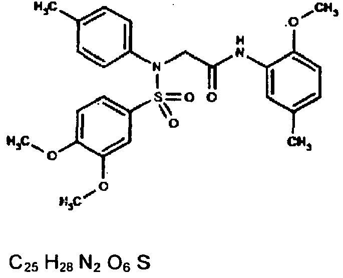 | 6465 972 | N~2~-[(3,4-diméthoxy phényl) sulfonyl]-N~1~-(2-méthoxy-5-méthylphényl) -N~2~-(4-méthylphényl) glycinamide | 926 | 49 | >10µg/ml | - | 42 | III |
| 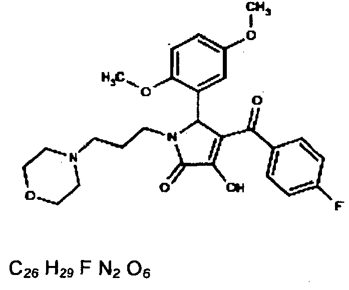 | 7723 671 | 5-(2,5-diméthoxy phényl)-4-(4-fluoro benzoyl)-3-hydroxy-1-[3-(4-morpholinyl)pr opyl]-1,5-dihydro-2H-pyrrol-2-one | 34 | 48,5 | >10µg/ml | - | 43 | I |
| 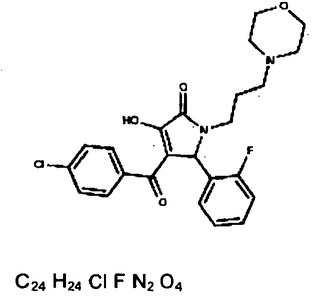 | 6648 368 | 4-(4-chlorobenzoyl )-5-(2-fluorophényl)-3-hydroxy-1-[3-(4-morpholinyl)pr opyl]-1,5-dihydro-2H-pyrrol-2-one | 465 | 48,5 | >10µg/ml | - | 44 | I |
| 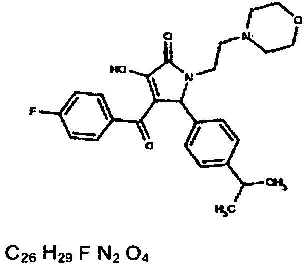 | 6656 195 | 4-(4-fluoro benzoyl)-3-hydroxy-5-(4-isopropyl phényl)-1-[2-(4-morpholiny/)ét hyl]-1,5-dihydro-2H-pyrrol-2-one | 613 | 48,5 | >10µg/ml | - | 45 | I |
| 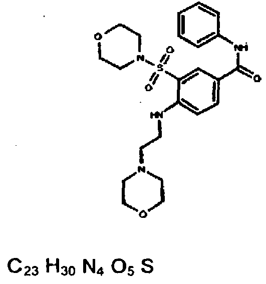 | 7778 331 | 4-{[2-(4-morpholinyl)ét hyl]amino}-3-(4-morpholinyl sulfonyl)-N-phénylbenza mide | 998 | 48 | >10µg/ml | 3 µM | 46 | - |
| 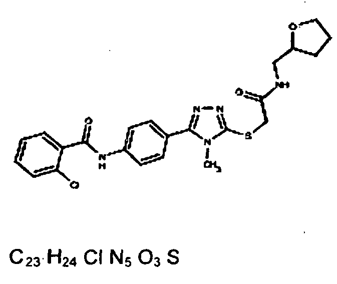 | 6994 060 | 2-chloro-N-{4-[4-méthyl-5-({2-oxo-2-[(tetrahydro-2-furanyl méthyl) amino] éthyl}thio)-4H-1,2,4-triazol-3-yl]phényl} benzamide | 623 | 47,5 | >10µg/ml | - | 47 | II |
| 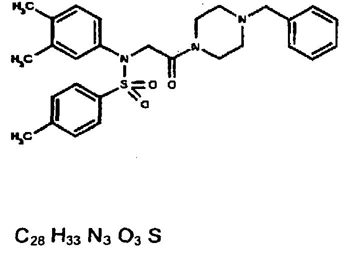 | 6458 830 | N-[2-(4-benzyl-1-piperazinyl)-2-oxoéthyl]-N-(3,4-diméthyl phényl)-4-méthyl benzène sulfonamide | 837 | 47 | >10µq/ml | - | 48 | III |
| 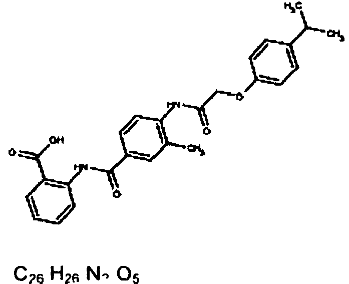 | 7524 107 | acide 2-[(4-{[(4-isopropyl phénoxy) acétyl]amino}-3-méthyl benzoyl) amino] benzoïque | 789 | 46,5 | ~10µg/ml | 5,5 µM | 49 | - |
| 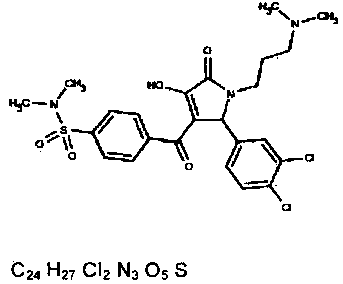 | 6661 524 | 4-({2-(3,4-dichloro phényl)-1-[3-(diméthyl amino)propyl]-4-hydroxy-5-oxo-2,5-dihydro-1H-pyrrol-3-yl} carbonyl)-N,N-diméthyl benzène sulfonamide | 428 | 46,5 | >10µg/ml | - | 50 | I |
| 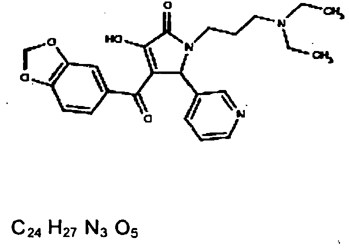 | 7739 436 | 4-(1,3-benzodioxol-5-yl carbonyl)-1-[3-(diéthyl amino)propyl]-3-hydroxy-5-(3-pyridinyl)-1,5-dihydro-2H-pyrrol-2-one | 976 | 46 | >10µg/ml | - | 51 | I |
| 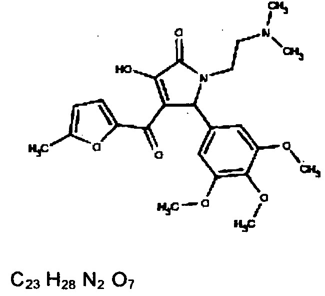 | 6881 804 | 1-[2-(diméthyl amino)éthyl]-3-hydroxy-4-(5-méthyl-2-furoyl)-5-(3,4,5-triméthoxy phényl)-1,5-dihydro-2H-pyrrol-2-one | 230 | 46 | >10µg/ml | - | 52 | I |
| 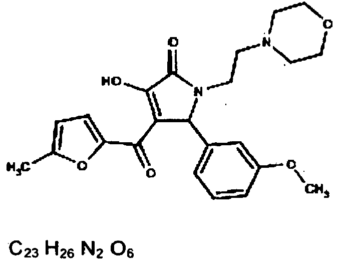 | 6907 921 | 3-hydroxy-5-(3-méthoxy phényl)-4-(5-méthyl-2-furoyl)-1-[2-(4-morpholinyl)ét hyl]-1,5-dihydro-2H-pyrrol-2-one | 609 | 45,5 | >10µg/ml | - | 53 | I |
| 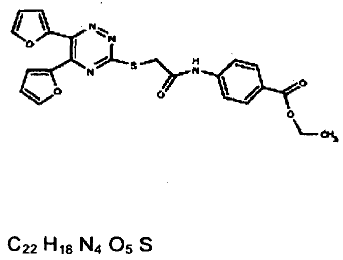 | 7495 334 | éthyl 4-({[(5,6-di-2-furyl-1,2,4-triazin-3-yl)thio]acétyl}a mino) benzoate | 380 | 43 | 1,6 ~5µg/ml | 1,6 µM | 54 | |
| 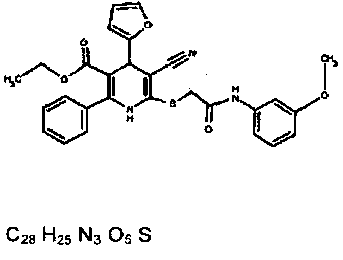 | 7509 862 | éthyl 5-cyano-4-(2-furyl)-6-({2-[(3-méthoxy phényl) amino]-2-oxoéthyl} thio)-2-phényl-1,4-dihydro-3-pyridine carboxylate | 170 | 42,5 | ~5µg/ml | - | 55 | - |
| 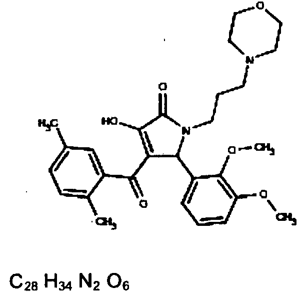 | 7325 385 | 5-(2,3-diméthoxy phényl)-4-(2,5-diméthyl benzoyl)-3-hydroxy-1-[3-(4-morpholinyl)pr opyl]-1,5-dihydro-2H-pyrrol-2-one | 65 | 42 | ~10µg/ml | - | 56 | I |
| 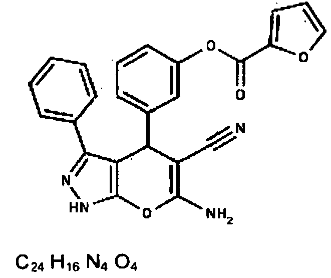 | 6669 449 | 3-(6-amino-5-cyano-3-phényl-1,4-dihydro pyrano[2,3-c]pyrazol-4-yl)phényl 2-furoate | 140 | 42 | >10µg/ml | - | 57 | - |
| 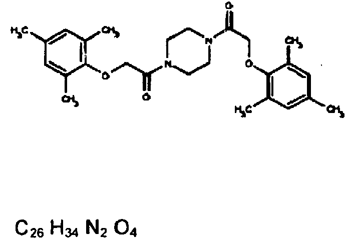 | 7348 779 | 1,4-bis [(mésityloxy)a cétyl] pipérazine | 209 | 40 | ~10µg/ml | - | 58 | - |
| 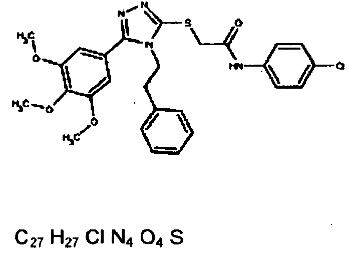 | **7501 888** | **N-(4-chloro phényl)-2-{[4-(2-phényl éthyl)-5-(3,4,5-triméthoxy phényl)-4H-1,2,4-triazol-3-yl]thio} acétamide** | 544 | 40 | ~2µg/ml | 8,2 µM | **59** | - |
| 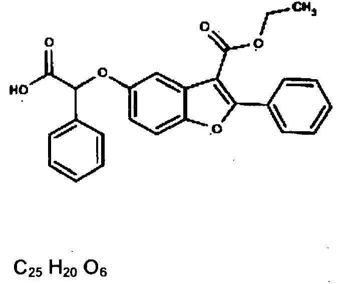 | 6946 138 | acide {[3-(éthoxy carbonyl)-2-phényl-1-benzofuran-5-yl]oxy} (phényl) acétique | 298 | 40 | >10µg/ml | - | 60 | - |
| 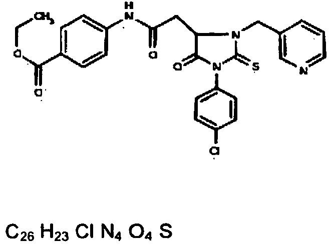 | **7722 851** | **éthyl 4-({[1-(4-chloro phényl)-5-oxo-3-(3-pyridinyl méthyl)-2-thioxo-4-imidazolidiny l]acétyl} amino) benzoate** | 638 | 39,5 | ~2µg/ml | 21,8 µM | **61** | **IV** |
| 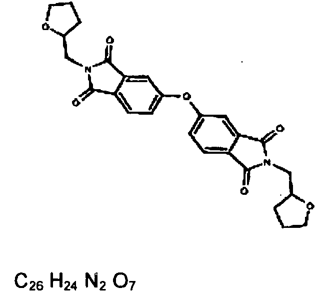 | 7517 583 | 5,5'-oxybis [2-(tétra hydro-2-furanyl méthyl)-1 H-isoindole-1,3(2H)-dione] | 912 | 38,5 | >10µg/ml | - | 62 | - |
| 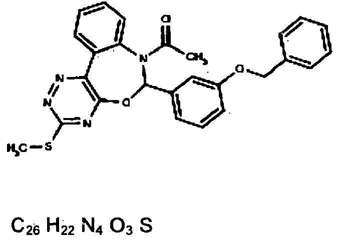 | 6634 701 | 7-acétyl-6-[3-(benzyloxy) phényl]-3-(méthylthio)-6,7-dihydro [1,2,4] triazino[5,6-d][3,1] benzoxazepin e | 934 | 38,5 | ∼5µg/ml | - | 63 | - |
| 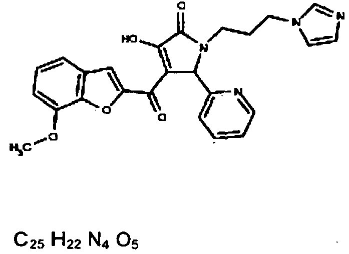 | 7726 450 | 3-hydroxy-1-[3-(1 H-imidazol-1-yl)propyl]-4-[(7-méthoxy-1-benzofuran-2-yl) carbonyl]-5-(2-pyridinyl)-1,5-dihydro-2H-pyrrol-2-one | 472 | 37,5 | >10µg/ml | - | 64 | I |
| 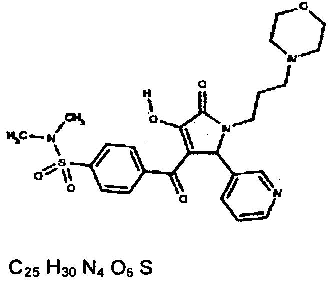 | 6662 088 | 4-{[4-hydroxy-1-[3-(4-morpholinyl)pr opyl]-5-oxo-2-(3-pyridinyl)-2,5-dihydro-1 H-pyrrol-3-yl]carbonyl}-N,N-diméthyl benzène sulfonamide | 156 | 37 | >10µg/ml | - | 65 | I |
| 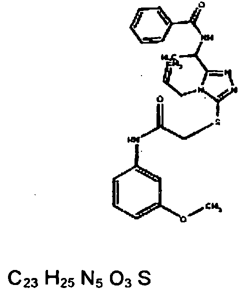 | 7752 193 | N-{1-[4-allyl-5-({2-[(3-méthoxy phényl) amino]-2-oxoéthyl} thio)-4H-1,2,4-triazol-3-yl]éthyl} benzamide | 248 | 35 | ~5µg/ml | 8 µM | 66 | |
| 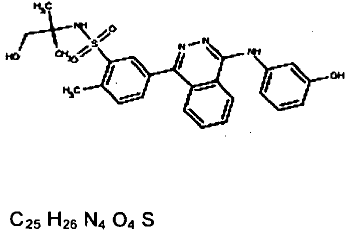 | 7238 569 | N-(2-hydroxy-1,1-diméthyléthyl) -5-{4-[(3-hydroxy phényl) amino]-1-phthalazinyl}-2-méthyl benzène sulfonamide | 749 | 32 | >10µg/ml | - | 67 | - |
| 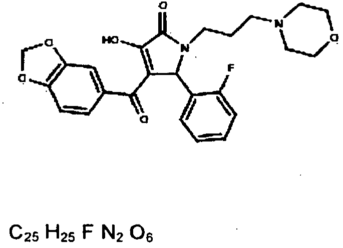 | 7724 000 | 4-(1,3-benzodioxol-5-ylcarbonyl)-5-(2-fluorophényl)-3-hydroxy-1-[3-(4-morpholinyl)pr opyl]-1,5-dihydro-2H-pyrrol-2-one | 303 | 30 | ~10µg/ml | - | 68 | I |
| 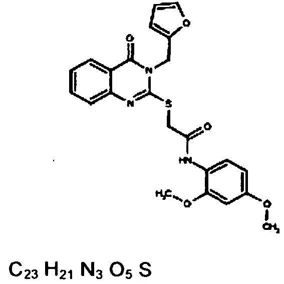 | 7443 270 | N-(2,4-diméthoxyphé nyl)-2-{[3-(2-furylméthyl)-4-oxo-3,4-dihydro-2-quinazolinyl] thio} acétamide | 895 | 30 | ~5µg/ml | - | 69 | - |
| 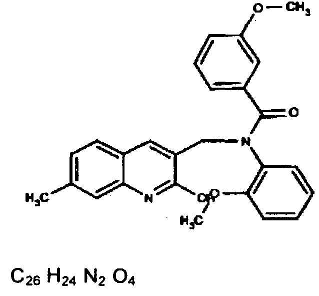 | 7245 019 | N-[(2-hydroxy-7-méthyl-3-quinolinyl) méthyl]-3-méthoxy-N-(2-méthoxy phényl) benzamide | 608 | 30 | >10µg/ml | - | 70 | - |
| 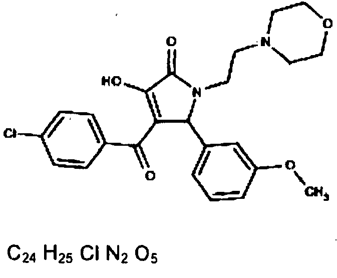 | 6909 597 | 4-(4-chlorobenzoyl )-3-hydroxy-5-(3-méthoxy phényl)-1-[2-(4-morpholinyl)ét hyl]-1,5-dihydro-2H-pyrrol-2-one | 790 | 29 | >10µg/ml | - | 71 | I |
| 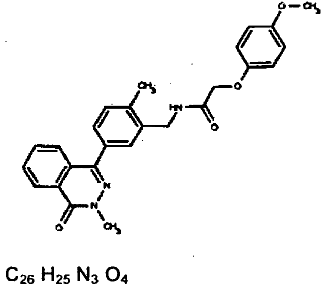 | 7661 882 | 2-(4-méthoxy phénoxy)-N-[2-méthyl-5-(3-méthyl-4-oxo-3,4-dihydro-1-phthalazinyl)b enzyl] acétamide | 941 | 28 | ~1µg/ml | - | 72 | - |
| 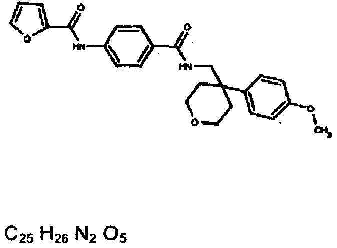 | 7667 791 | N-{4-[({[4-(4-méthoxy phényl) tétrahydro-2H-pyran-4-yl] méthyl} amino) carbonyl] phényl}-2-furamide | 797 | 28 | > 10µg/ml | - | 73 | - |
| 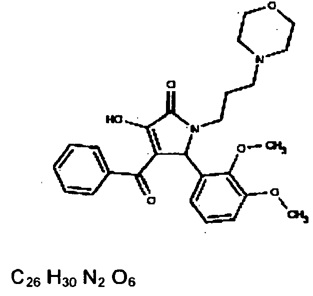 | 6891 745 | 4-benzoyl-5-(2,3-diméthoxy phényl)-3-hydroxy-1-[3-(4-morpholinyl)pr opyl]-1,5-dihydro-2H-pyrrol-2-one | 115 | 27,5 | >10µg/ml | - | 74 | I |
| 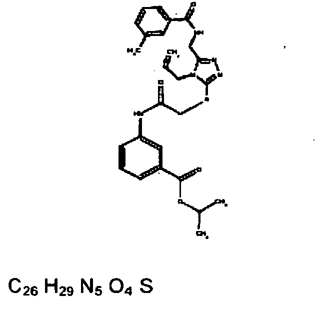 | 7783 660 | isopropyl 3-({[(4-allyl-5-{[(3-méthyl benzoyl) amino] méthyl}-4H-1,2,4-triazol-3-yl) thio]acétyl) amino) benzoate | 266 | 27 | ~µg/ml | 4,8 µM | 75 | II |
| 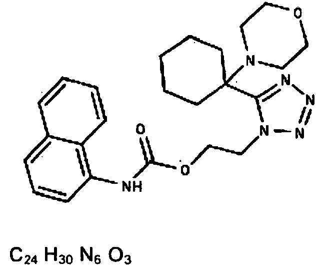 | 7653 478 | 2-{5-[1-(4-morpholinyl)cy clohexyl]-1H-tétrazol-1-yl}éthyl 1-naphthyl carbamate | 365 | 27 | > 10µg/ml | - | 76 | - |
| 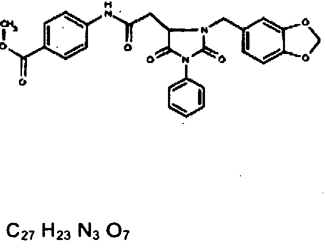 | 7723 330 | méthyl 4-({[3-(1,3-benzodioxol-5-ylméthyl)-2,5-dioxo-1-phényl-4-imidazol idinyl]acétyl}a mino) benzoate | 965 | 26 | >10µg/ml | - | 77 | IV |
| 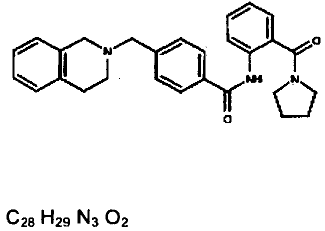 | 7199 725 | 4-(3,4-dihydro-2(1H)-isoquinolinylm éthyl)-N-[2-(1-pyrrolidinyl carbonyl) phényl] benzamide | 987 | 25 | >10µg/ml | - | 78 | - |
| 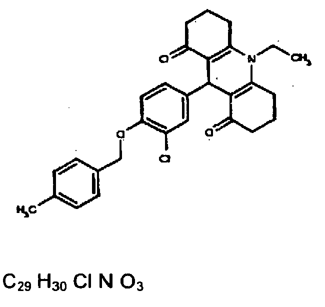 | 6987 235 | 9-{3-chloro-4-[(4-méthyl benzyl)oxy] phényl}-10-éthyl-3,4,6,7,9,10-hexahydro-1,8(2H,5H)-acridine dione | 680 | 25 | >10µg/ml | - | 79 | - |
| 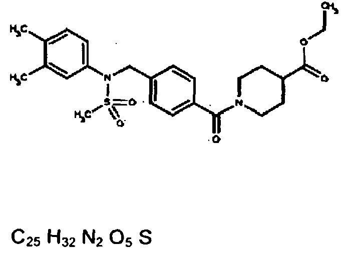 | 7140 931 | éthyl 1-(4-{[(3,4-diméthyl phényl) (méthyl sulfonyl) amino] méthyl} benzoyl)-4-piperidine carboxylate | 14 | 24.5 | >10µg/ml | - | 80 | - |
| 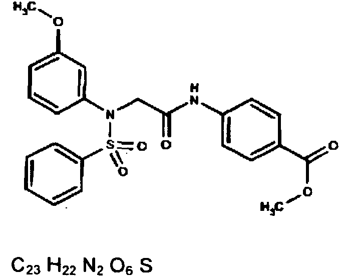 | 7787 455 | méthyl 4-{[N-(3-méthoxy phényl)-N-(phényl sulfonyl) glycyl] amino} benzoate | 407 | 20 | >10µg/ml | - | 81 | III |
| 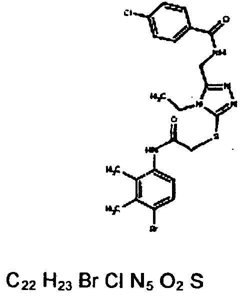 | 7660 465 | N-{[5-({2-[(4-bromo-2,3-diméthyl phényl) amino]-2-oxoéthyl} thio)-4-éthyl-4H-1,2,4-triazol-3-yl]méthyl}-4-chloro benzamide | 881 | 20 | ~7µg/ml | 3,2 µM | 82 | II |
| 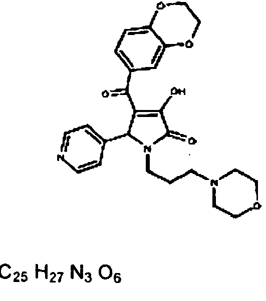 | 7661 751 | 4-(2,3-dihydro-1,4-benzodioxin-6-yl carbonyl)-3-hydroxy-1-[3-(4-morpholinyl)pr opyl]-5-(4-pyridinyl)-1,5-dihydro-2H-pyrrol-2-one | 951 | 19 | ~10µg/ml | - | 83 | I |
| 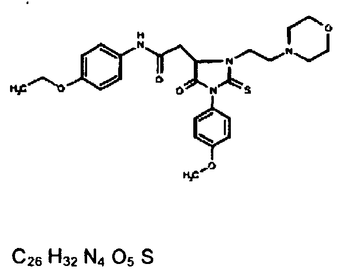 | 7722 914 | N-(4-ethoxy phényl)-2-{1-(4-méthoxy phényl)-3-[2-(4-morpholinyl)ét hyl]-5-oxo-2-thioxo-4-imidazol idinyl}acétami de | 806 | 19 | ~7µg/ml | 15,6 µM | 84 | IV |
| 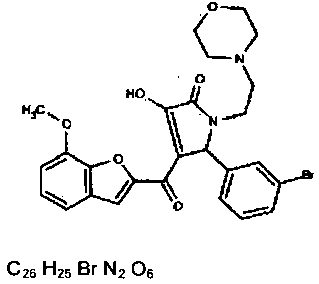 | 7745 040 | 5-(3-bromo phényl)-3-hydroxy-4-[(7-méthoxy-1-benzo furan-2-yl) carbonyl]-1-[2-(4-morpholinyl)ét hyl]-1,5-dihydro-2H-pyrrol-2-one | 313 | 18 | ~1µg/ml | - | 85 | I |
| 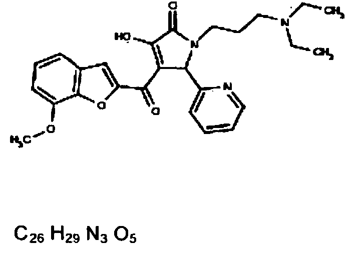 | 7735 385 | 1-[3-(diéthyl amino)propyl]-3-hydroxy-4-[(7-méthoxy-1-benzofuran-2-yl) carbonyl]-5-(2-pyridinyl)-1,5-dihydro-2H-pyrrol-2-one | 21 | 17 | ~10µg/ml | - | 86 | I |
| 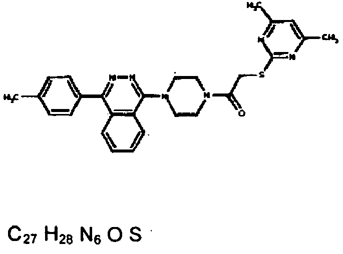 | 7756 003 | 1-(4-{[(4,6-diméthyl-2-pyrimidinyl) thio]acétyl}-1-piperazinyl)-4-(4-méthyl phényl) phthalazine | 361 | 11 | ~5µg/ml | - | 87 | |

| | | | | | | | | |
|---|---|---|---|---|---|---|---|---|
| - ***Ref CB* :** *Référence ChemBridge ;* - ***Rang :** rang de chaque molécule sur la liste des 1000 meilleures molécules après docking in silico ;* - ***Inhib. In vit. :*** % *moyen d'inhibition obtenu avec chaque molécule pour le déplacement de la liaison du scFv* G4G11 *avec Syk in vitro dans le procédé ADA;* - ***CI50 dégran. :** concentration inhibant de 50% la dégranulation des mastocytes ;* - ***Kd :** constante de dissociation vis-à-vis de Syk mesurée in vitro par spectrofluorométrie* - ***N° :** numéro attribué par les inventeurs ;* - ***Gp :** groupes auxquels appartiennent les molécules.* | | | | | | | | |

### Exemple 3 - Matériels et méthodes.

**1) Produits chimiques et anticorps.** Une banque chimique de 3000 molécules (un sous-ensemble varié) a été acquise auprès de ChemBridge, Inc. (San Diego, CA). Des stocks de solutions de petites molécules ont été préparés à raison de 10 mM dans du DMSO (diméthylsulfoxyde), sauf pour C-13 (méthyl 2-{5-[(3-benzyl-4-oxo-2-thioxo-1,3-thiazolidin-5-ylidène)méthyl]-2-furyl}benzoate, ChemBridge numéro ID 6197026) préparé dans du DMF (diméthylformamide). Sauf indication contraire, tous les réactifs proviennent de Sigma. L'haptène dinitrophényl (DNP) a été acquis auprès de Calbiochem. La Sépharose GammaBind G et tous les anticorps secondaires proviennent de GE Health Amersham Biosciences. Les anticorps anti-Syk, anti-Lyn, anti-Btk, anti-PLC-y1, anti-PLC-y2, anti-LAT, anti-SLP-76, anti-p38, anti-JNK, anti-Vav, anti-Akt1 et 9E10 conjugé à HRP ont été acquis auprès de Santa Cruz Biotechnology. Les anticorps anti-phospho-p44/42 MAP Kinase, anti-p44/42 MAP Kinase, anti-phospho-p38, anti-phospho-JNK, anti-phospho-Akt1 ont été acquis auprès de Cell Signaling. Les anticorps anti-phospho-LAT et anti-phospho-PLC-y1 proviennent de Biosource et les anticorps anti-phospho-SLP-76 de BD Pharmingen. L'anticorps monoclonal 4G10 anti-phosphotyrosine a été acquis auprès de Upstate Biotechnology.
**2) Procédé ADA : test de criblage de molécules à haut débit de type ELISA, basé sur le déplacement d'un anticorps (**WO 2005106481**).** La protéine de fusion GST:Syk 6-242 recombinante ⁸ comprenant les résidus 6 à 242 de la protéine tyrosine kinase Syk murine illustrée à la figure 8B (SEQ ID N°4) a été immobilisée sur une plaque ELISA à une concentration finale de 10 µg ml⁻¹. Pour le criblage de la banque de molécules chimiques, les petites molécules, diluées dans du PBS à une concentration finale de 10 µM ont été ajoutées dans les puits pendant une heure à température ambiante, avant ajout du fragment scFv G4G11 à une concentration finale de 100 nM pour une heure supplémentaire. La liaison de G4G11 à Syk a été évaluée en ajoutant l'anticorps monoclonal 9E10 conjugué à HRP qui détecte la séquence d'acides aminés EQKLISEEDLN de la protéine c-myc humaine localisée à l'extrémité C-terminale du fragment scFv. Pour générer des mutants de Syk, la mutagenèse dirigée a été utilisée sur la protéine GST:Syk 6-242 et la liaison de G4G11 aux mutants a été évaluée en présence de 5 µM de C-13.
**3) Cellules, conditions de culture et essais fonctionnels.** L'anticorps monoclonal IgE de souris 2682-I anti-DNP a été utilisée comme surnageant de culture des hybridomes contenant 1 µg/ml d'IgE. Des cellules de la moelle épinière fémorale ont été prélevées et cultivées dans un milieu Opti-MEM (Gibco) additionné de 10% de sérum foetal bovin et 4% de surnageant de transfectants X63 secrétant l'IL-3 murine. Des cellules basophiles leucémiques de rat RBL-2H3 (ATCC) ont été maintenues en culture monocouche dans un milieu RPMI 1640 additionné de 10% de sérum foetal bovin (Gibco). Les mesures de la libération de β-hexosaminidase par les cellules RBL-2H3 ont été effectuées comme précédemment décrit ⁷, à l'exception du fait qu'après 12-16 h d'incubation avec l'IgE anti-DNP (0,5µg/ml), les cellules ont été incubées 90 min à 37°C dans du milieu RPMI additionné avec les concentrations indiquées de C-13 ou de DMF (0,25%). Les cellules ont été stimulées pendant 45 min avec du DNP-BSA (50 ng ml⁻¹) ou de l'ionomycine (1.5 µM). Les cellules BMMC ont été incubées pendant une heure à 37°C avec l'IgE anti-DNP (100ng/ml). Elles ont ensuite été incubées avec C-13 (3 µM) ou du DMF (0,3%) pendant 3 heures à 37°C, et stimulées avec des concentrations variées de DNP-BSA. Le niveau de libération de la β-hexosaminidase a été mesuré 10 min plus tard et la titration de TNF-α a été réalisée par un essai de cytotoxicité sur des cellules L929 comme précédemment décrit ¹⁰, 3 heures après la stimulation. Les résultats illustrés sur la figure 3 sont représentatifs de trois expériences indépendantes.
**4) Immunoprécipitations, essais kinase *in vitro* et immunodétection.** Toutes les expériences ont été réalisées tel que précédemment décrit ⁷, à l'exception du fait qu'avant la stimulation avec le DNP-BSA (50 ng ml⁻¹, 3 min), les cellules RBL-2H3 ont été incubées pendant 90 min à 37°C dans du milieu RPMI additionné avec les concentrations indiquées de C-13 ou DMF. Les cellules ont été solubilisées dans du tampon de lyse modifié DOC (1% NP-40, 0.25% déoxycholate de sodium, 0.1% SDS dans du tampon PBS additionné d'inhibiteurs de protéases et de phosphatases) et la concentration protéique a été déterminée (Essai Protéine BCA, PIERCE). Pour les immunoprécipitations, des lysats de cellules non stimulées et stimulées avec IgE/DNP ont été incubées avec des complexes préformés d'anticorps et de Sépharose GammaBind G, et l'activité kinase *in vitro* des immunoprécipitats Syk, Btk and Lyn ont a été examinée. Avant la séparation par gel SDS-PAGE, les lysats ou immunoprécipitats ont été préparés par l'addition de tampon d'échantillon SDS (60 mM Tris, pH 6.8, 2.3% SDS, 10% glycérol, 0.01% bleu de bromophénol). Les protéines ont été transférées sur une membrane de nitrocellulose (Schleicher & Schuell), et détectées par des anticorps appropriés et le système de chimioluminescence amélioré (ExactaCruz, Santa Cruz Biotechnology).
**5) Analyse par cytométrie de flux du niveau de mobilisation du calcium et de l'expression membranaire de FCεRI.** La concentration intracellulaire en calcium libre a été déterminée en chargeant au préalable 1x10⁶ cellules avec 5 mM de Fluo-3 AM (Molecular Probes, Invitrogen) en présence de 0.2% de Pluronic F-127 pendant 30 min à température ambiante. Préalablement à la stimulation avec du DNP-BSA ou de l'ionomycine, les cellules ont été traitées pendant 90 min à 37°C dans un milieu RPMI additionné avec C-13 ou DMF (0,25%), et la concentration intracellulaire en calcium libre a été mesurée avec un cytomètre de flux (Beckton Dickinson). Pour l'évaluation de l'expression en surface de FcεRI, les cellules ont été incubées pendant 2 h à 37°C avec l'IgE anti-DNP. L'IgE lié à la membrane a été détectée en utilisant des Ig biotinylées anti-souris, puis de la streptavidine conjuguée au Fitc.
**6) Induction de l'anaphylaxie.** Des souris femelles BALB/c (âgées de 6-8 semaines) ont été acquises auprès de Charles River et conservées à l'animalerie de l'IRCM dans des conditions sans germes pathogènes. Les protocoles pour l'anaphylaxie passive systémique (PSA) et l'anaphylaxie passive cutanée (PCA) IgE-dépendantes ont été menés comme précédemment décrit¹¹. Brièvement, les souris ont reçu par injection intraveineuse 100 µg d'IgE (SPE-7, Sigma) dans 200 µl de PBS pour la PSA, ou par injection intradermale 25 ng d'IgE 10 µl de PBS pour la PCA, et ont été stimulées 24h plus tard par injection intraveineuse de 1 mg de DNP-KLH dans 2% de bleu d'Evans. C-13, une molécule chimique non pertinente ou le véhicule ont été administrées 1 heure avant la stimulation, soit par voie orale (PSA) dans 200 µl de carboxyméthylcellulose à 1%, ou localement au niveau de l'oreille par injection intra-dermale(PCA) dans un mélange d'acétone/huile d'olive (4:1). Les animaux ont été sacrifiés 20 min après la stimulation. Les oreilles ont été prélevées, hachées et le bleu d'Evans a été extrait après incubation une nuit dans du formamide à 80°C. Pour les mesures de températures dans la PSA, C-13 (100mg/kg) ou le véhicule ont été administrés oralement, 3 heures avant la stimulation réalisée en l'absence de bleu d'Evans. La température a été mesurée à l'aide d'un thermomètre électronique avec une sonde rectale (YSI, Yellow Springs, OH) avant la stimulation et durant 60 minutes après, avant le sacrifice. L'absorbance a été mesurée à 610 nm. Les expériences ont été réalisées avec 4-5 souris par condition. Les données illustrées dans la figure 4 sont représentatives de trois expériences différentes.
7) Etudes structurales. Les poches tridimensionnelles susceptibles d'être des cibles pharmaceutiques ont été prédites en utilisant Q-SiteFinder ¹² et ICM ¹³. Le docking de la molécule C-13 a été réalisé en utilisant LigandFit ¹⁴ et Surflex ¹⁵. Les 20 premières positions ont été analysées et une position consensus est présentée à la Fig. 1A. Les images ont été générées avec PyMol.
**8) Péritonite (****Fig. 5****).** Le recrutement péritonéal Syk-dépendant des neutrophiles a été induit chez des souris BALB/c femelles âgées de 8 semaines comme décrit dans ³⁶ par injection intraveineuse de 4µg de toxine *Bordetella pertuissis* (don du Dr D. Raze, Inserm, Lille, France) et 2 heures plus tard par injection intrapéritonéale de 4% de thioglycollate dans de l'eau stérile. Un lavage péritonéal a été réalisé avec 5ml de PBS 4 heures plus tard et le nombre total de neutrophiles a été déterminé après marquage avec anti-Gr1 conjugué à APC (Becton-Dickinson) et analyse par cytométrie de flux. C-13 (100 mg/kg) dans CMC ou le véhicule seul a été administré oralement une heure avant l'injection de la toxine *Bordetella pertuissis.*
**9) Purification et prolifération *in vitro* des Lymphocytes B.** Les lymphocytes B spléniques ont été purifiés à partir de souris BALB/c femelles âgées de 8 semaines sur des billes magnétiques par sélection négative utilisant des micro billes revêtues de CD43 et des colonnes LS (Miltenyi Biotec) comme précédemment décrit³⁷. Après deux heures d'incubation avec des concentrations variables de C-13 ou du véhicule, 50.000 cellules ont été cultivées pendant 48 heures dans des plaques de 96 puits en présence ou en absence de 10 µg/ml de fragment F(ab')2 d'âne anti-lgM de souris (Jacson Immunoresearch). Du bleu d'Alamar (Serotec) a été ajouté aux cultures 24 avant la mesure des formes réduites *versus* oxydées du réactif à 570 et 620 nm, conformément aux instructions du fabriquant.
**10) Production d'anticorps (****Fig. 5C****).** Cette expérience a été réalisée comme décrit dans³⁸. Des souris BALB/c femelles âgées de 8 semaines ont reçu une administration orale de C-13 (150 mg/kg) dans 1% CMC ou le véhicule seul, été ont été immunisées 3 heures plus tard par une injection intrapéritonéale de 10 µg de trinitophényl-hémocyanine de patelle (TNP-KLP) dans du Rehydragel alum (Reheiss). Le sérum a été collecté avant immunisation et au jour 12. Les niveaux d'immunoglobulines spécifiques des antigènes ont été mesurés par ELISA avec 10 µg/ml de TNP-OVA lié aux plaques (Biosearch technologies) en tant qu'agent de capture. Les niveaux d'IgM, IgG1, IgG2a, IgG2b, IgG3 et IgA ont été mesurés à partir d'échantillons dilués au 1:5000 en utilisant des anticorps de chèvre conjugués à la péroydase et isotype spécifiques (Southern Biotechnology), tandis que les niveaux d'IgE ont été mesurés à partir d'échantillons dilués au 1:50 en utilisant des anticorps de rat anti-IgE de souris biotinylés (Becton Dickinson) et de la spreptavidine conjuguée à la péroxydase (R&D Systems). Après incubation avec le substrat TMB, la densité optique (OD) a été mesurée à 450nm.
**11) Analyses statistiques.** Les données numériques moyennes sont exprimées en tant que moyennes ± écarts-types (SD). Le test t de Student a été utilisé pour déterminer la signification statistique des différences entre groupes.
**12) Test de toxicité de C-13 sur les cellules BMMC.** Afin d'évaluer l'éventuel effet toxique de C-13 sur les mastocytes, les cellules BMMC ont été incubées durant 5 jours à 37°C en présence de 2.5 µM ou 5 µM de C-13, ou de DMF à 0.25% (correspondant à la concentration de DMF utilisée avec 5 µM de C-13) dans les mêmes conditions que pour les tests fonctionnels. Un double marquage à l'Annexine-V et Iodure de Propidium a permis de mettre en évidence le niveau de viabilité des cellules BMMC après 3 heures et après 5 jours. La viabilité des cellules of BMMC traitées dans les mêmes conditions avec la Staurosporine a été mesurée dans les mêmes conditions.

### Références

1. Kambayashi T, Koretzky GA. Proximal signaling events in Fc epsilon RI-mediated mast cell activation. J Allergy Clin Immunol 2007; 119:544-52; quiz 53-4.
2. Rivera J, Gilfillan AM. Molecular regulation of mast cell activation. J Allergy Clin Immunol 2006; 117:1214-25; quiz 26.
3. Costello PS, Turner M, Walters AE, Cunningham CN, Bauer PH, Downward J, et al. Critical role for the tyrosine kinase Syk in signalling through the high affinity IgE receptor of mast cells. Oncogene 1996; 13:2595-605.
4. Wong WS, Leong KP. Tyrosine kinase inhibitors: a new approach for asthma. Biochim Biophys Acta 2004; 1697:53-69.
5. Turner M, Schweighoffer E, Colucci F, Di Santo JP, Tybulewicz VL. Tyrosine kinase Syk: essential functions for immunoreceptor signalling. Immunol Today 2000; 21:148-54.
6. Coopman PJ, Mueller SC. The Syk tyrosine kinase: a new negative regulator in tumor growth and progression. Cancer Lett 2006; 241:159-73.
7. Dauvillier S, Merida P, Visintin M, Cattaneo A, Bonnerot C, Dariavach P. Intracellular single-chain variable fragments directed to the Src homology 2 domains of Syk partially inhibit Fc epsilon RI signaling in the RBL-2H3 cell line. J Immunol 2002; 169:2274-83.
8. Peneff C, Lefranc MP, Dariavach P. Characterisation and specificity of two single-chain Fv antibodies directed to the protein tyrosine kinase Syk. J Immunol Methods 2000; 236:105-15.
9. Lobato MN, Rabbitts TH. Intracellular antibodies and challenges facing their use as therapeutic agents. Trends Mol Med 2003; 9:390-6.
10. Latour S, Bonnerot C, Fridman WH, Daeron M. Induction of tumor necrosis factor-alpha production by mast cells via Fc gamma R. Role of the Fc gamma RIII gamma subunit. J Immunol 1992; 149:2155-62.
11. Dombrowicz D, Flamand V, Miyajima I, Ravetch JV, Galli SJ, Kinet JP. Absence of Fc epsilonRI alpha chain results in upregulation of Fc gammaRIII-dependent mast cell degranulation and anaphylaxis. Evidence of compétition between Fc epsilonRI and Fc gammaRIII for limiting amounts of FcR beta and gamma chains. J Clin Invest 1997; 99:915-25.
12. Laurie AT, Jackson RM. Q-SiteFinder: an energy-based method for the prediction of protein-ligand binding sites. Bioinformatics 2005; 21:1908-16.
13. An J, Totrov M, Abagyan R. Comprehensive identification of "druggable" protein ligand binding sites. Genome Inform 2004; 15:31-41.
14. Venkatachalam CM, Jiang X, Oldfield T, Waldman M. LigandFit: a novel method for the shape-directed rapid docking of ligands to protein active sites. J Mol Graph Model 2003; 21:289-307.
15. Jain AN. Surflex: fully automatic flexible molecular docking using a molecular similarity-based search engine. J Med Chem 2003; 46:499-511.
16. Frank R, Overwin H. SPOT synthesis. Epitope analysis with arrays of synthetic peptides prepared on cellulose membranes. Methods Mol Biol 1996; 66:149-69.
17. Futterer K, Wong J, Grucza RA, Chan AC, Waksman G. Structural basis for Syk tyrosine kinase ubiquity in signal transduction pathways revealed by the crystal structure of its regulatory SH2 domains bound to a dually phosphorylated ITAM peptide. J Mol Biol 1998; 281:523-37.
18. Nadler MJ, Matthews SA, Turner H, Kinet JP. Signal transduction by the high-affinity immunoglobulin E receptor Fc epsilon RI: coupling form to function. Adv Immunol 2000; 76:325-55.
19. Parravicini V, Gadina M, Kovarova M, Odom S, Gonzalez-Espinosa C, Furumoto Y, et al. Fyn kinase initiates complementary signals required for IgE-dependent mast cell degranulation. Nat Immunol 2002; 3:741-8.
20. Rameh LE, Rhee SG, Spokes K, Kazlauskas A, Cantley LC, Cantley LG. Phosphoinositide 3-kinase regulates phospholipase Cgamma-mediated calcium signaling. J Biol Chem 1998; 273:23750-7.
21. Saitoh S, Odom S, Gomez G, Sommers CL, Young HA, Rivera J, et al. The four distal tyrosines are required for LAT-dependent signaling in FcepsilonRI-mediated mast cell activation. J Exp Med 2003; 198:831-43.
22. Silverman MA, Shoag J, Wu J, Koretzky GA. Disruption of SLP-76 interaction with Gads inhibits dynamic clustering of SLP-76 and FcepsilonRI signaling in mast cells. Mol Cell Biol 2006; 26:1826-38.
23. Hendricks-Taylor LR, Motto DG, Zhang J, Siraganian RP, Koretzky GA. SLP-76 is a substrate of the high affinity IgE receptor-stimulated protein tyrosine kinases in rat basophilic leukemia cells. J Biol Chem 1997; 272:1363-7.
24. Takata M, Kurosaki T. A role for Bruton's tyrosine kinase in B cell antigen receptor-mediated activation of phospholipase C-gamma 2. J Exp Med 1996; 184:31-40.
25. Law CL, Chandran KA, Sidorenko SP, Clark EA. Phospholipase C-gamma1 interacts with conserved phosphotyrosyl residues in the linker région of Syk and is a substrate for Syk. Mol Cell Biol 1996; 16:1305-15.
26. Pivniouk VI, Martin TR, Lu-Kuo JM, Katz HR, Oettgen HC, Geha RS. SLP-76 deficiency impairs signaling via the high-affinity IgE receptor in mast cells. J Clin Invest 1999; 103:1737-43.
27. Kettner A, Pivniouk V, Kumar L, Falet H, Lee JS, Mulligan R, et al. Structural requirements of SLP-76 in signaling via the high-affinity immunoglobulin E receptor (Fc epsilon RI) in mast cells. Mol Cell Biol 2003; 23:2395-406.
28. Manetz TS, Gonzalez-Espinosa C, Arudchandran R, Xirasagar S, Tybulewicz V, Rivera J. Vav1 regulates phospholipase cgamma activation and calcium responses in mast cells. Mol Cell Biol 2001; 21:3763-74.
29. Wilson BS, Pfeiffer JR, Oliver JM. FcepsilonRI signaling observed from the inside of the mast cell membrane. Mol Immunol 2002; 38:1259-68.
30. Wu JN, Jordan MS, Silverman MA, Peterson EJ, Koretzky GA. Differential requirement for adapter proteins Src homology 2 domain-containing leukocyte phosphoprotein of 76 kDa and adhesion- and degranulation-promoting adapter protein in FcepsilonRI signaling and mast cell function. J Immunol 2004; 172:6768-74.
31. Barker SA, Caldwell KK, Pfeiffer JR, Wilson BS. Wortmannin-sensitive phosphorylation, translocation, and activation of PLCgamma1, but not PLCgamma2, in antigen-stimulated RBL-2H3 mast cells. Mol Biol Cell 1998; 9:483-96.
32. Fluckiger AC, Li Z, Kato RM, Wahl MI, Ochs HD, Longnecker R, et al. Btk/Tec kinases regulate sustained increases in intracellular Ca2+ following B-cell receptor activation. Embo J 1998; 17:1973-85.
33. Scharenberg AM, El-Hillal O, Fruman DA, Beitz LO, Li Z, Lin S, et al. Phosphatidylinositol-3,4,5-trisphosphate (Ptdlns-3,4,5-P3)/Tec kinase-dependent calcium signaling pathway: a target for SHIP-mediated inhibitory signais. Embo J 1998; 17:1961-72.
34. Wen R, Jou ST, Chen Y, Hoffmeyer A, Wang D. Phospholipase C gamma 2 is essential for specific functions of Fc epsilon R and Fc gamma R. J Immunol 2002; 169:6743-52.
35. Qi Q, August A. Keeping the (Kinase) Party Going: SLP-76 and ITK Dance to the Beat. Sci STKE 2007; 2007:pe39.
36. Mocsai A, Zhou M, Meng F, Tybulewicz VL, Lowell CA. Syk is required for integrin signaling in neutrophils. Immunity 2002; 16: 547-558.
37. Matsumoto T, Guo YJ, Ikejima T, Yamada H. Induction of cell cycle regulatory proteins by murine B cell proliferating pectic polysaccharide from the roots of Bupleurum falcatum L. Immunology letters 2003; 89: 111-118.
38. Okkenhaug K, Bilancio A, Farjot G, Priddle H, Sancho S, Peskett E, et al. Impaired B and T cell antigen receptor signaling in p110delta PI 3-kinase mutant mice. Science 2002; 297: 1031-1034.
39. Gell PGH, Coombs RRA, eds. Clinical Aspects of Immunology. 1st ed. Oxford, England: Blackwell; 1963
40. S. M. Berge et al. Pharmaceutical Salts. J. Pharm. Sci, 1977, 66:p.1-19

## Revendications

1. Molécule se liant à la protéine tyrosine kinase Syk humaine au niveau d'une poche tridimensionnelle comprenant le résidu Arginine situé en position 68 et les deux résidus acide glutamique situés en positions 121 et 155 de la protéine Syk humaine dont la séquence est illustrée par la SEQ ID N°1, et capable d'inhiber d'au moins 10% in vitro la liaison
(i) du fragment d'anticorps G4G11 (SEQ ID N°2), ou
(ii) d'un anticorps ou fragment d'anticorps se liant à la protéine tyrosine kinase Syk humaine au niveau d'un épitope comprenant au moins l'un des résidus 65 à 74 de la séquence en acides aminés de la protéine tyrosine kinase Syk humaine représentée par la séquence SEQ ID N°1, ou
(iii) d'un anticorps ou fragment d'anticorps se liant à la protéine tyrosine kinase Syk humaine et inhibant d'au moins 10% la liaison du fragment d'anticorps G4G11 avec la protéine tyrosine kinase Syk humaine (SEQ ID N°1),
à la protéine tyrosine kinase Syk humaine ou à l'une de ses variantes chez l'animal, pour une utilisation dans la prévention ou le traitement d'une pathologie dépendante d'une voie métabolique impliquant Syk chez l'homme ou l'animal,
où ladite pathologie est causée par une hypersensibilité de type I, ou est la polyarthrite rhumatoïde, une maladie auto-immune, une inflammation ou un cancer,
où ladite molécule est la molécule C-13 ayant pour formule 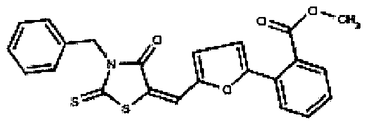 ou une molécule organique de poids moléculaire compris entre 50 et 2500 Dalton équivalente sur le plan fonctionnel à la molécule C-13, choisie parmi les molécules ayant l'une des formules suivantes : 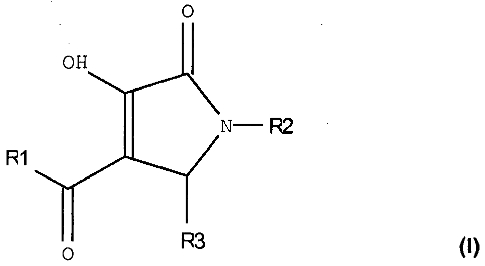 où
- R1 est un groupement aromatique substitué ou non, ou un hétérocycle substitué ou non comprenant au moins un atome S, O ou N ;
- R2 est un groupement aromatique substitué ou non, un hétérocycle substitué ou non, une chaîne carbonée saturée ou non, comprenant un groupement amine, une chaîne carbonée saturée ou non comprenant un groupement aromatique substitué ou non ou une chaîne carbonée saturée ou non comprenant un hétérocycle substitué ou non comprenant au moins un atome S, O ou N ;
- R3 est un groupement phényle, 2-pyridinyle, 3-pyridinyle ou 4-pyridinyle, substitué ou non ;
ou 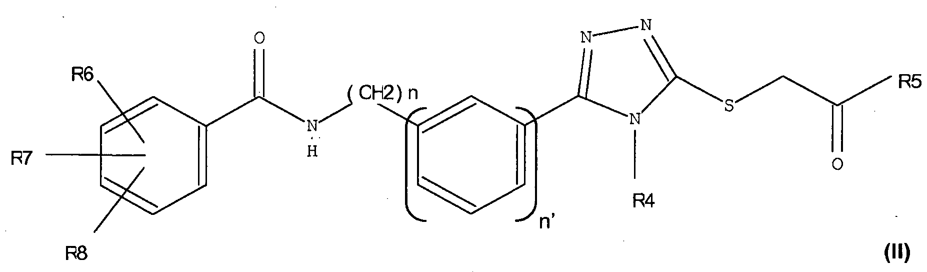
où
- n = 0 ou 1 ; n' = 0 ou 1 ;
- R4 est une chaîne carbonée saturée ou non comprenant 1 à 5 atomes de carbone, substituée ou non par un groupement aromatique ;
- R5 est un groupement aromatique substitué ou non ou un groupement amine substitué ou non ;
- R6 est un atome d'hydrogène, un groupement alkoxy, un groupement alkyle ou un halogène ;
- R7 est un atome d'hydrogène, un groupement alkoxy, un groupement alkyle ou un halogène ;
- R8 est un atome d'hydrogène, un groupement alkoxy, un groupement alkyle ou un halogène ;
ou 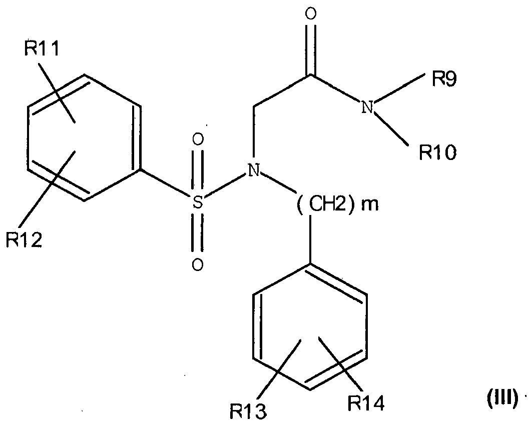
où
- m = 0, 1 ou 2;
- R9 est un atome d'hydrogène et R10 est un groupement phényle substitué ou non, ou R9 et R10 font partie d'un même hétérocycle substitué ou non, ou R9 et R10 font partie d'un même groupement aromatique substitué ou non;
- R11 est un atome hydrogène, un groupement alkoxy ou un groupement alkyle ;
- R12 est un atome hydrogène, un groupement alkoxy ou un groupement alkyle ;
- R13 est un atome hydrogène ou un groupement alkyle ou alkoxy;
- R14 est un atome hydrogène ou un groupement alkyle ou alkoxy ;
ou 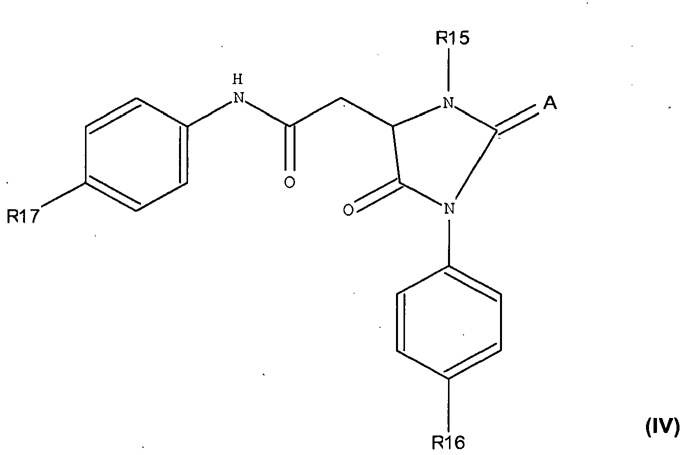
où
- A est un atome d'oxygène ou de Soufre ;
- R15 est une chaîne carbonée saturée ou non comprenant 1, 2 ou 3 atomes de carbone, substituée ou non par un groupement aromatique substitué ou non, par un hétérocycle substitué ou non ou par un groupement amine faisant partie d'un hétérocycle substitué ou non ;
- R16 est un atome d'hydrogène, un halogène ou un groupement alkoxy ;
- R17 est un atome d'hydrogène, un groupement alkoxy ou un groupement acétoxy ; 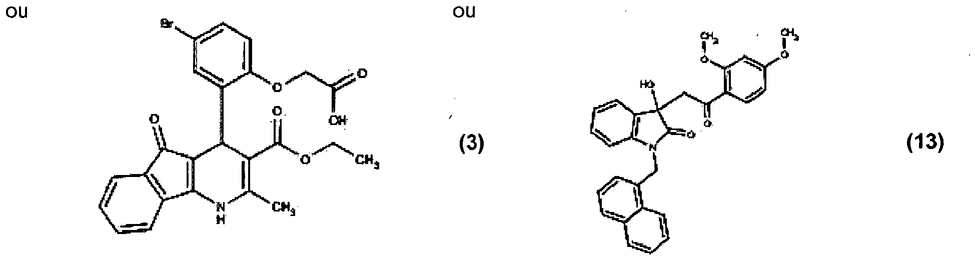 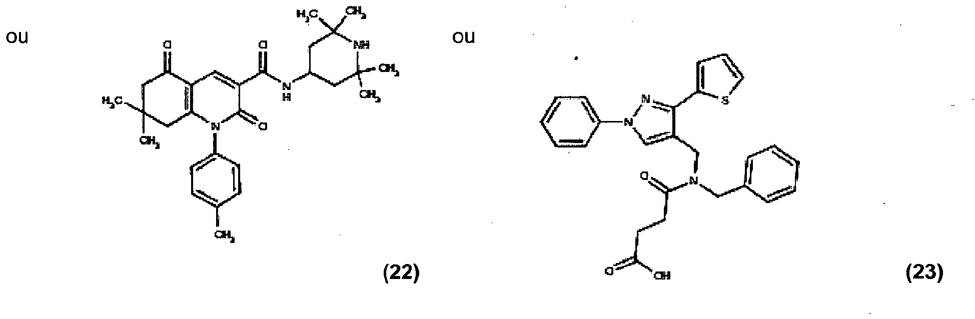 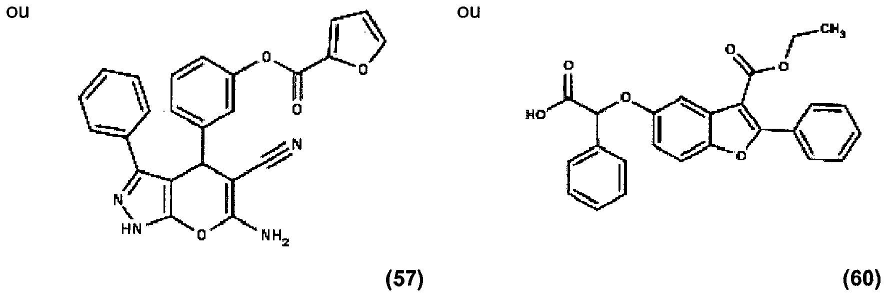 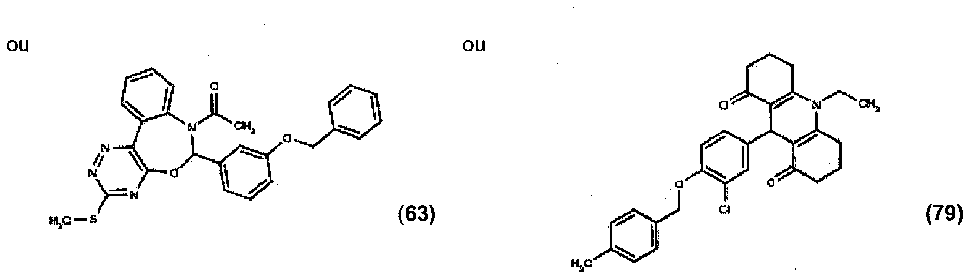 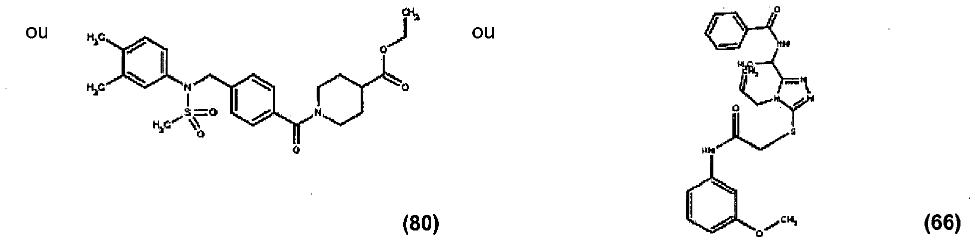 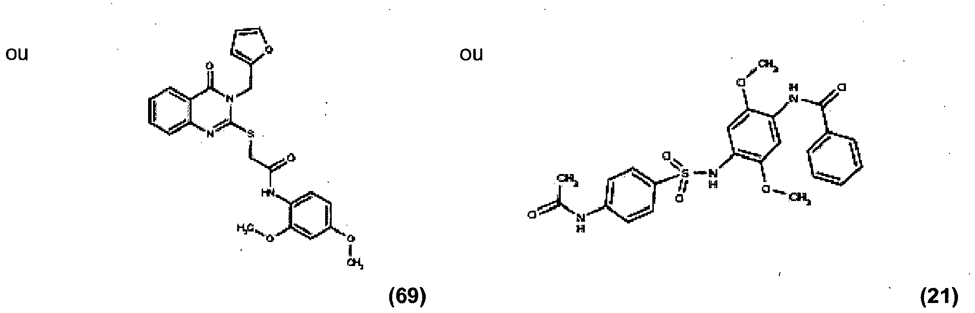 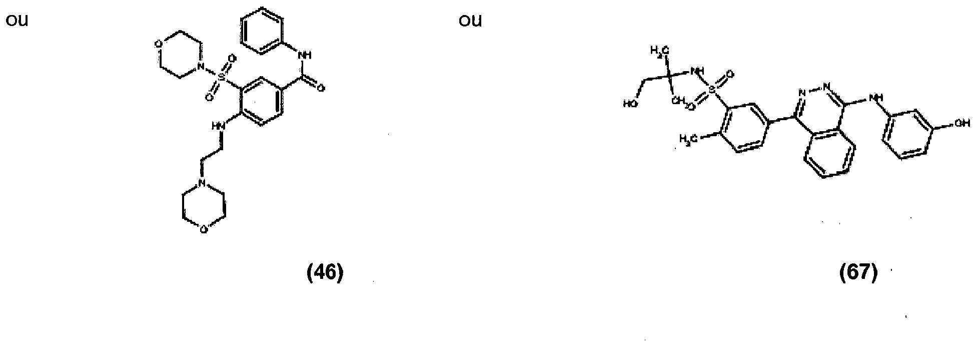 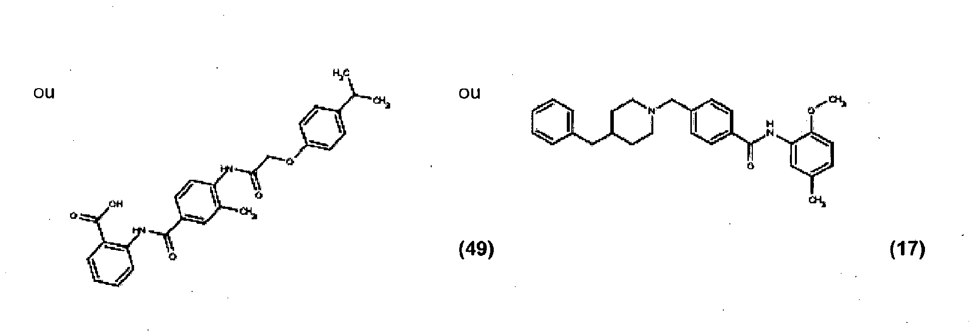 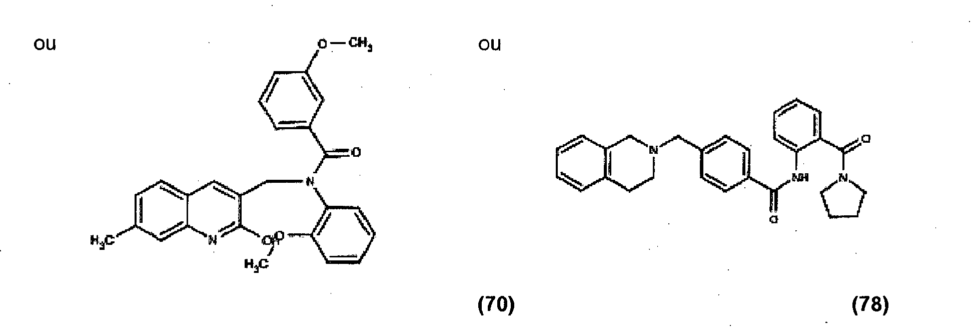 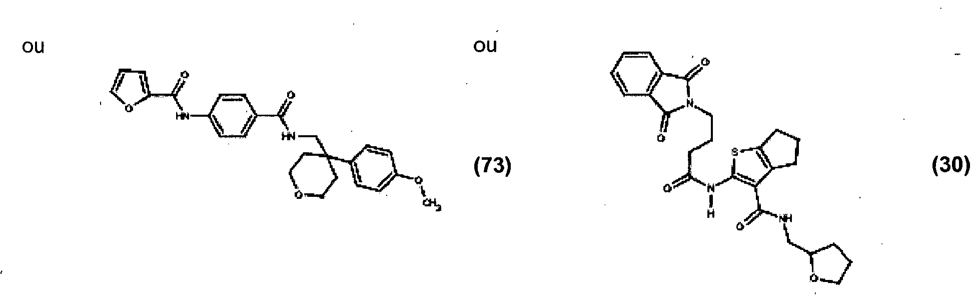 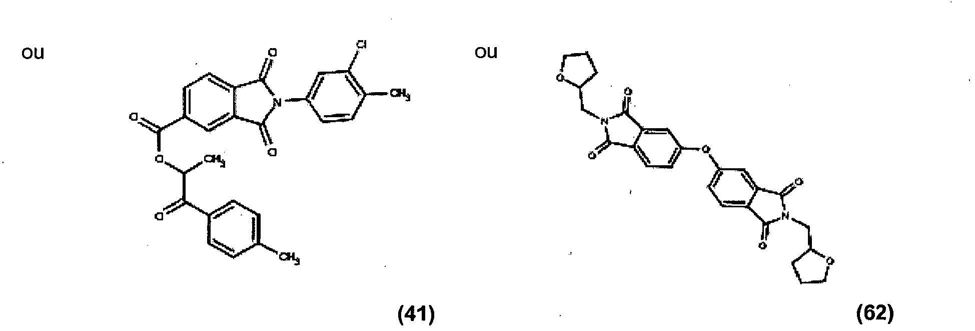 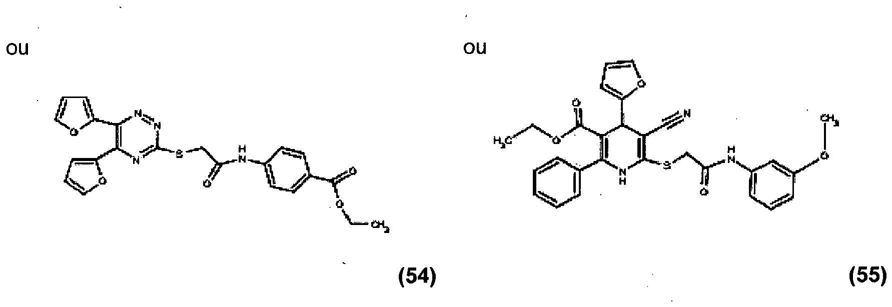 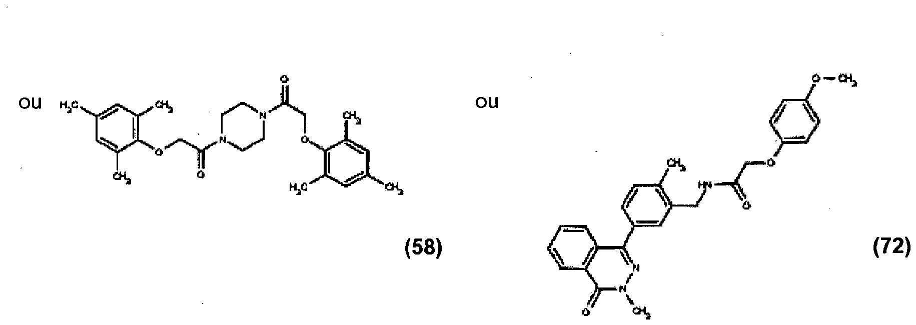 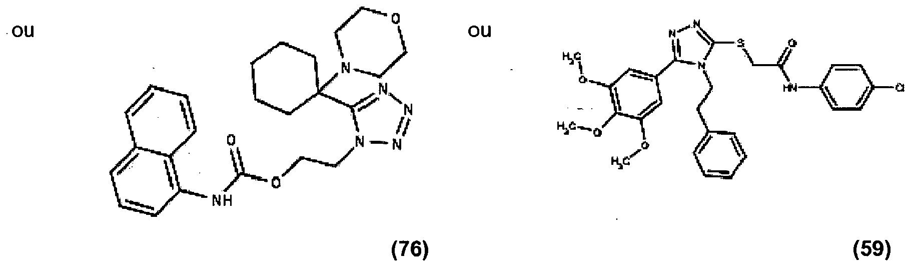 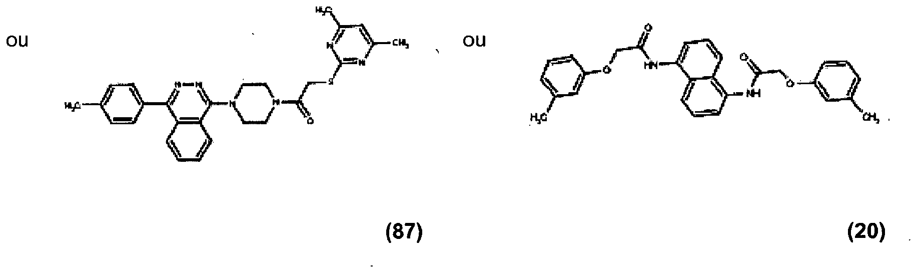 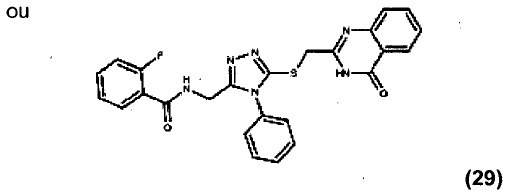
ou un stéréo-isomère, racémate ou sel pharmacologiquement acceptable de C-13 ou de ladite molécule équivalente ;
où si ladite molécule est sélectionnée parmi les molécules de formules suivantes 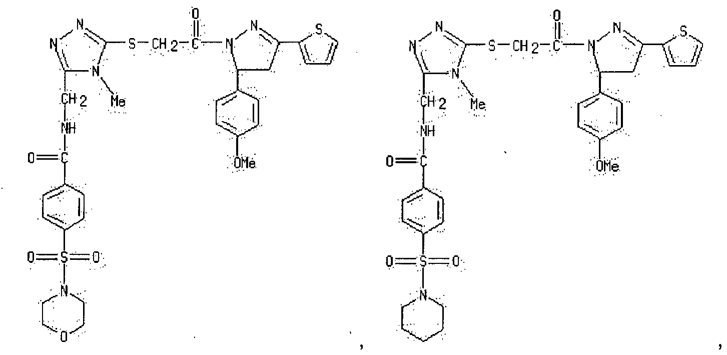 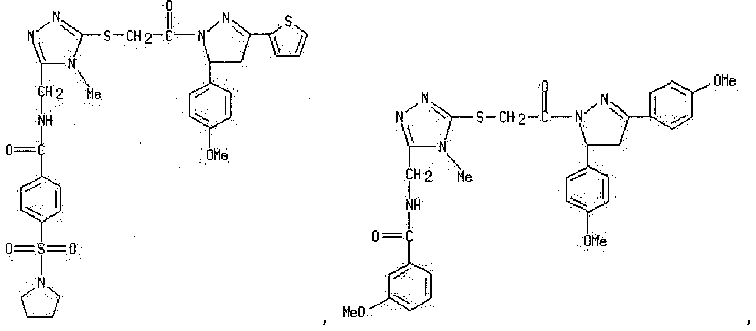 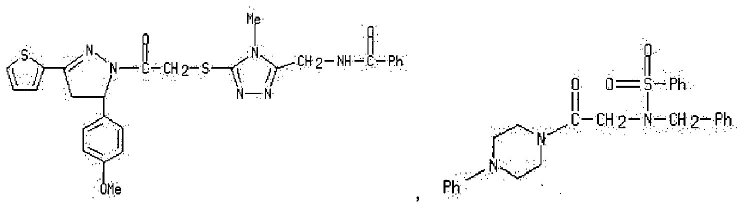 et 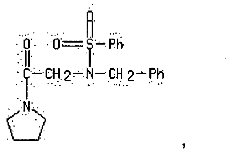 elle n'est pas utilisée en combinaison avec un agoniste d'un récepteur de glucocorticoïde.

2. Molécule pour son utilisation selon la revendication 1, où ledit anticorps ou fragment d'anticorps se lie à la protéine tyrosine kinase Syk humaine au niveau d'un épitope comprenant au moins 5 des résidus 65 à 74 de la séquence en acides aminée de la protéine Syk humaine illustrée par la SEQ ID N°1.

3. Molécule pour son utilisation selon la revendication 1 ou 2 où ladite poche tridimensionnelle comprend également le résidu Sérine situé en position 9, le résidu Glutamine situé en position 43, le résidu Phénylalanine situé en position 51, le résidu Isoleucine situé en position 66, les résidus Glutamate situés en position 67 et 69, le résidu Leucine situé en position 70, le résidu Asparagine situé en position 71, le résidu Glycine situé en position 72, le résidu Thréonine situé en position 73, le résidu Tyrosine situé en position 74 et le résidu Alanine situé en position 75 de la protéine Syk humaine dont la séquence est illustrée par la SEQ ID N°1.

4. Molécule pour son utilisation selon l'une quelconque des revendications 1 à 3 où ladite molécule est utilisée pour la prévention ou le traitement de réactions d'hypersensibilité de type I, et où ladite molécule inhibe de 50% *in vitro* la dégranulation des mastocytes dépendante des IgE à une concentration (CI50) comprise entre 1 ng/ml et 1 mg/ml.

5. Molécule pour son utilisation selon la revendication 4 où ladite voie métabolique impliquant Syk est une voie d'activation des mastocytes ou des basophiles.

6. Molécule pour son utilisation selon la revendication 4 ou 5 où ladite pathologie est l'asthme allergique, la conjonctivite allergique, la rhinite allergique, l'anaphylaxie, l'angioedème, l'urticaire, l'éosinophilie ou une allergie à un antibiotique.

7. Molécule pour son utilisation selon l'une quelconque des revendications 5 à 6 où ladite molécule n'a pas d'effets sur les voies métaboliques impliquant la protéine Syk humaine (SEQ ID N° 1) autres que celles conduisant à la dégranulation des mastocytes et/ou aux réactions d'hypersensibilité de type I, en particulier sur la réponse anticorps faisant suite à une immunisation par un antigène thymo-dépendant ou sur le recrutement Syk-dépendant des neutrophiles.

8. Molécule pour son utilisation selon l'une quelconque des revendications 1 à 3 où ladite voie métabolique impliquant Syk est une voie d'activation des lymphocytes B, des lymphocytes T, des neutrophiles, des éosinophiles, des cellules NK, des plaquettes, des érythrocytes, des ostéoclastes, des cellules épithéliales ou de cellules cancéreuses.

9. Molécule pour son utilisation selon l'une quelconque des revendications 1 à 8 où ladite molécule est utilisée en combinaison avec une autre molécule thérapeutique, et où ladite autre molécule thérapeutique
- est également une molécule utilisée pour la prévention ou le traitement d'une pathologie dépendante d'une voie métabolique impliquant Syk, ou
- est une molécule utilisée pour la prévention ou le traitement d'une pathologie non dépendante d'une voie métabolique impliquant Syk.

10. Molécule pour son utilisation selon l'une quelconque des revendications 1 à 9 où ladite molécule est destiné à être administrée par voie orale, sublinguale, nasale, oculaire, locale, intraveineuse, intrapéritonéale, sous-cutanée, par aérosol ou par inhalation, à des patients humains adultes, enfants ou nouveau-nés, et à des doses comprises entre 0,01 mg/kg et 200mg/kg.

11. Molécule pour son utilisation selon la revendication 10 où ladite molécule équivalente est choisie parmi les molécules ayant l'une des formules suivantes :
• la formule (I) où
- R1 est
∘ un groupement phényle non substitué ou substitué par un atome F ou Cl, par un groupement méthyle ou éthyle, par un groupement N,N-diméthyl-sulfonamide ou par deux groupements choisis parmi les groupements méthyle, éthyle, hydroxy, méthoxy et éthoxy,
∘ ou un groupement 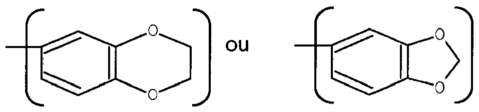 ou 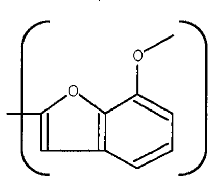
∘ ou un groupement furane non substitué ou substitué par un groupement méthyle, éthyle, hydroxy, méthoxy ou éthoxy,
∘ ou un groupement thiophène non substitué ou substitué par un groupement méthyle, éthyle, hydroxy, méthoxy ou éthoxy ;
- R2 est
∘ un groupement 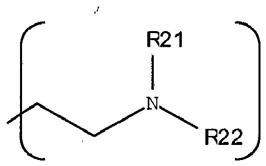 où R21 et R22 sont des atomes de carbone faisant chacun partie d'une chaîne alkyle comprenant 1, 2 ou 3 atomes de carbone, ou bien faisant tous deux partie avec
l'atome d'azote auquel ils sont liés d'un même hétérocycle saturé ou non comprenant également un atome d'oxygène ou un second atome d'azote,
∘ ou un groupement 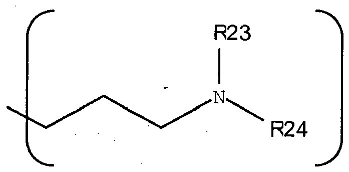 où R23 et R24 sont des atomes de carbone faisant chacun partie d'une chaîne alkyle comprenant 1, 2 ou 3 atomes de carbone, ou bien faisant tous deux partie avec l'atome d'azote auquel ils sont liés d'un même hétérocycle saturé ou non comprenant également un atome d'oxygène ou un second atome d'azote,
∘ ou un groupement 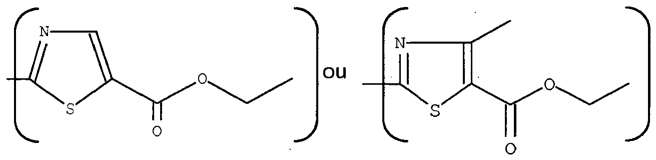 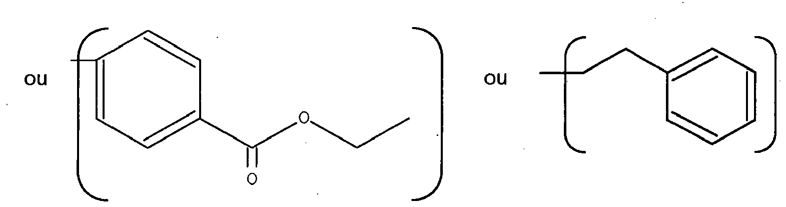 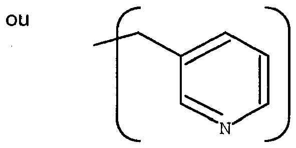
- et R3 est
∘ un groupement 2-pyridinyle, 3-pyridinyle ou 4-pyridinyle non substitué,
∘ ou un groupement phényle non substitué ou substitué par un groupement benzoxy, et/ou par un groupement hydroxy, et/ou par un groupement méthyle, et/ou par un groupement éthyle, et/ou par un groupement propyle, et/ou par un ou deux atomes Br, F ou CI, et/ou par un à trois groupements hydroxy, méthoxy ou éthoxy.
ou
• la formule **(II)** où
- R4 est une chaîne carbonée saturée ou insaturée comprenant 1, 2 ou 3 atomes de carbone ;
- R5 est un groupement phényle ou un groupement amine secondaire substitué par un groupement phényle substitué ou non, ou par un groupement 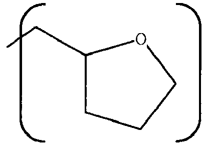
- R6 est un atome d'hydrogène ou de chlore ou un groupement méthyle, éthyle, hydroxy, méthoxy ou éthoxy ;
- R7 est un atome d'hydrogène ou de chlore ou un groupement méthyle, éthyle, hydroxy, méthoxy ou éthoxy ;
- R8 est un atome d'hydrogène ou de chlore ou un groupement méthyle, éthyle, hydroxy, méthoxy ou éthoxy.
ou
• la formule **(III)** où
- R9 est un atome d'hydrogène et le groupement R10 est un groupement phényle substitué ou non, ou les groupements R9 et R10 font partie d'un même hétérocycle substitué ou non comprenant 2 atomes d'azote et 4 atomes de carbone ;
- R11 est un atome hydrogène ou un groupement méthyle, éthyle, hydroxy, méthoxy ou éthoxy ;
- R12 est un atome hydrogène ou un groupement méthyle, éthyle, hydroxy, méthoxy ou éthoxy ;
- R13 est un atome hydrogène ou un groupement méthyle, éthyle, hydroxy, méthoxy ou éthoxy ;
- R14 est un atome hydrogène ou un groupement méthyle, éthyle, hydroxy, méthoxy ou éthoxy ;
ou
• la formule **(IV)** où
- A est un atome d'Oxygène ou de Soufre ;
- R15 est un groupement 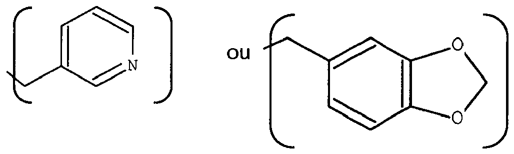 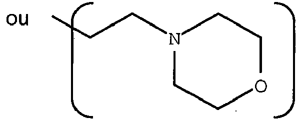
- R16 est un atome d'hydrogène ou de chlore ou un groupement méthyle, éthyle, hydroxy, méthoxy ou éthoxy;
- R17 est un méthyle, éthyle, hydroxy, méthoxy, éthoxy, acétoxy, méthoxycarbonyle ou éthoxycarbonyle.

12. Molécule pour son utilisation selon la revendication 10 où ladite molécule équivalente est choisie parmi les molécules suivantes :

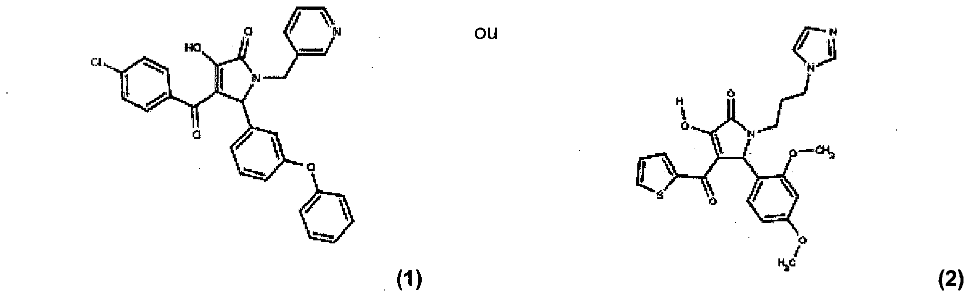

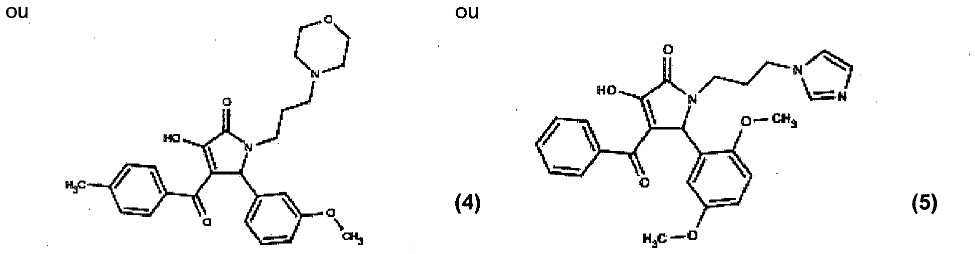

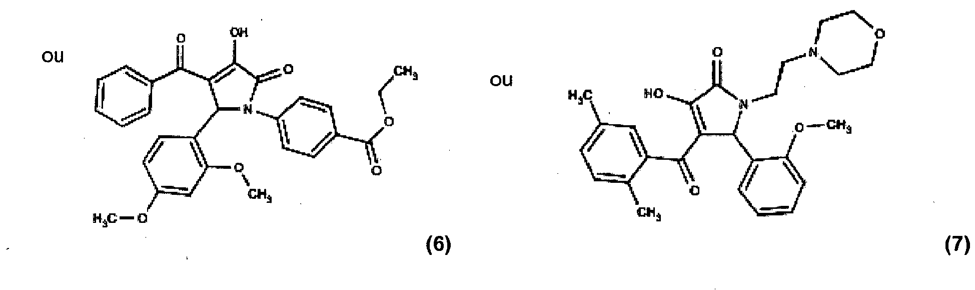

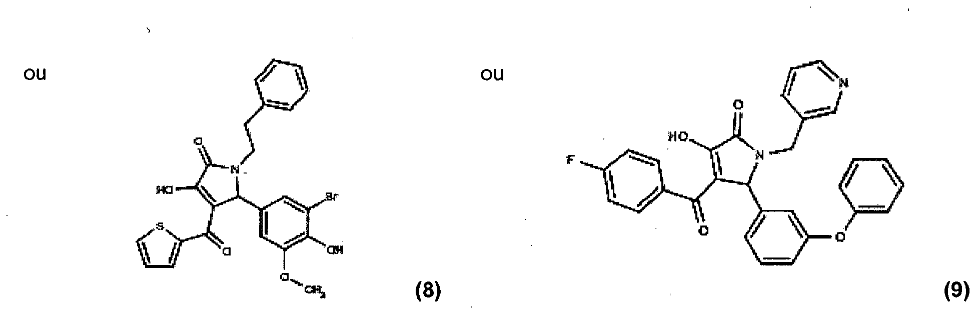

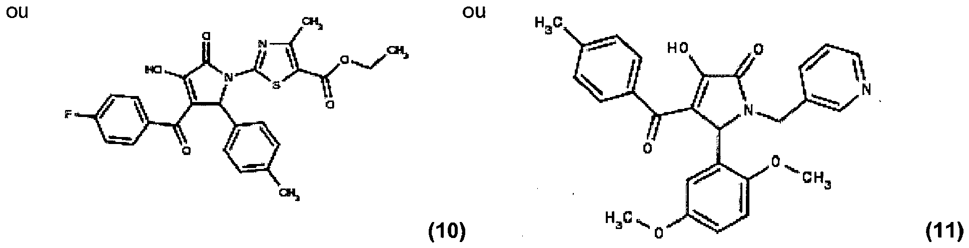

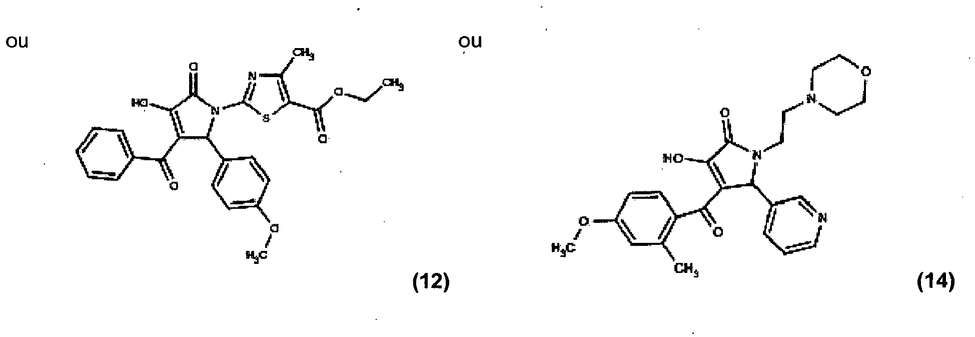

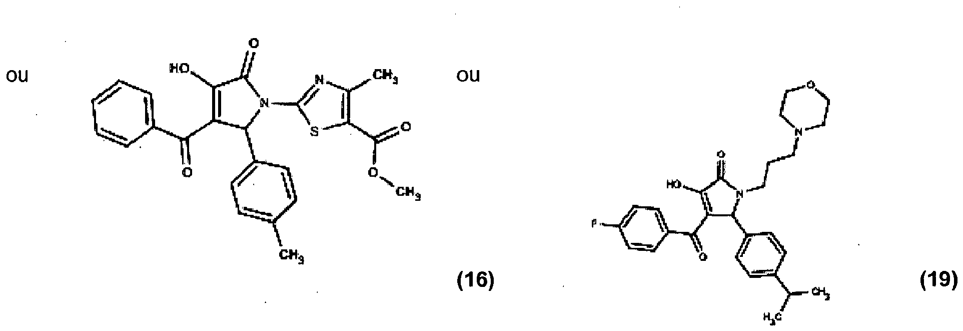

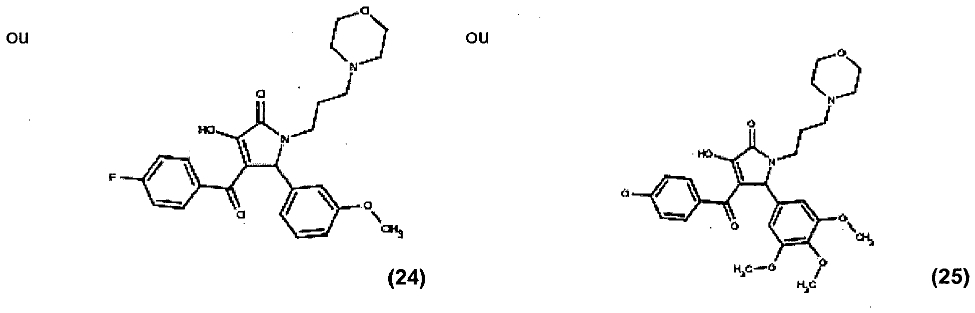

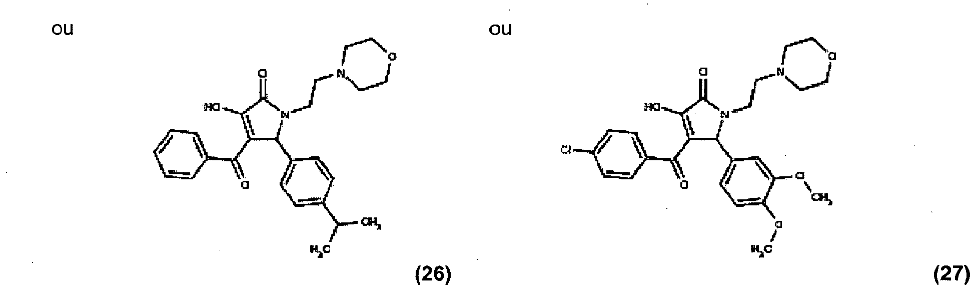

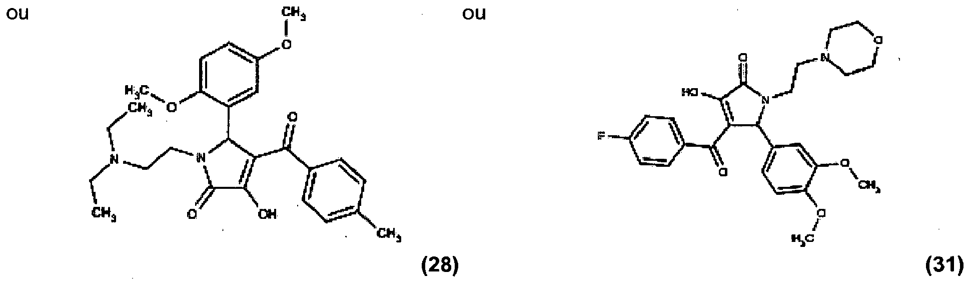

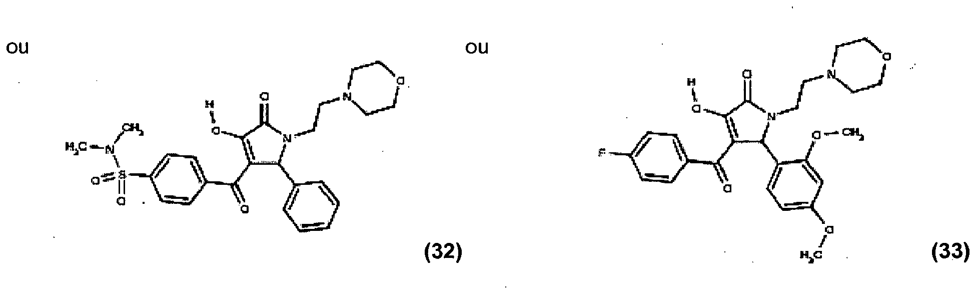

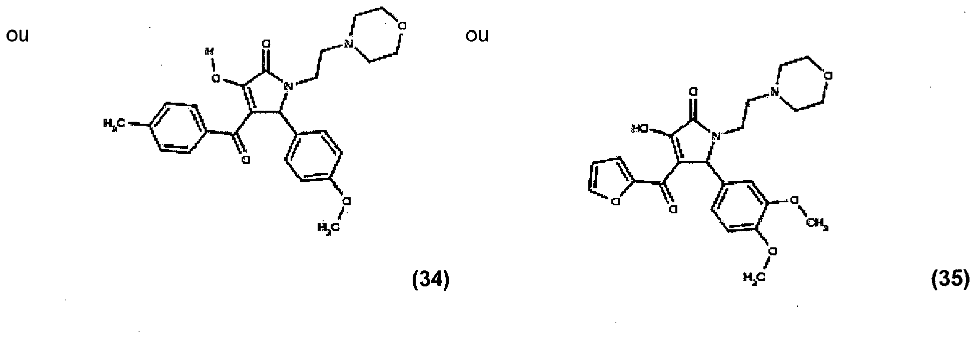

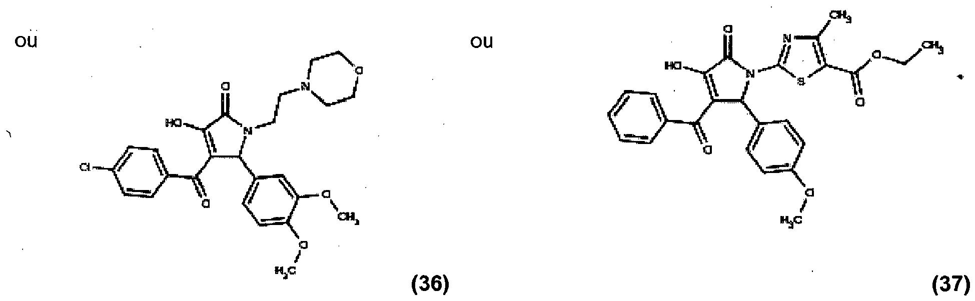

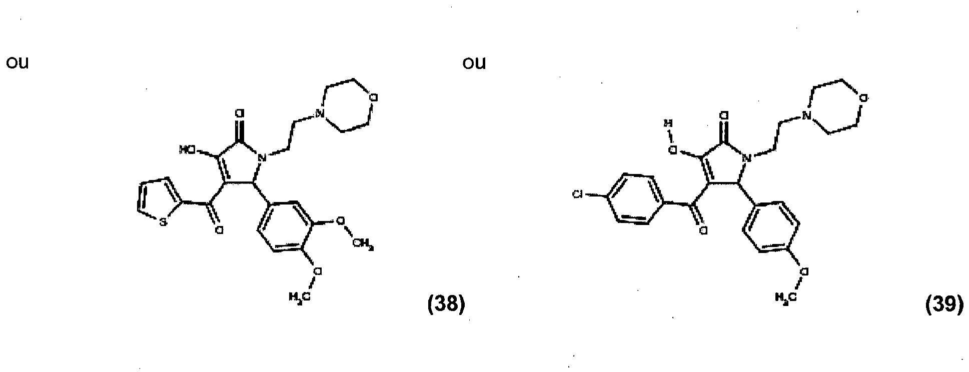

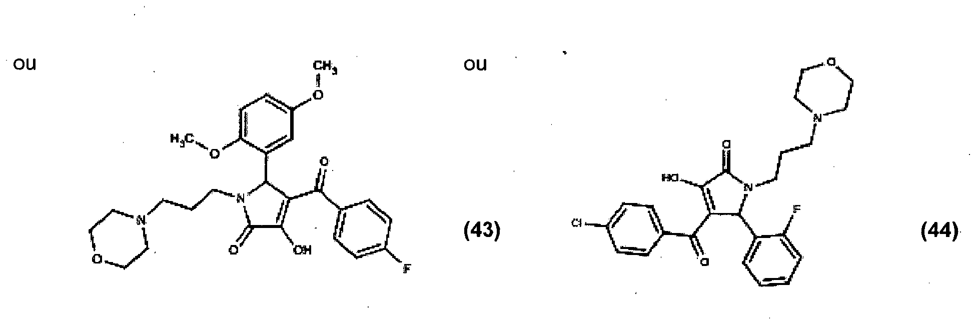

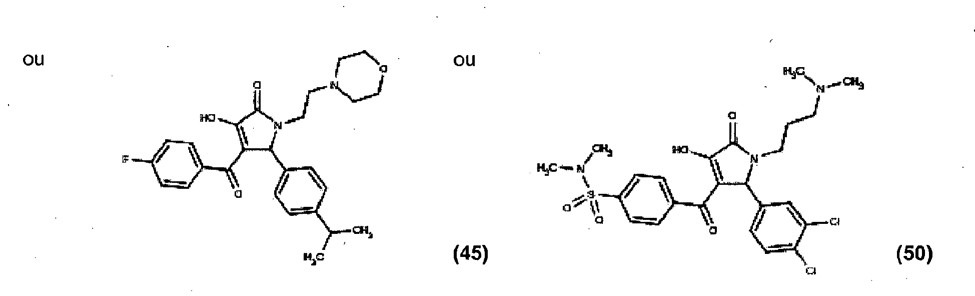

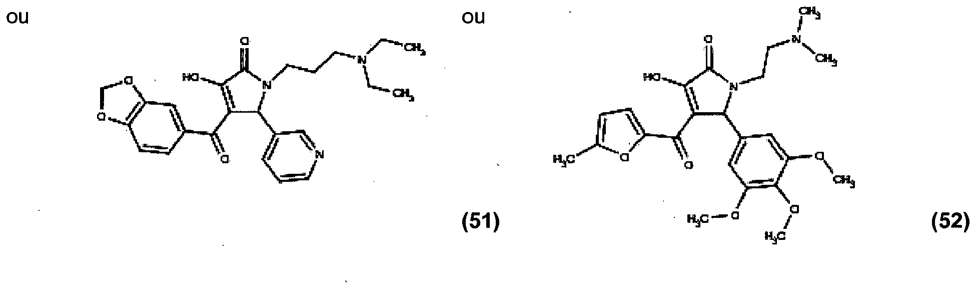

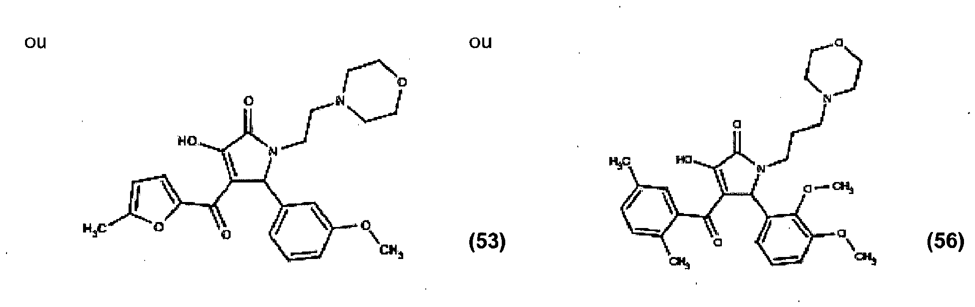

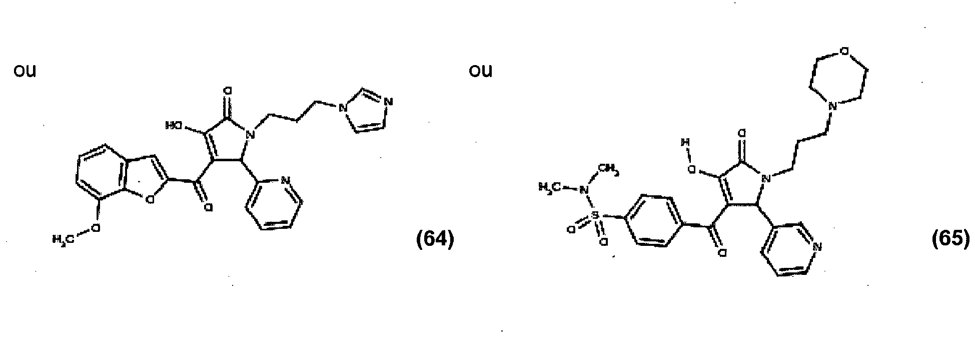

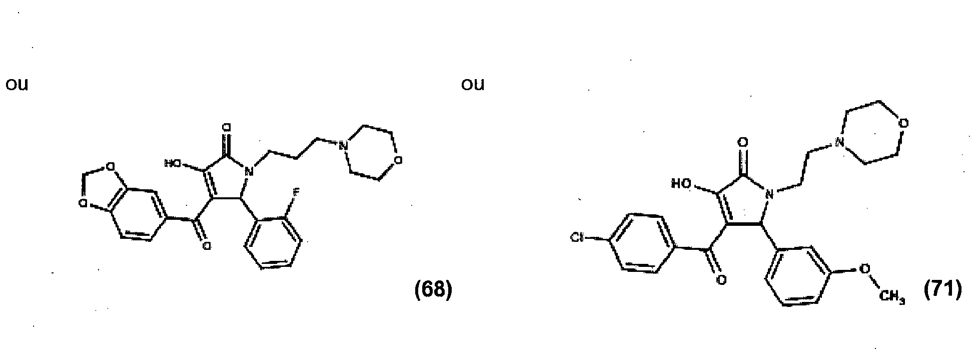

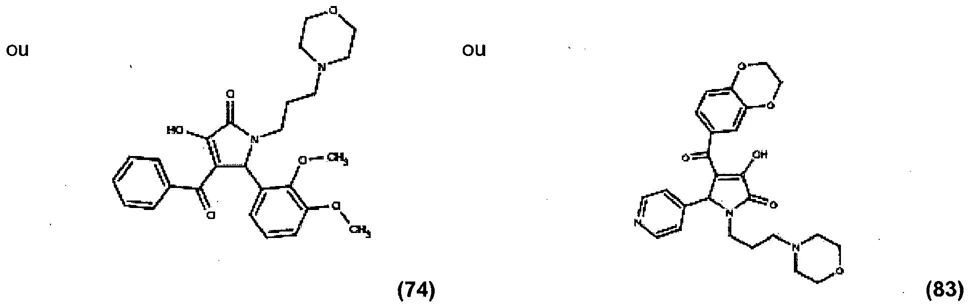

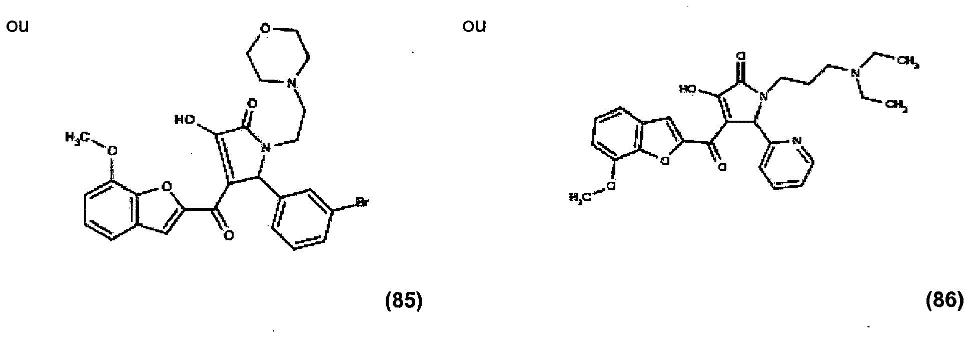

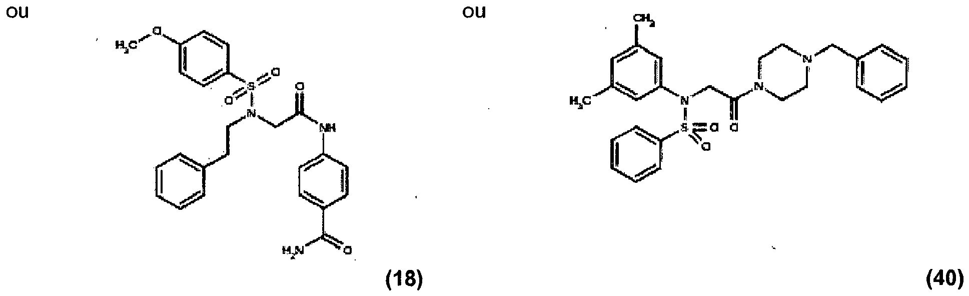

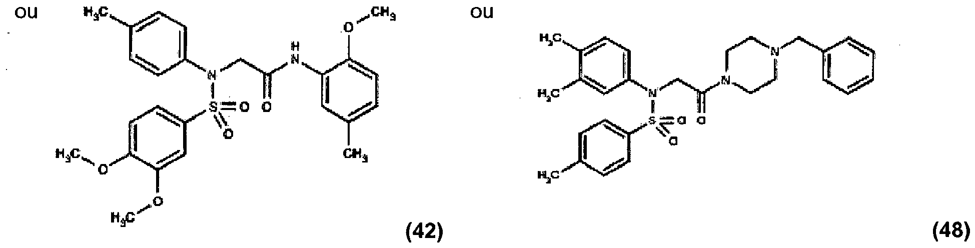

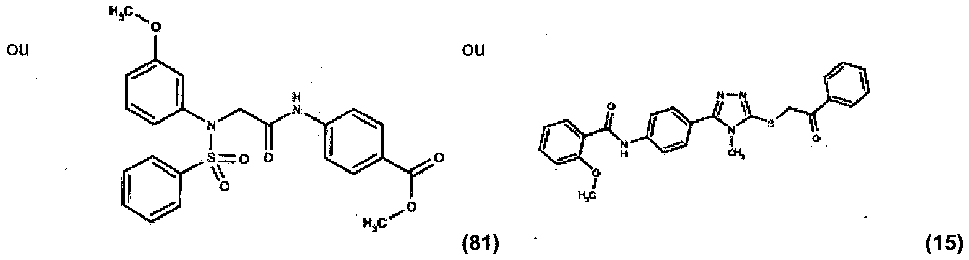

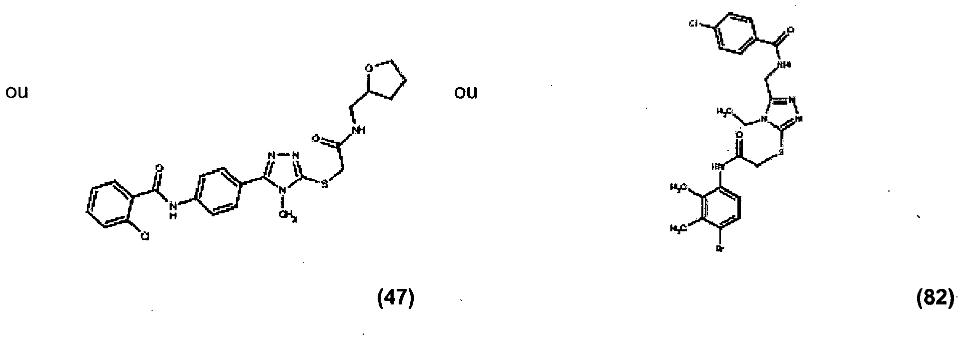

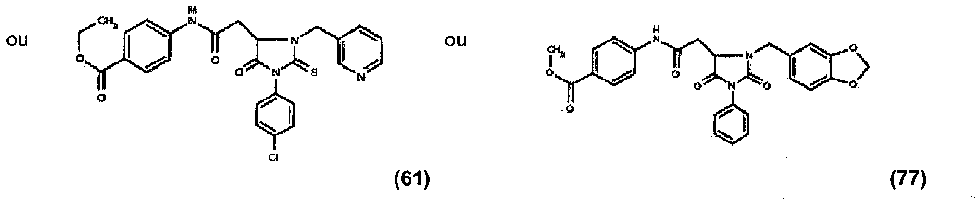

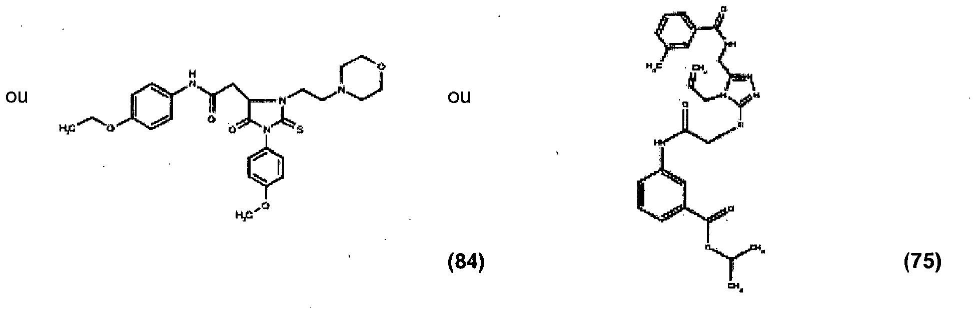

13. Composition comprenant une molécule selon l'une quelconque des revendications 1 à 12 et un excipient acceptable sur le plan pharmacologique pour une utilisation dans la prévention ou le traitement d'une pathologie dépendante d'une voie métabolique impliquant Syk chez l'homme ou l'animal, où ladite pathologie est causée par une hypersensibilité de type I, ou est la polyarthrite rhumatoïde, une maladie auto-immune, une inflammation ou un cancer.

14. Composition pour son utilisation selon la revendication 13 où ladite composition comprend également une autre molécule thérapeutique.

15. Procédé pour l'identification d'une molécule organique de poids moléculaire compris entre 50 et 2500 Dalton se liant à la protéine tyrosine kinase Syk et capable d'inhiber d'au moins 10% *in vitro* la liaison
(i) du fragment d'anticorps G4G11 (SEQ ID N°2), ou
(ii) d'un anticorps ou fragment d'anticorps se liant à la protéine tyrosine kinase Syk humaine au niveau d'un épitope comprenant au moins l'un des résidus 65 à 74 de la séquence en acides aminés de la protéine tyrosine kinase Syk humaine représentée par la séquence SEQ ID N°1, ou
(iii) d'un anticorps ou fragment d'anticorps se liant à la protéine tyrosine kinase Syk humaine et inhibant d'au moins 10% la liaison du fragment d'anticorps G4G11 avec la protéine tyrosine kinase Syk humaine (SEQ ID N°1),
à la protéine tyrosine kinase Syk humaine ou à l'une de ses variantes chez l'animal, comprenant au moins les étapes suivantes :
a) le criblage à partir d'une banque de molécules organiques candidates de poids moléculaire compris entre 50 et 2500 Da de celles susceptibles de se lier à ladite protéine Syk au niveau de la poche tridimensionnelle de liaison sur ladite protéine Syk d'une molécule choisies parmi les molécules de formule C-13, I, II, II, IV ou 1 à 87 définies ci-dessus ;
b) la sélection parmi les molécules identifiées en a) de celles capables d'inhiber d'au moins 10% *in vitro* la liaison de l'anticorps ou fragment d'anticorps (i), (ii) ou (iii) à la protéine Syk.

16. Procédé selon la revendication 15 où la molécule de l'étape a) est la molécule **C-13**, la molécule (**59**) ou la molécule (**61**).

17. Procédé selon la revendication 15 ou 16 comprenant une étape supplémentaire préalable à l'étape a) d'identification de la poche tridimensionnelle de liaison sur la protéine Syk de la molécule choisies parmi les molécules de formule C-13, I, II, II, IV ou 1 à 87, où ladite étape préalable est réalisée par la mise en oeuvre d'un docking *in silico*, et
une étape supplémentaire c) de sélection parmi les molécules identifiées en b) de celles capables d'inhiber de 50% *in vitro* la dégranulation des mastocytes à une concentration (CI50) comprise entre 1ng/ml et 1mg/ml.

## Patentansprüche

1. Molekül das an das humane Protein Tyrosinkinase Syk auf Höhe einer dreidimensionalen Tasche bindet, umfassend den in Position 68 liegenden Argininrest und die in den Positionen 121 und 155 liegenden zwei Glutaminsäurereste des humanen Syk-Proteins, dessen Sequenz durch SEQ ID-Nr. 1 veranschaulicht ist und in vitro wenigstens 10% der Bindung
(i) des Antikörperfragments G4G11 (SEQ ID-Nr. 2) oder
(ii) eines Antikörpers oder Antikörperfragments, welches an das humane Protein Tyrosinkinase Syk auf Höhe eines Epitops bindet, umfassend wenigstens einen der Reste 65 bis 74 der Aminosäurensequenz des humanen Proteins Tyrosinkinase Syk, dargestellt durch die Sequenz SEQ ID-Nr. 1, oder
(iii) eines Antikörpers oder Antikörperfragments, das an das humane Protein Tyrosinkinase Syk bindet und zu wenigstens 10% die Bindung des Antikörperfragments G4G11 mit dem humanen Protein Tyrosinkinase Syk (SEQ ID-Nr. 1) inhibiert,
an das humane Protein Tyrosinkinase Syk oder eine ihrer Varianten beim Tier inhibieren kann, zur Verwendung bei der Vorbeugung oder Behandlung einer Krankheit, welche von einem Stoffwechselweg herrührt, welcher Syk beim Menschen oder Tier umfasst,
wobei die Krankheit durch eine Hypersensibilität vom Typ I verursacht wird oder rheumatoide Polyarthritis, eine Autoimmunerkrankung, eine Entzündung oder Krebs ist,
wobei das genannte Molekül ein C-13 Molekül mit der Formel 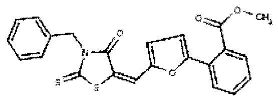 oder ein organisches Molekül mit einem zwischen 50 und 2500 Dalton enthaltenen Molekulargewicht und auf funktioneller Ebene äquivalent zum C-13 Molekül ist, ausgewählt unter den Molekülen mit einer der folgenden Formeln: 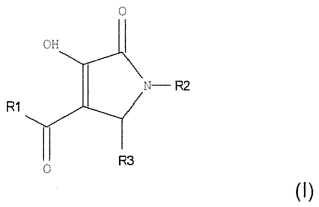 worin
- R1 eine substituierte oder nicht-substituierte aromatische Gruppe oder ein substituierter oder nicht-substituierter Heterozyklus, umfassend wenigstens ein S-, O- oder N-Atom ist;
- R2 eine substituierte oder nicht-substituierte aromatische Gruppe oder ein substituierter oder nicht-substituierter Heterozyklus, eine gesättigte oder ungesättigte Kohlenstoffkette, umfassend eine Amingruppe, eine gesättigte oder ungesättigte Kohlenstoffkette, umfassend eine substituierte oder nicht-substituierte aromatische Gruppe oder eine gesättigte oder ungesättigte Kohlenstoffkette, umfassend einen substituierten oder nicht-substituierten Heterozyklus, umfassend wenigstens ein S-, O- oder N-Atom ist;
- R3 eine substituiert oder nicht-substituierte Phenyl-, 2-Pyridinyl-, 3-Pyridinyl- oder 4-Pyridinyl-Gruppe ist;
oder 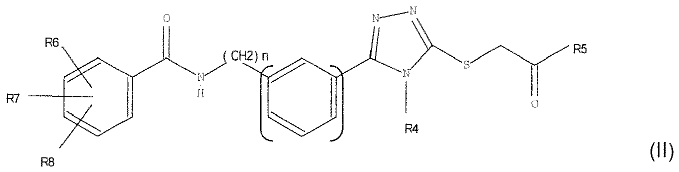 worin
- n=0 oder 1; n'=0 oder 1 ist;
- R4 eine gesättigte oder nicht-gesättigte Kohlenstoffkette, umfassend 1 bis 5 Kohlenstoffatome, welche mit eine aromatische Gruppe substituiert ist oder nicht ist;
- R5 eine substituierte oder nicht-substituierte aromatische Gruppe oder eine substituierte oder nicht-substituierte Amingruppe ist;
- R6 ein Wasserstoffatom, eine Alkoxygruppe, eine Alkylgruppe oder ein Halogen ist;
- R7 ein Wasserstoffatom, eine Alkoxygruppe, eine Alkylgruppe oder ein Halogen ist;
- R8 ein Wasserstoffatom, eine Alkoxygruppe, eine Alkylgruppe oder ein Halogen ist;
oder 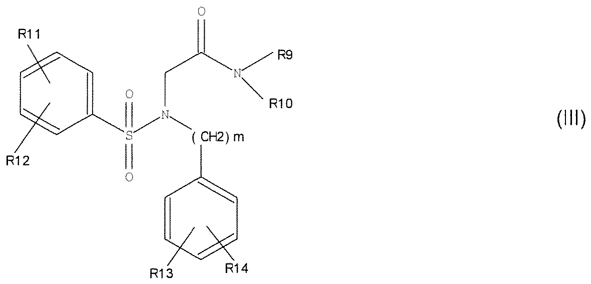 worin
- m = 0, 1 oder 2;
- R9 ein Wasserstoffatom und R10 eine substituierte oder nicht-substituierte Phenylgruppe ist oder R9 und R10 Teil eines selben substituierten oder nicht-substituierten Heterozyklus sind, oder R9 und R10 Teil einer selben substituierten oder nicht-substituierten aromatischen Gruppe sind;
- R11 ein Wasserstoffatom, eine Alkoxygruppe oder eine Alkylgruppe ist;
- R12 ein Wasserstoffatom, eine Alkoxygruppe oder eine Alkylgruppe ist;
- R13 ein Wasserstoffatom oder eine Alkyl- oder eine Alkoxygruppe ist;
- R14 ein Wasserstoffatom oder eine Alkyl- oder Alkoxygruppe ist;
oder 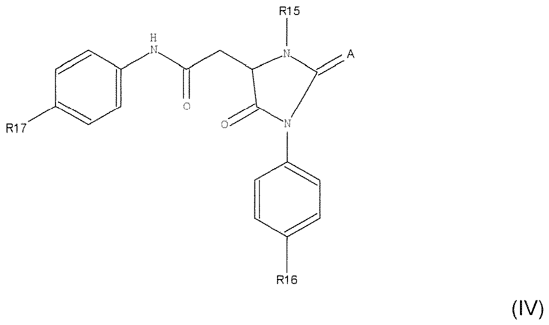 worin
- A ein Sauerstoff- oder Schwefelatom ist;
- R15 eine gesättigte oder nicht-gesättigte Kohlenstoffkette mit 1, 2 oder 3 Kohlenstoffatomen ist, welche durch eine substituierte oder nicht-substituierte aromatische Gruppe, einen substituierten oder nicht-substituierten Heterozyklus oder eine Amingruppe, welche Teil eines substituierten oder nicht-substituierten Heterozyklus ist;
- R16 ein Wasserstoffatom, ein Halogen oder eine Alkoxy-gruppe ist;
- R17 ein Wasserstoffatom, eine Alkoxygruppe oder eine Acetoxygruppe ist; 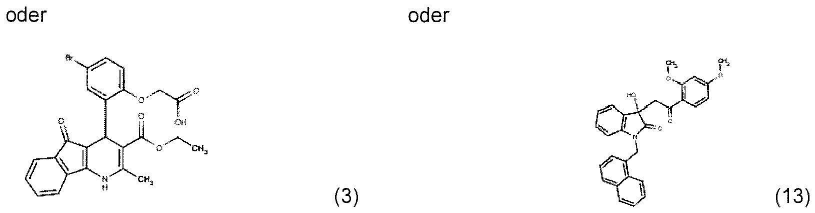 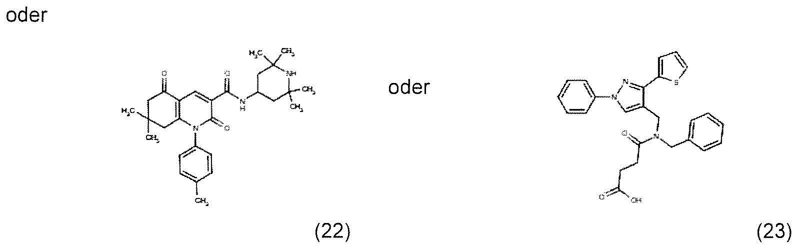 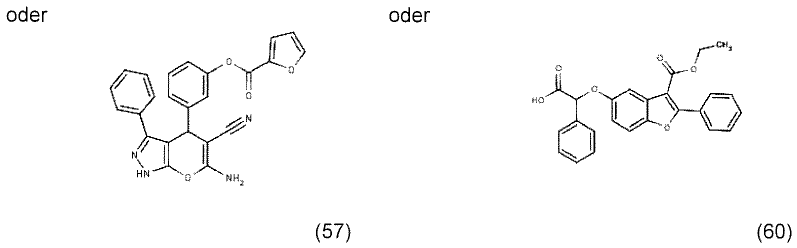 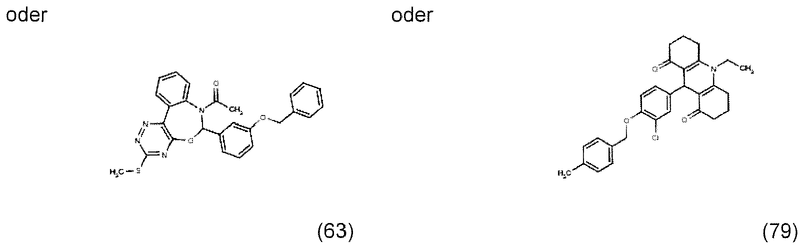 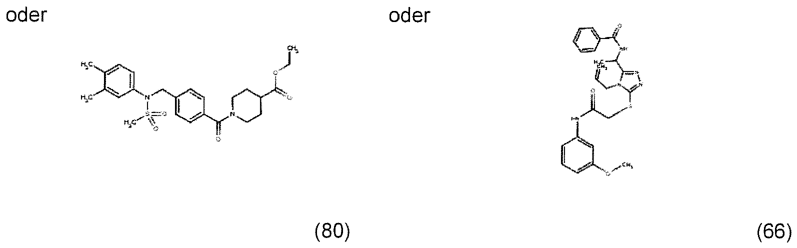 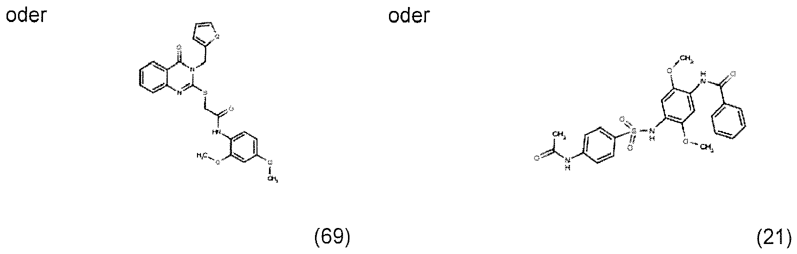 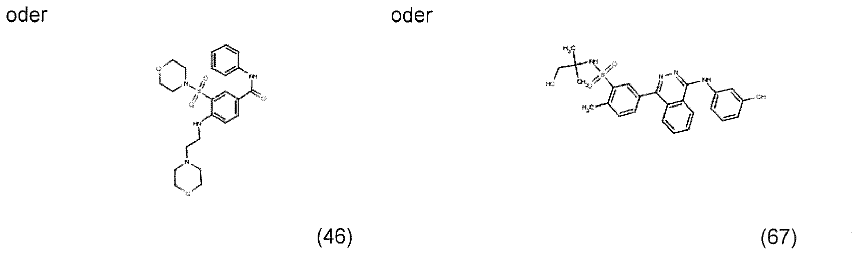 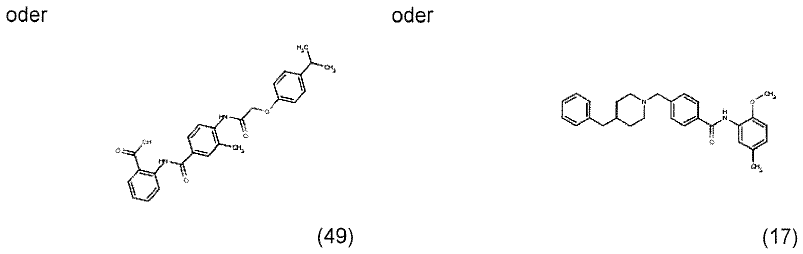 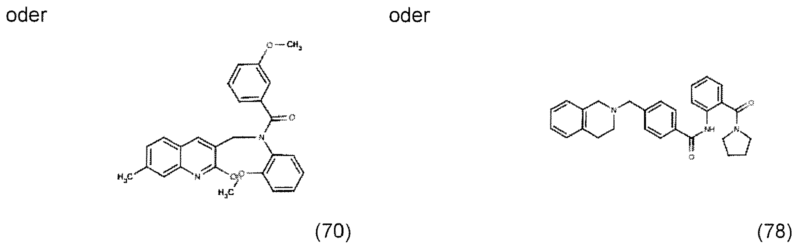 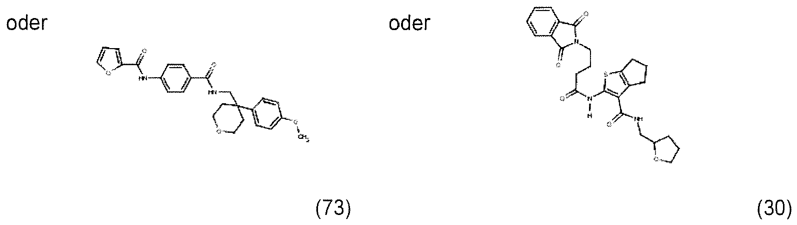 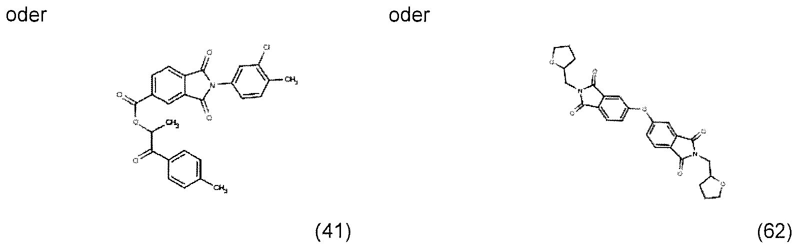 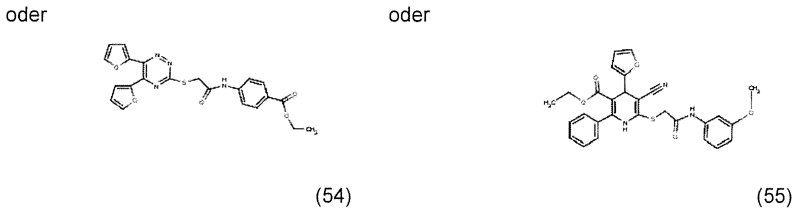 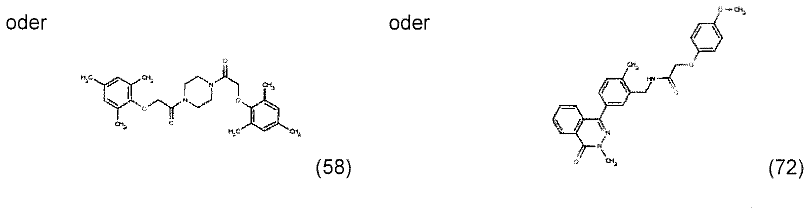 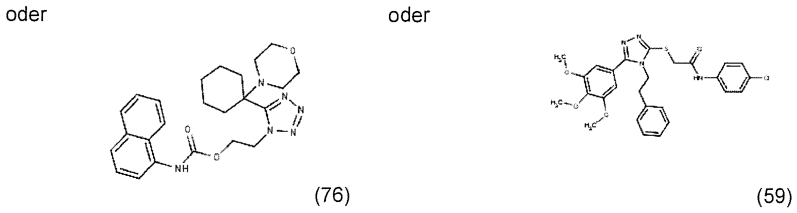 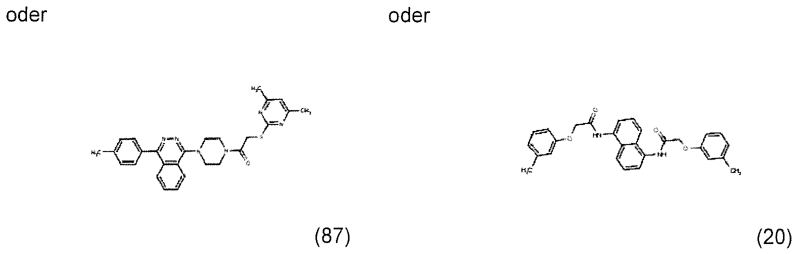 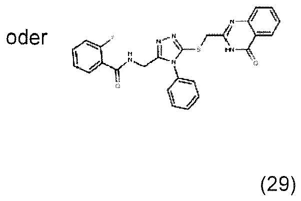 oder ein Stereoisomer, Racemat oder pharmazeutisch annehmbares Salz von C-13 oder dem äquivalenten Molekül ist;
worin, wenn das Molekül aus den Molekülen der folgenden Formeln ausgewählt wird 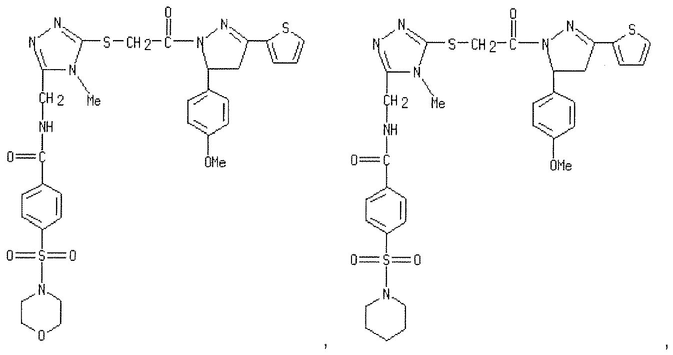 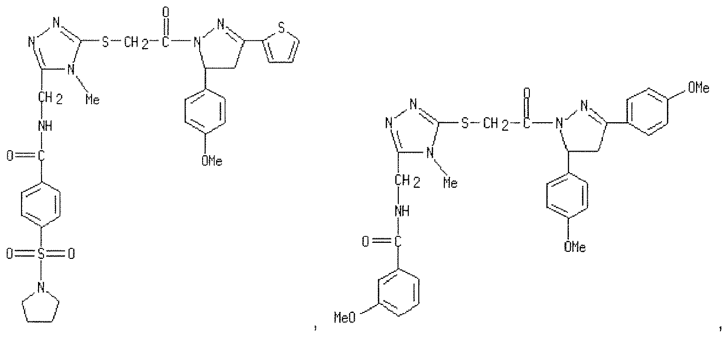 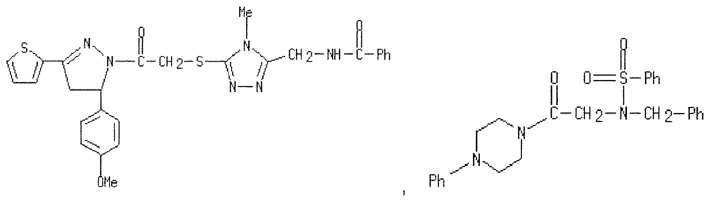 und 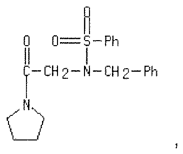 es nicht in Kombination mit einem Agonisten eines Glucocorticoid-Rezeptors verwendet wird.

2. Molekül zur Verwendung nach Anspruch 1, worin der Antikörper oder das Antikörperfragment an das humane Protein Tyrosinkinase Syk in Höhe eines Epitops bindet, umfassend wenigstens 5 der Reste 65 bis 74 der Aminosäurensequenz des humanen Syk-Proteins, dargestellt in SEQ ID-Nr. 1.

3. Molekül zur Verwendung nach Anspruch 1 oder 2, worin die dreidimensionale Tasche gleichermaßen den in Position 9 liegenden Serinrest, den in Position 43 liegenden Glutaminrest, den in Position 51 liegenden Phenylalaninrest, den in Position 66 liegenden Isoleucinrest, die in den Positionen 67 und 69 liegenden Glutamatreste, den in Position 70 liegenden Leucinrest, den in Position 71 liegenden Asparaginrest, den in Position 72 liegenden Glycinrest, den in Position 73 liegenden Threoninrest, den in Position 74 liegenden Tyrosinrest, den in Position 75 liegenden Alaninrest des humanen Syk-Proteins umfasst, dessen Sequenz durch SEQ ID-Nr. 1 veranschaulicht ist.

4. Molekül zur Verwendung nach einem jeden der Ansprüche 1 bis 3, worin das Molekül zur Vorbeugung oder Behandlung von Hyperempfindlichkeitsreaktionen vom Typ I verwendet wird und worin das Molekül in vitro zu 50% die Mastozytendegranulierung inhibiert, welche von IgE abhängig ist, auf eine Konzentration (CI50) zwischen 1 ng/ml und 1mg/ml.

5. Molekül zur Verwendung nach Anspruch 4, worin der Syk umfassende Stoffwechselweg einen Weg der Aktivierung von Mastozyten und von Basophilen ist.

6. Molekül zur Verwendung nach Anspruch 4 oder 5, worin die Krankheit allergisches Asthma, allergische Konjunktivitis, allergische Rhinitis, Anaphylaxie, angioneurotisches Ödem, Nesselsucht, Eosinophilie oder eine Allergie gegen ein Antibiotikum ist.

7. Molekül zur Verwendung nach einem jeden der Ansprüche 5 bis 6, worin das Molekül keine weitere Wirkung auf den das humane Syk-Protein (SEQ ID-Nr. 1) umfassenden Stoffwechselweg besitzt, als jene, welche zur Degranulierung von Mastozyten führen und/oder auf Hyperempfindlichkeitsreaktionen vom Typ I, insbesondere auf die Antikörperantwort, welche auf eine Immunisierung durch ein Tymo-abhängiges Antigen folgt oder auf die Syk abhängige Rekrutierung von Neutrophilen.

8. Molekül zur Verwendung nach einem jeden der Ansprüche 1 bis 3, worin der Syk umfassende Stoffwechselweg ein Weg der Aktivierung von B-Lymphozyten, T-Lymphozyten, Neutrophilen, Eosinophilen, NK-Zellen, Plättchen, Erythrozyten, Osteoklasten, Epithelialzellen oder Krebszellen ist.

9. Molekül zur Verwendung nach einem jeden der Ansprüche 1 bis 8, worin das Molekül in Kombination mit einem weiteren therapeutischen Molekül verwendet wird und worin das weitere therapeutische Molekül
- ebenfalls ein Molekül ist, das zur Vorbeugung oder Behandlung einer von einem Syk umfassenden Stoffwechselweg abhängt oder
- ein Molekül ist, das zur Vorbeugung oder Behandlung einer Krankheit verwendet wird, die nicht von dem Syk umfassenden Stoffwechselweg abhängig ist.

10. Molekül zur Verwendung nach einem jeden der Ansprüche 1 bis 9, worin das Molekül zur Verabreichung auf oralem, sublingualem, nasalem, über das Auge, lokalem, intravenösem, intraperitonealem, subkutanen, als Aerosol oder durch Inhalation an erwachsene menschliche Patienten, Kinder oder Neugeborene bestimmt ist und in Dosen, enthalten zwischen 0,01 mg/kg bis 200 mg/kg.

11. Molekül zur Verwendung nach Anspruch 10, worin das äquivalente Molekül aus den Molekülen mit den nachfolgenden Formeln ausgewählt ist:
• Formel (I), worin
- R1
∘ eine nicht substituierte oder durch ein F-Atom oder Cl-Atom, durch eine Methyl- oder Ethylgruppe, durch eine N,N-Dimethyl-sulfonamid-Gruppe oder durch zwei Gruppen, ausgewählt aus den Methyl-, Ethyl-, Hydroxy-, Methoxy- und Ethoxy-Gruppen substituierte Phenylgruppe,
∘ oder eine Gruppe 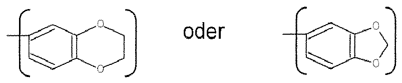 oder 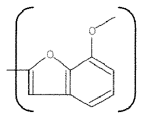
∘ oder eine durch Methyl-, Ethyl-, Hydroxy-, Methoxy- oder Ethoxygruppe substituierte Furan-gruppe,
∘ oder eine nicht-substituierte oder durch eine Methyl-, Ethyl-, Hydroxy-, Methoxy, oder Ethoxy-Gruppe substituierte Thiophenegruppe ist;
- R2
∘ eine Gruppe 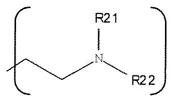 ist,
worin R21 und R22 Kohlenstoffatome sind, wovon jedes zu einer Alkylkette mit 1, 2 oder 3 Kohlenstoffatomen gehört oder auch beide mit dem Stickstoffatom, an welches sie gebunden sind, zu einem gleichen, gesättigten oder nicht-gesättigten Heterozyklus gehören, welcher gleichermaßen auch ein Sauerstoffatom oder ein zweites Stickstoffatom umfasst,
∘ eine Gruppe 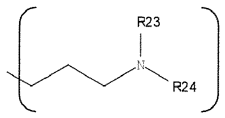 ist,
worin R23 und R24 Kohlenstoffatome sind, welche zu einer Alkylkette mit 1, 2 oder 3 Kohlenstoffatomen gehört oder beide mit dem Stickstoffatom, an welches sie gebunden sind, zu einem gleichen, gesättigten oder nicht-gesättigten Heterozyklus gehören, welcher auch ein Sauerstoffatom oder ein zweites Stickstoffatom umfasst,
∘ oder eine Gruppe 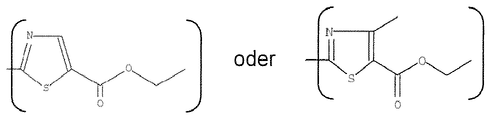 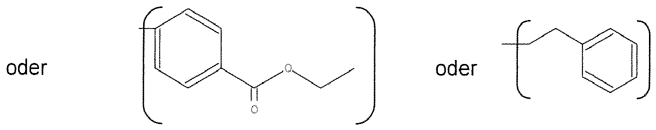 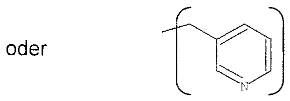 ist,
- und R3
∘ eine nicht-substituierte 2-Pyridinyl-, 3-Pyridi-nyl- oder 4-pyridinyl-Gruppe
∘ oder eine nicht-substituierte oder durch eine Benzoxygruppe und/oder durch eine Hydroxygruppe und/oder durch eine Methylgruppe und/oder durch eine Ethylgruppe und/oder durch eine Propylgruppe und/oder durch ein oder zwei Atome Br, F oder Cl und/oder durch ein bis drei Hydroxy-, Methoxy- oder Ethoxygruppe substituierte Phenylgruppe ist,
oder
• Formel (II), worin
- R4 eine gesättigte oder ungesättigte Kohlenstoffkette mit 1, 2 oder 3 Kohlenstoffatomen ist;
- R5 eine Phenylgruppe oder eine durch eine substituierte oder nicht-substituierte Phenylgruppe
oder durch eine Gruppe 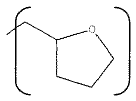 substituierte sekundäre Amingruppe ist,
- R6 ein Wasserstoff- oder Chloratom oder eine Methyl-, Ethyl-, Hydroxy-, Methoxy- oder Ethoxygruppe ist;
- R7 ein Wasserstoff- oder Chloratom oder eine Methyl-, Ethyl-, Hydroxy-, Methoxy- oder Ethoxygruppe ist;
- R8 ein Wasserstoff- oder Chloratom oder eine Methyl-, Ethyl-, Hydroxy-, Methoxy- oder
Ethoxygruppe ist;
oder
• Formel (III), worin
- R9 ein Wasserstoffatom und die Gruppe R10 eine substituierte oder nicht-substituierte Phenylgruppe ist, oder die Gruppen R9 und R10 Teil desselben substituierten oder nicht-substituierten Heterozyklus, umfassend zwei Stickstoffatome und 4 Kohlenstoffatome, sind;
- R11 ein Wasserstoffatom oder eine Methyl-, Ethyl-, Hydroxy-, Methoxy- oder Ethoxygruppe ist;
- R12 ein Wasserstoffatom oder eine Methyl-, Ethyl-, Hydroxy-, Methoxy- oder Ethoxygruppe ist;
- R13 ein Wasserstoffatom oder eine Methyl-, Ethyl-, Hydroxy-, Methoxy- oder Ethoxygruppe ist;
- R14 ein Wasserstoffatom oder eine Methyl-, Ethyl-, Hydroxy-, Methoxy- oder Ethoxygruppe ist:
oder
• Formel (IV), worin
- A ein Sauerstoff- oder Schwefelatom ist;
- R15 eine Gruppe 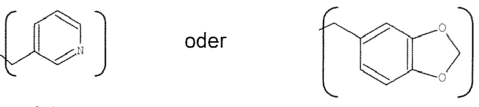 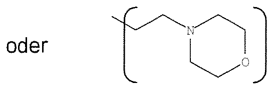 ist;
- R16 ein Wasserstoff- oder Chloratom oder eine Methyl-, Ethyl-, Hydroxy-, Methoxy- oder Ethoxygruppe ist;
- R17 eine Methyl-, Ethyl-, Hydroxy-, Methoxy-, Ethoxy-, Acetoxy-, Methoxycarbonyl- oder Ethoxycarbonylgruppe ist.

12. Molekül zur Verwendung gemäß Anspruch 10, worin das äquivalente Molekül aus den folgenden Molekülen ausgewählt ist:

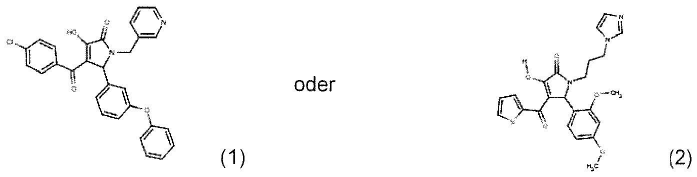

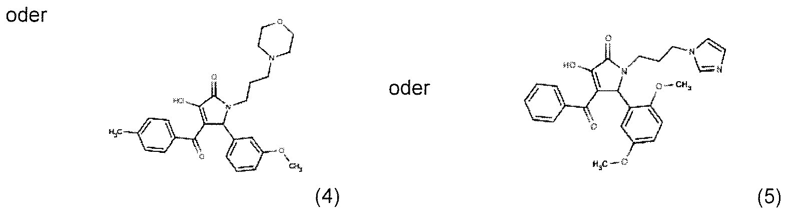

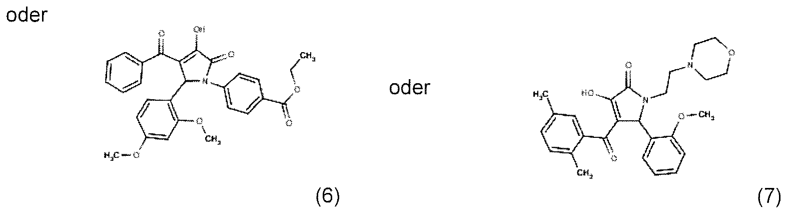

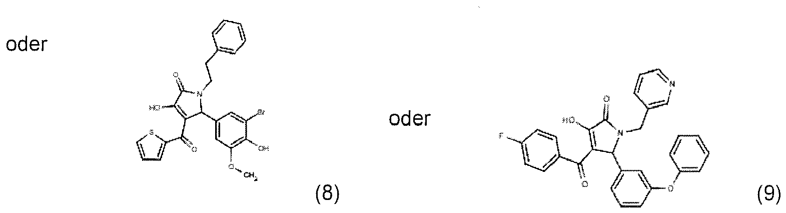

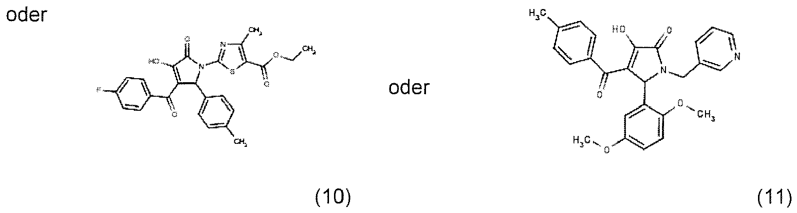

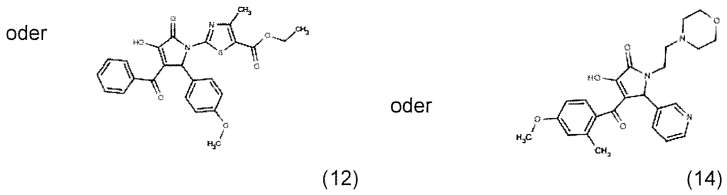

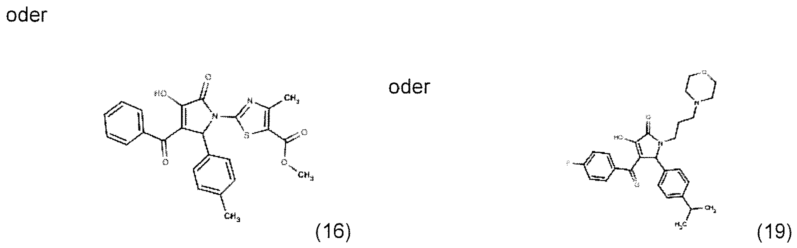

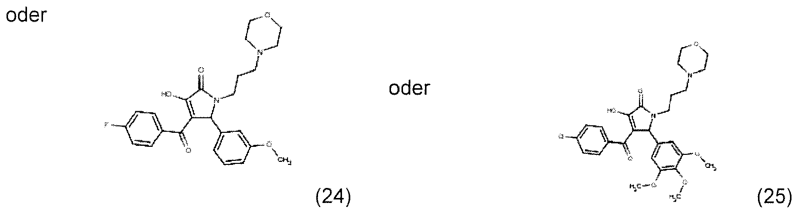

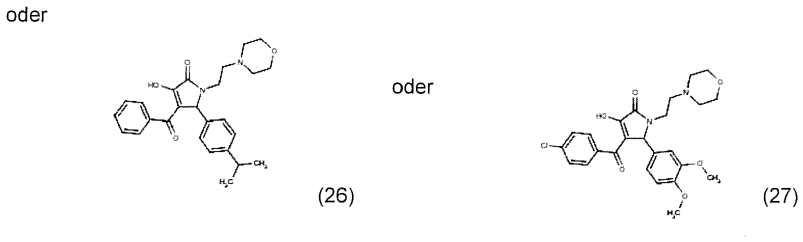

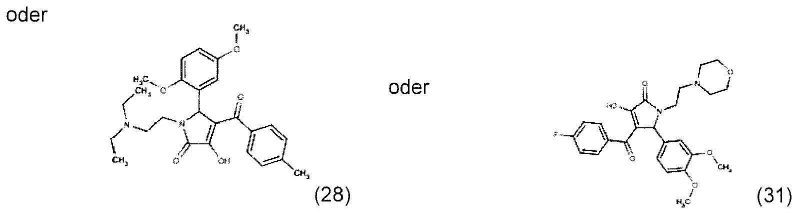

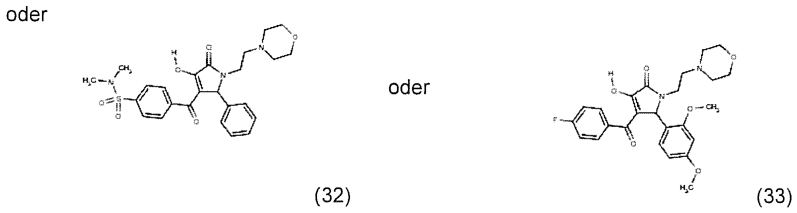

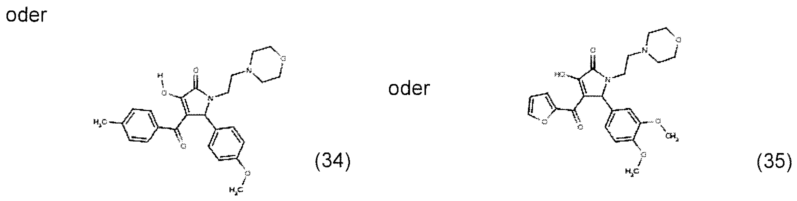

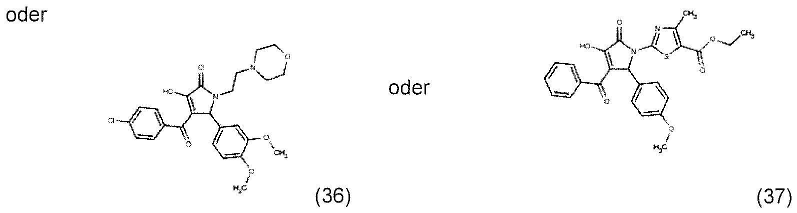

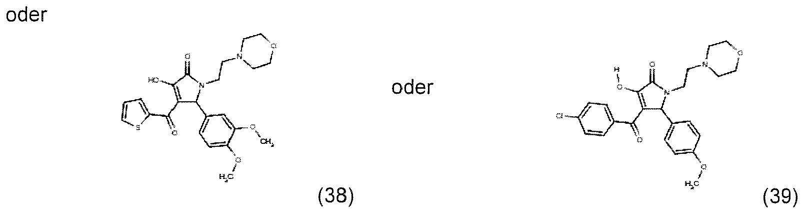

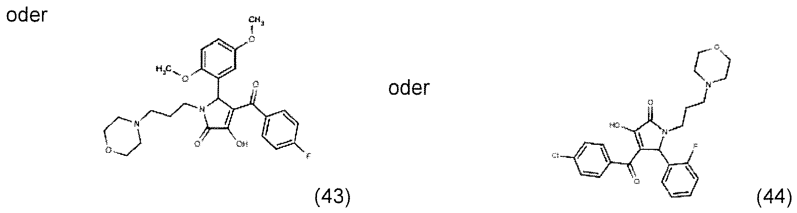

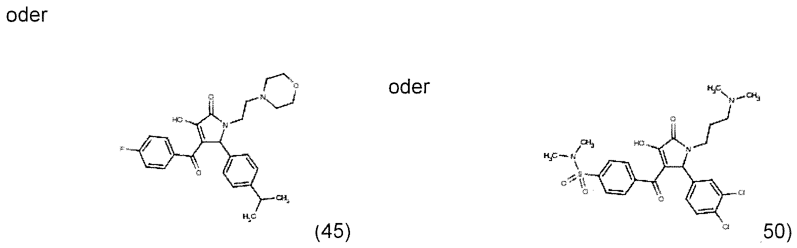

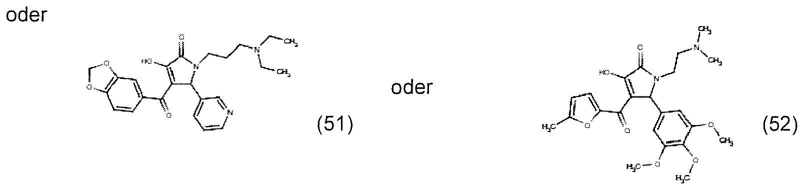

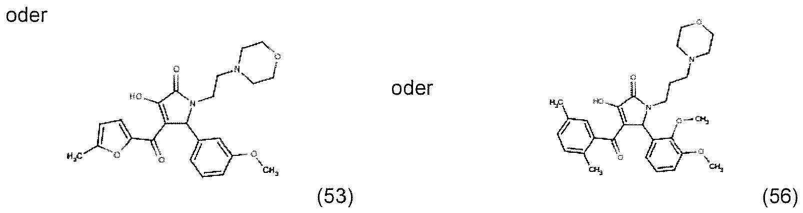

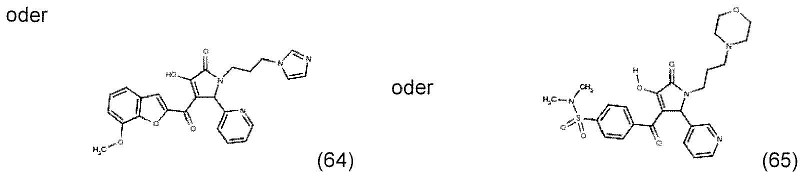

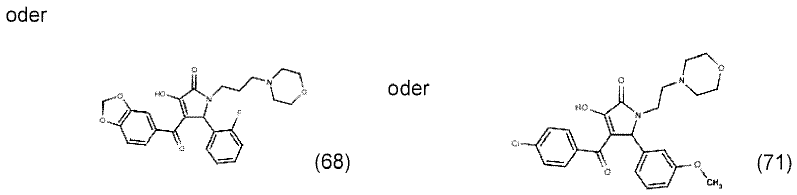

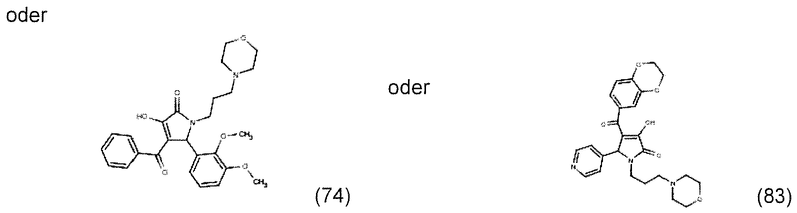

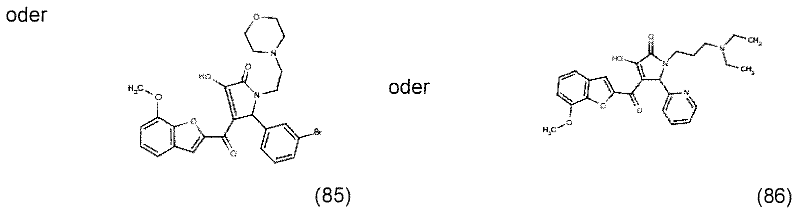

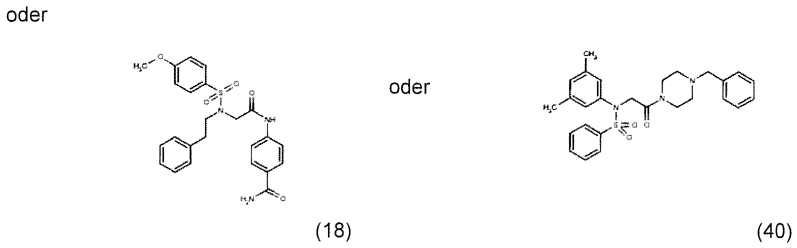

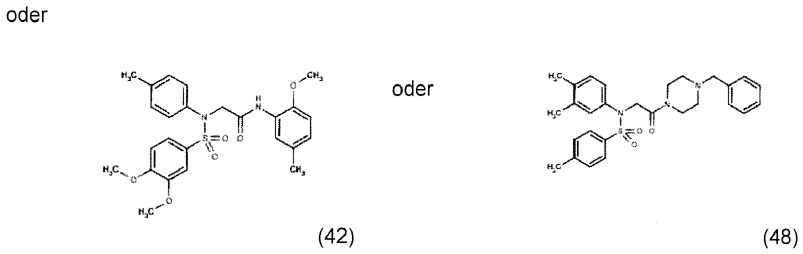

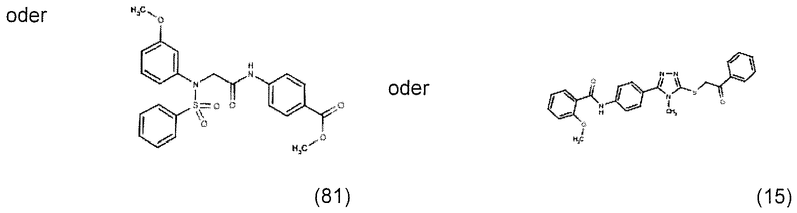

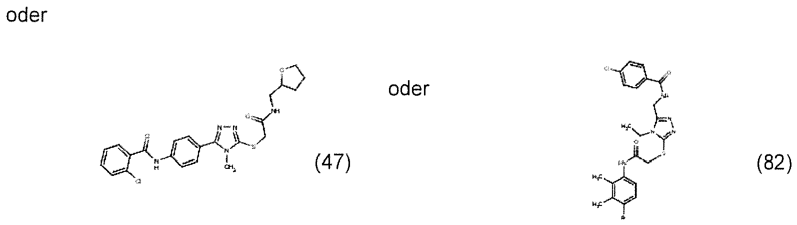

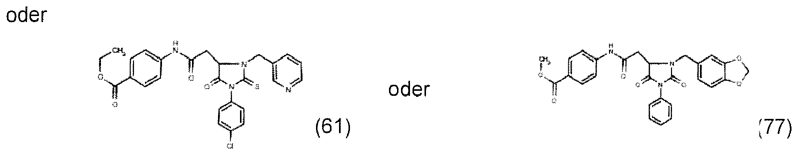

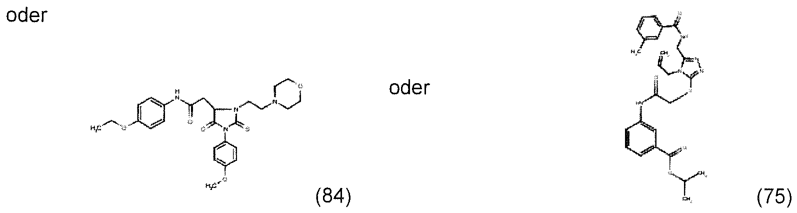

13. Zusammensetzung, umfassend ein Molekül gemäß einem jeden der Ansprüche 1 bis 12 und einen pharmakologisch annehmbaren Träger zur Verwendung bei der Vorbeugung oder der Behandlung einer Krankheit, welche von einem Syk umfassenden Stoffwechselweg abhängt, bei Mensch oder Tier, worin die Krankheit durch eine Hypersensibilität vom Typ 1 verursacht wird oder rheumatoide Polyarthris, eine Immunkrankheit, eine Entzündung oder Krebs ist.

14. Zusammensetzung gemäß Anspruch 13, worin die Zusammensetzung des weiteren ein weiteres therapeutisches Molekül umfasst.

15. Verfahren zur Identifizierung eines organischen Moleküls mit einem zwischen 50 und 2500 Dalton enthaltenen Molekulargewicht, das sich an das Protein Tyrosinkinase Syk bindet und in vitro wenigstens 10% der Bindung
(i) des Antikörperfragmentes G4G11 (SEQ ID-Nr. 2), oder
(ii) eines Antikörpers oder eines Antikörperfragments, das sich an das humane Protein Tyrosinkinase Syk auf Höhe eines Epitops, umfassend wenigstens einen der Reste 65 bis 74 der Aminosäurensequenz des humanen Protein Tyrosinkinase Syk, dargestellt durch die Sequenz SEQ ID-Nr. 1, oder
(iii) eines Antikörpers oder eines Antikörperfragments, das sich an das humane Protein Tyrosinkinase Syk bindet und wenigstens 10% die Bindung des Antikörperfragments G4G11 mit dem humanen Protein Tyrosinkinase Syk (SEQ ID-Nr. 1) inhibiert,
an das humane Protein Tyrosinkinase Syk oder eine ihrer Varianten beim Tier inhibieren kann, umfassend wenigstens die folgenden Schritte:
a) das Durchsuchen einer Bank organischer Moleküle mit zwischen 50 und 2500 Dalton Molekulargewicht nach jenen, welche sich an das Syk-Protein in Höhe der dreidimensionalen Tasche der Bindung an dem Syk-Protein eines Moleküls binden können, ausgewählt unter den Molekülen der Formel C-13, I, II, IV oder 1 bis 87, wie oben definiert;
b) Auswählen unter den in klein a) identifizierten Molekülen nach jenen, welche wenigstens in vitro 10% Antikörperbindung oder Antikörperfragmentbindung (i), (ii) oder (iii) an das Syk-Protein inhibieren können.

16. Verfahren nach Anspruch 15, worin das Molekül in Schritt a) ein C-13 Molekül, das Molekül (59) oder das Molekül (61) ist.

17. Verfahren nach Anspruch 15 oder 16, umfassend einen zusätzlichen Schritt der Identifizierung der dreidimensionalen Tasche der Bindung an das Syk-Protein des Moleküls, ausgewählt unter den Molekülen der Formel C-13, I, II, IV oder 1-87 vor dem Schritt a), worin der vorangehende Schritt durch "in silico"-Andocken durchgeführt wird und
einen zusätzlichen Schritt c) des Auswählens unter den in b) identifizierten Molekülen nach jenen, welche in vitro 50% Mastozytendegranulierung auf eine Konzentration (Cl50) inhibieren können, welche zwischen 1 ng/ml und 1mg/ml enthalten ist.

## Claims

1. Molecule binding with Syk tyrosine kinase protein at a three-dimensional cavity comprising the Arginine residue situated in position 68 and the two glutamic acid residues situated in positions 121 and 155 of human Syk protein, the sequence of which is illustrated by SEQ ID No. 1, and capable of inhibiting by at least 10% in vitro binding of
(i) antibody fragment G4G11 (SEQ ID No. 2), or
(ii) an antibody or antibody fragment which binds with human Syk tyrosine kinase protein on an epitope comprising at least one of residues 65 to 74 of the amino acid sequence of human Syk tyrosine kinase protein represented by the sequence SEQ ID No. 1, or
(iii) an antibody or antibody fragment which binds with human Syk tyrosine kinase protein and inhibiting by at least 10% the binding of antibody fragments G4G11 with human Syk tyrosine kinase protein (SEQ ID No. 1),
with human Syk tyrosine kinase protein or with any of the variants thereof in animals, for use in the prevention or treatment of a condition dependent on a metabolic pathway involving Syk in humans or animals, wherein said condition is caused by type I hypersensitivity, or is rheumatoid arthritis, an autoimmune disease, inflammation or cancer,
where said molecule is molecule C-13 having the formula 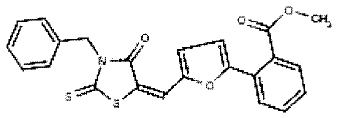 or an organic molecule functionally equivalent to molecule C-13 having a molecular weight between 50 and 2500 Dalton, selected from the molecules having any of the following formulas:
n 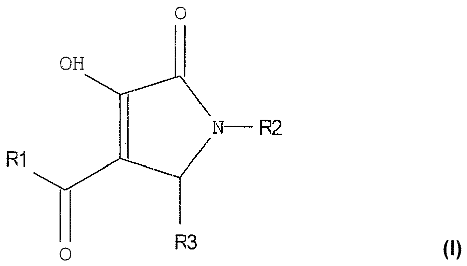
where
- R1 is an optionally substituted aromatic group, or an optionally substituted heterocycle comprising at least one S, O or N atom;
- R2 is an optionally substituted aromatic group, an optionally substituted heterocycle, an optionally saturated carbon chain, comprising an amine group, an optionally saturated carbon chain comprising an optionally substituted aromatic group or an optionally saturated carbon chain comprising an optionally substituted heterocycle comprising at least one S, O or N atom;
- R3 is an optionally substituted phenyl, 2-pyridinyl, 3-pyridinyl or 4-pyridinyl group;
or 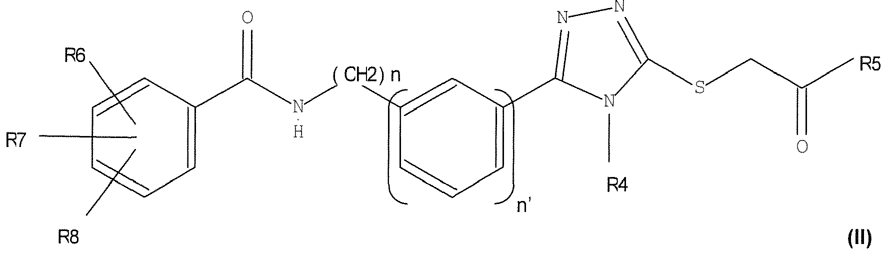
where
- n = 0 or 1; n' = 0 or 1;
- R4 is an optionally saturated carbon chain comprising 1 to 5 carbon atoms, optionally substituted with an aromatic group;
- R5 is an optionally substituted aromatic group or an optionally substituted amine group;
- R6 is a hydrogen atom, alkoxy group, alkyl group or halogen;
- R7 is a hydrogen atom, alkoxy group, alkyl group or halogen;
- R8 is a hydrogen atom, alkoxy group, alkyl group or halogen;
or 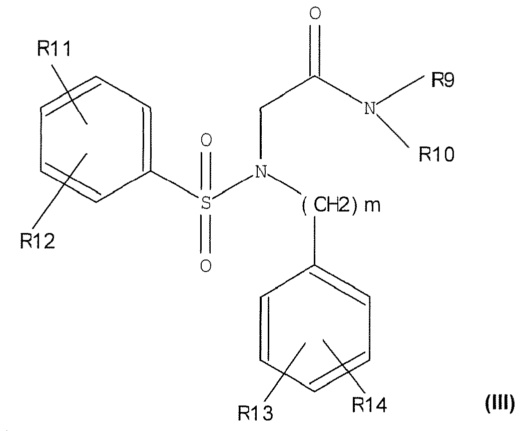
where
- m = 0, 1 or 2;
- R9 is a hydrogen atom and R10 is an optionally substituted phenyl group, or R9 and R10 are part of the same optionally substituted heterocycle, or R9 and R10 are part of the same optionally substituted aromatic group;
- R11 is a hydrogen atom, alkoxy group or alkyl group;
- R12 is a hydrogen atom, alkoxy group or alkyl group;
- R13 is a hydrogen atom or an alkyl or alkoxy group;
- R14 is a hydrogen atom or an alkyl or alkoxy group;
or 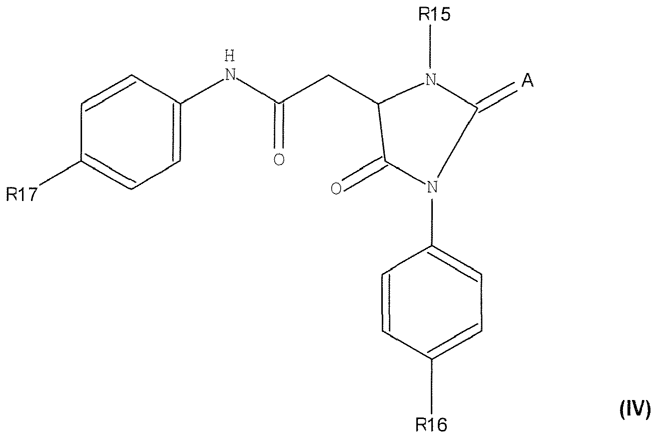
where
- A is an oxygen or sulphur atom;
- R15 is an optionally saturated carbon chain comprising 1, 2 or 3 carbon atoms, optionally substituted by an optionally substituted aromatic group, an optionally substituted heterocycle or an amine group belonging to optionally substituted heterocycle;
- R16 is a hydrogen atom, halogen or alkoxy group;
- R17 is a hydrogen atom, alkoxy group or acetoxy group; 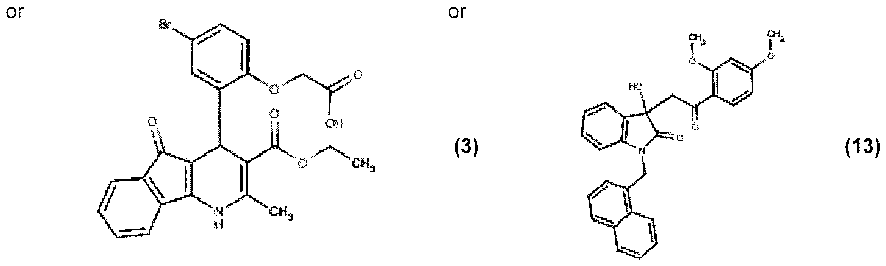 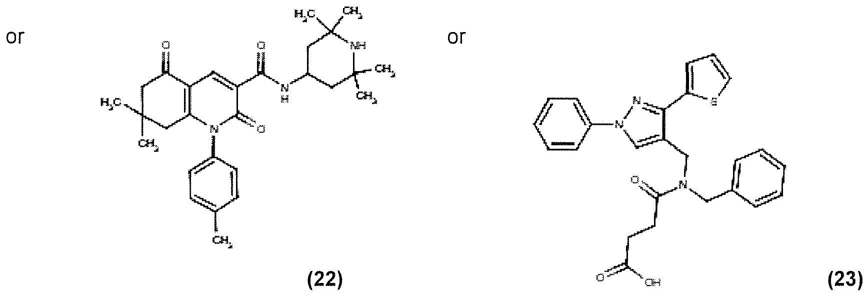 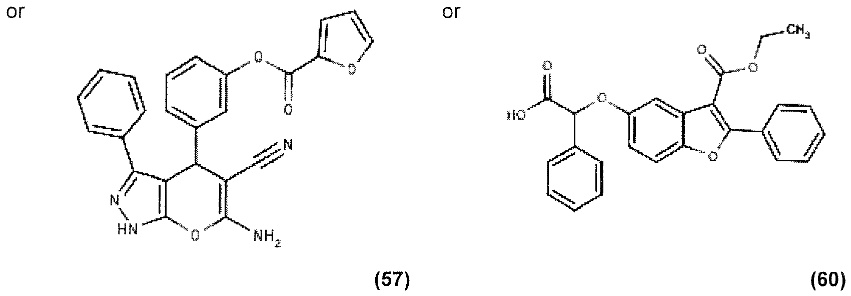 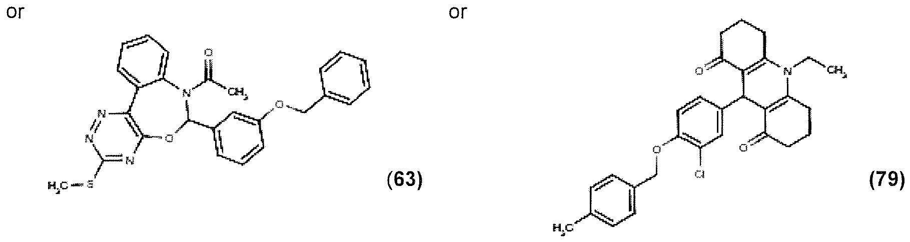 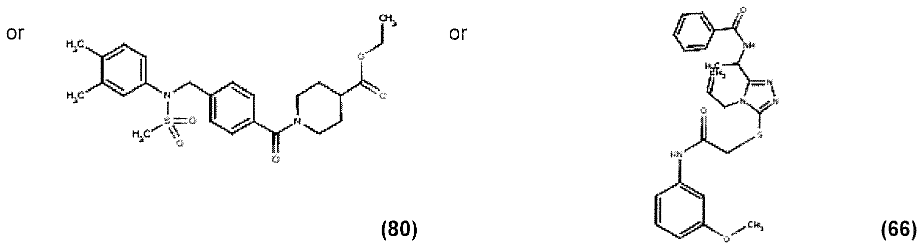 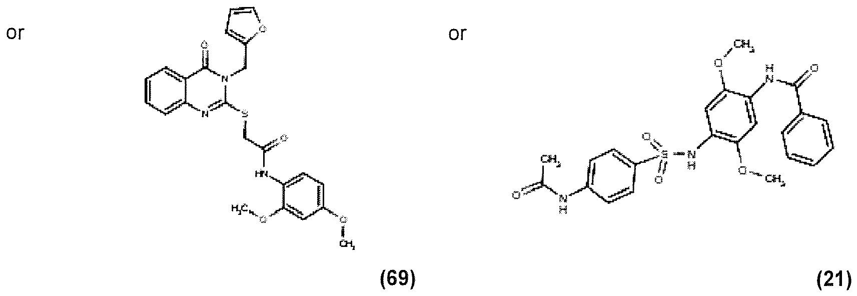 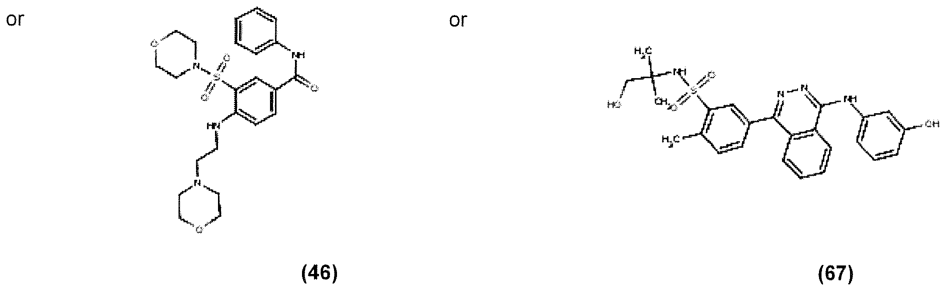 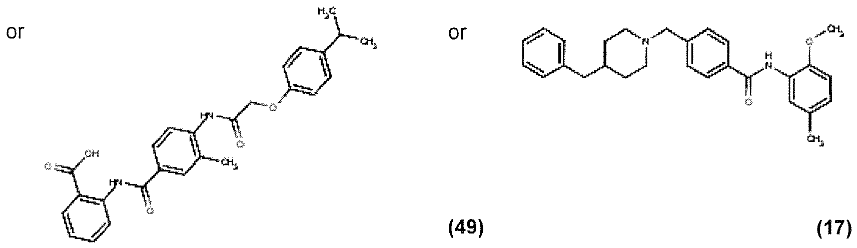 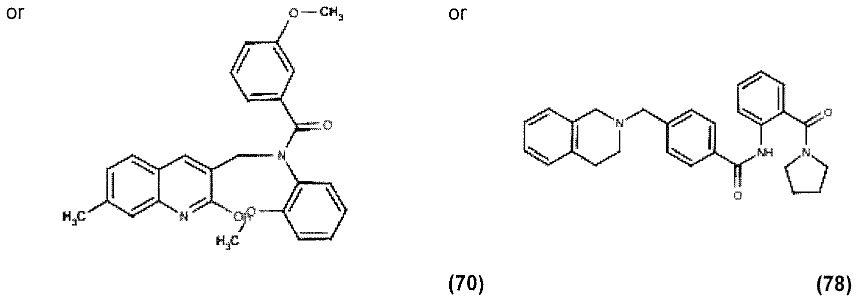 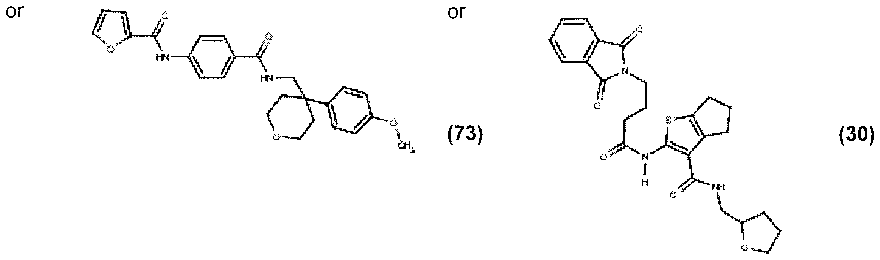 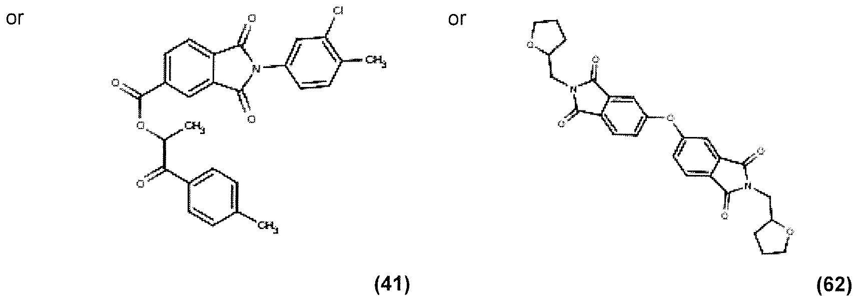 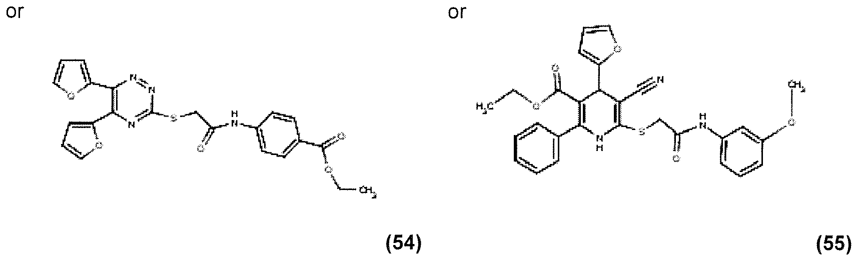 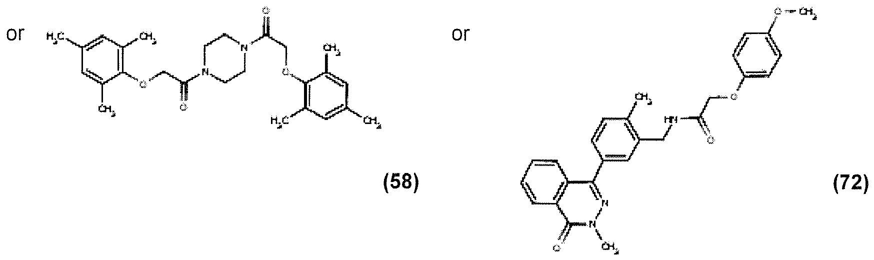 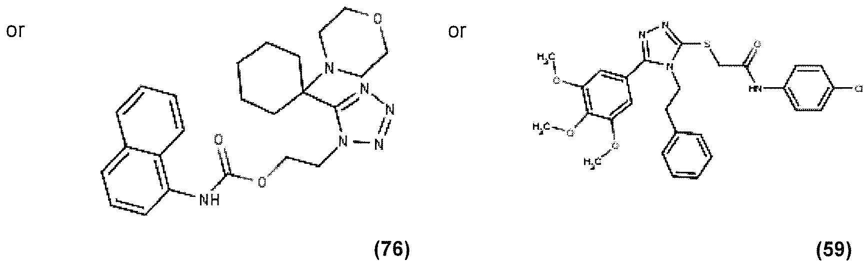 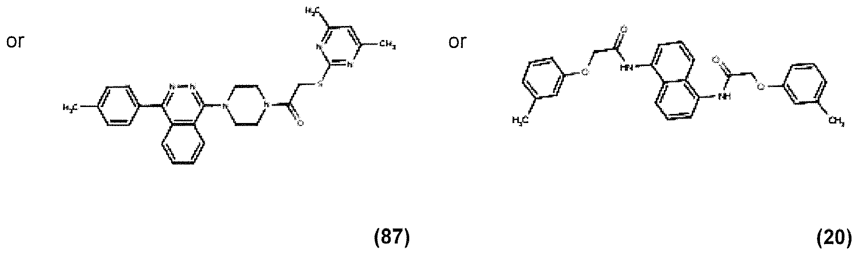 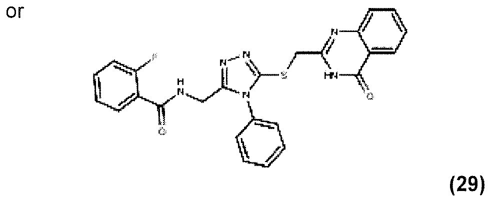
or a stereo-isomer, racemate or pharmacologically acceptable salt of C-13 or said equivalent molecule,
wherein if said molecule is selected from the molecules with the following formula 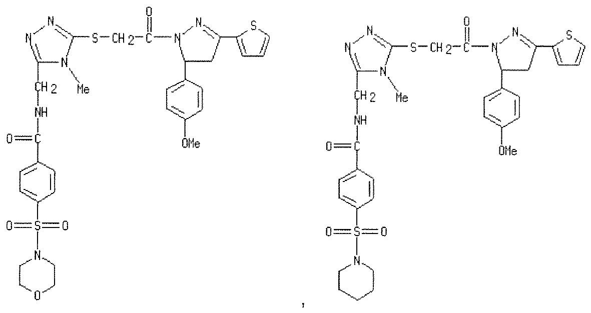 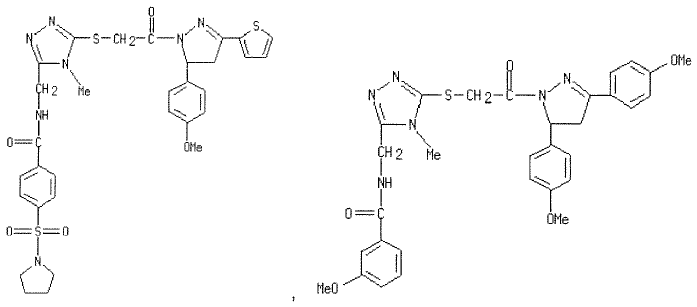 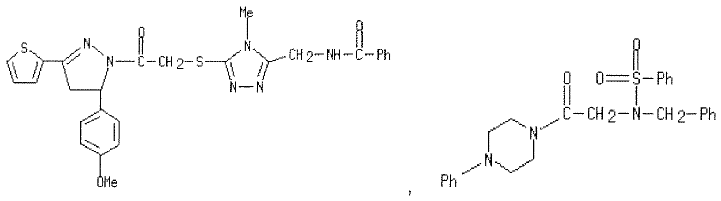 and 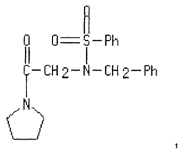 it is not used in combination with a glucocorticoid receptor agonist.

2. Molecule for its use according to claim 1, wherein said antibody or antibody fragment binds with human Syk tyrosine kinase protein on an epitope comprising at least 5 of residues 65 to 74 of the amino acid sequence of human Syk tyrosine kinase protein illustrated by SEQ ID No. 1.

3. Molecule for its use according to claim 1 or 2 wherein said three-dimensional cavity further comprises the Serine residue situated in position 9, the Glutamine residue situated in position 43, the Phenylalanine residue situated in position 51, the Isoleucine residue situated in position 66, the Glutamate residues situated in position 67 and 69, the Leucine residue situated in position 70, the Asparagine residue situated in position 71, the Glycine residue situated in position 72, the Threonine residue situated in position 73, the Tyrosine residue situated in position 74 and the Alanine residue situated in position 75 of human Syk protein, the sequence of which is illustrated by SEQ ID No. 1.

4. Molecule for its use according to any of claims 1 to 3 wherein said molecule is used for the prevention or treatment of type I hypersensitivity reactions, and wherein said molecule inhibits, by 50% *in vitro,* mast cell degranulation at a concentration (IC50) between 1ng/ml and 1 mg/ml..

5. Molecule for its use according to claim 4 wherein said metabolic pathway involving Syk is a mast cell or basophil activation pathway.

6. Molecule for its use according to any of claims 4 or 5 wherein said condition is allergic asthma, allergic conjunctivitis, allergic rhinitis, anaphylaxis, angioedema, urticaria, eosinophilia or an allergy to an antibiotic.

7. Molecule for its use according to any of claims 5 to 6 wherein said molecule has no effect on the metabolic pathways involving human Syk protein (SEQ ID No. 1) other than those giving rise to mast cell degranulation and/or type I hypersensitivity reactions, in particular on the antibody response which follows immunisation by a thymus-dependent antigen or on neutrophil-dependent Syk recruitment.

8. Molecule for its use according to any of claims 1 to 3 wherein said metabolic pathway involving Syk is a B lymphocyte, T lymphocyte, neutrophil, eosinophil, NK cell, platelet, erythrocyte, osteoclast, epithelial cell or cancer cell activation pathway.

9. Molecule for its use according to any of claims 1 to 8 wherein said molecule is used in combination with a further therapeutic molecule, and wherein said further therapeutic molecule
- is also a molecule used for the prevention or treatment of a condition dependent on a metabolic pathway involving Syk, or
- is a molecule used for the prevention or treatment of a condition non dependent on a metabolic pathway involving Syk.

10. Molecule for its use according to any of claims 1 to 9 wherein said molecule is intended to be administered by the oral, sublingual, nasal, ocular, local, intravenous, intraperitoneal, subcutaneous route, by aerosol or by inhalation, to adult, child or newborn human patients, and at doses between 0.01 mg/kg and 200mg/kg.

11. Molecule for its use according to claim 10 wherein said equivalent molecule is selected from the molecules having any of the following formulas:
• formula (I) where
- R1 is
o a phenyl group, optionally substituted by an F or Cl atom, a methyl or ethyl group, an N,N-dimethyl-sulphonamide or two groups selected from the methyl, ethyl, hydroxy, methoxy or ethoxy groups,
∘ or a group 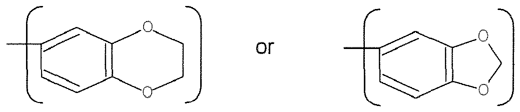 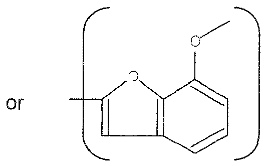
∘ a furan group optionally substituted by a methyl, ethyl, hydroxyl, methoxy or ethoxy group,
∘ a thiophene group optionally substituted by a methyl, ethyl, hydroxy, methoxy or ethoxy group;
- R2 is
∘ a group 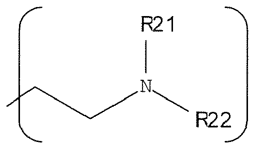 where R21 and R22 are carbon atoms each belonging to an alkyl chain comprising 1, 2 or 3 carbon atoms, or both belonging with the nitrogen atom with which they are bound to the same optionally saturated heterocycle also comprising an oxygen atom or a second nitrogen atom,
∘ or a group 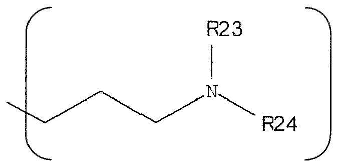 where R23 and R24 are carbon atoms each belonging to an alkyl chain comprising 1, 2 or 3 carbon atoms, or both belonging with the nitrogen atom with which they are bound to the same optionally saturated heterocycle also comprising an oxygen atom or a second nitrogen atom,
∘ or a group 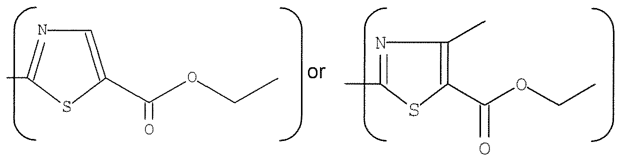 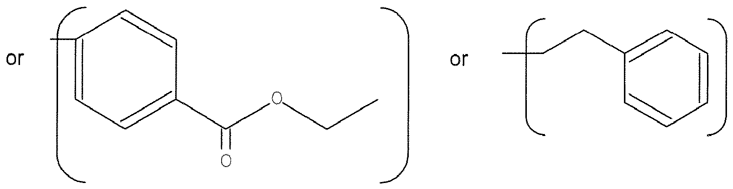 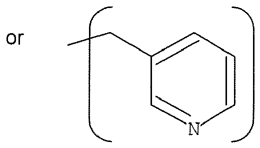
- and R3 is
∘ a non-substituted 2-pyridinyl, 3-pyridinyl or 4-pyridinyl group,
∘ a phenyl group optionally substituted by a benzoxy group, and/or by a hydroxyl group, and/or by a methyl group, and/or by an ethyl group, and/or by a propyl group, and/or by one or two Br, F or Cl atoms, and/or by one to three hydroxyl, methoxy or ethoxy groups.
or
• formula (II) where
- R4 is an optionally saturated carbon chain comprising 1, 2 or 3 carbon atoms;
- R5 is a phenyl group or a secondary amine group substituted by an optionally substituted phenyl group, or by a group 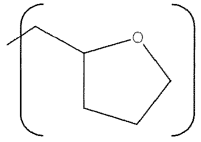
- R6 is a hydrogen or chlorine atom or a methyl, ethyl, hydroxyl, methoxy or ethoxy group;
- R7 is a hydrogen or chlorine atom or a methyl, ethyl, hydroxy, methoxy or ethoxy group;
- R8 is a hydrogen or chlorine atom or a methyl, ethyl, hydroxy, methoxy or ethoxy group.
or
• the formula **(III)** where
- R9 is a hydrogen atom and the group R10 is an optionally substituted phenyl group, or the groups R9 and R10 belong to the same optionally substituted heterocycle comprising 2 nitrogen atoms and 4 carbon atoms;
- R11 is a hydrogen atom or methyl, ethyl, hydroxy, methoxy or ethoxy group;
- R12 is a hydrogen atom or methyl, ethyl, hydroxy, methoxy or ethoxy group;
- R13 is a hydrogen atom or methyl, ethyl, hydroxy, methoxy or ethoxy group;
- R14 is a hydrogen atom or methyl, ethyl, hydroxy, methoxy or ethoxy group;
or
• formula **(IV)** where
- A is an Oxygen or Sulphur atom;
- R15 is a group 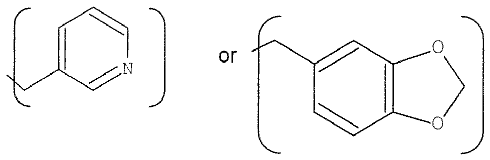 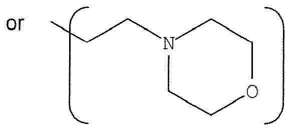
- R16 is a hydrogen or chlorine atom or a methyl, ethyl, hydroxy, methoxy or ethoxy group;
- R17 is a methyl, ethyl, hydroxy, methoxy, ethoxy, acetoxy, methoxycarbonyl or ethoxycarbonyl group.

12. Molecule according to claim 10 wherein said equivalent molecule is selected from the following molecules:

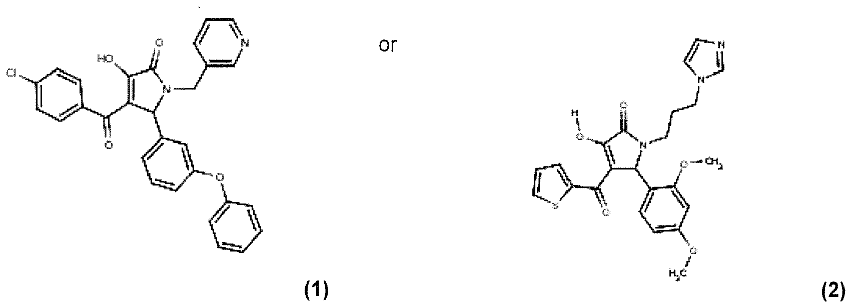

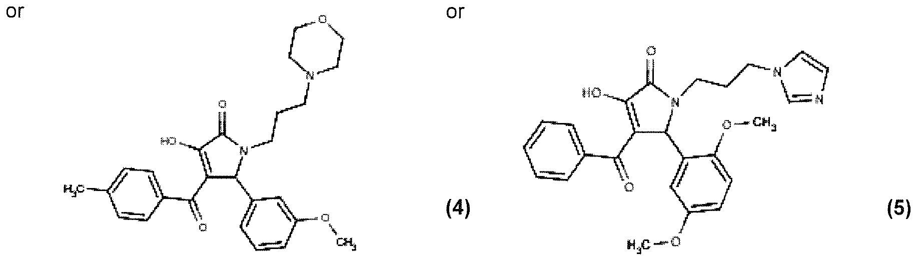

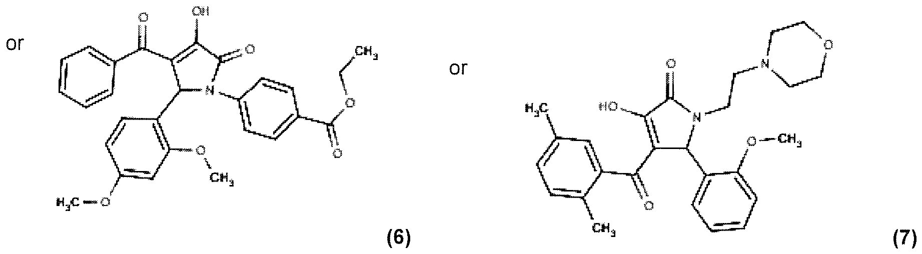

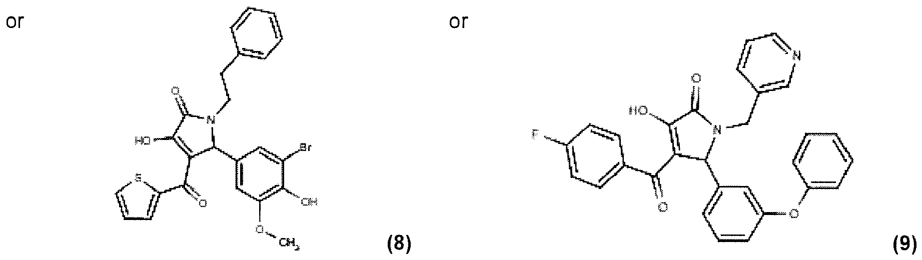

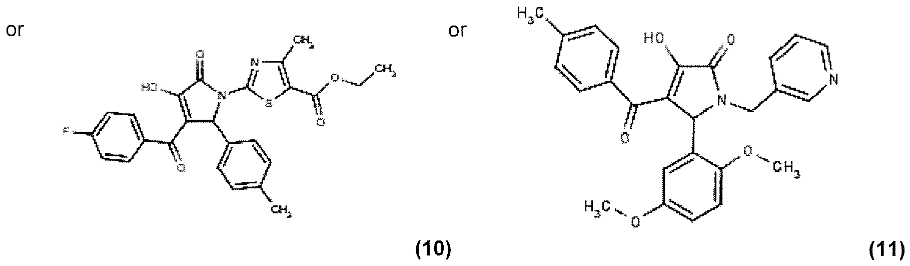

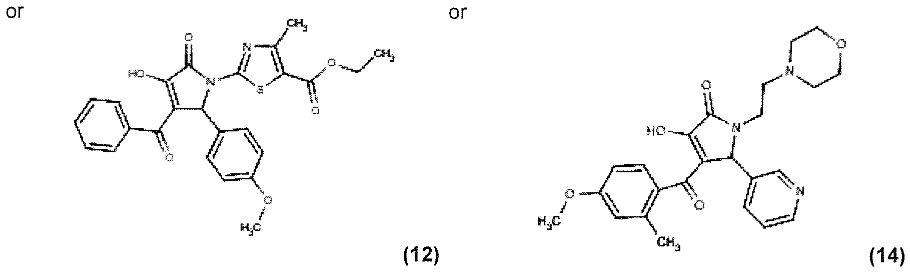

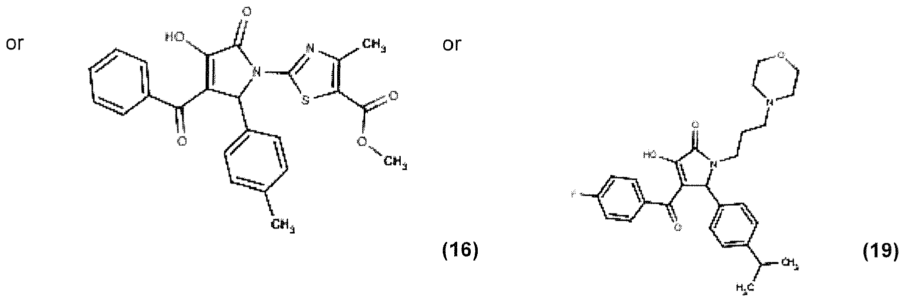

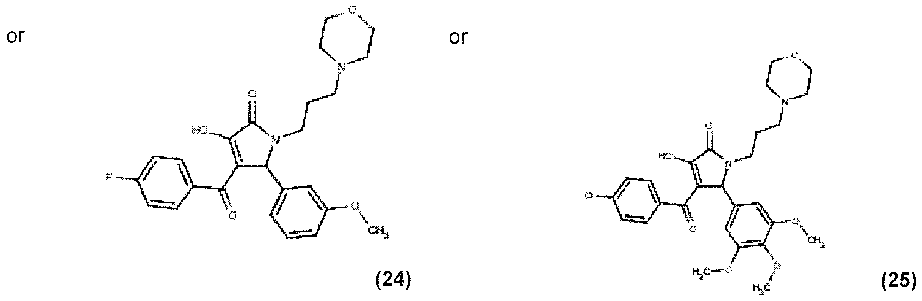

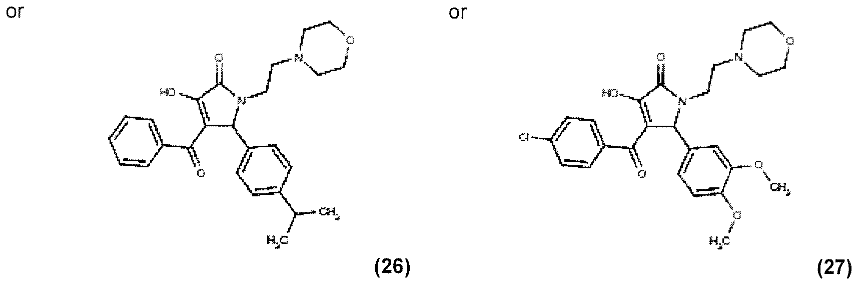

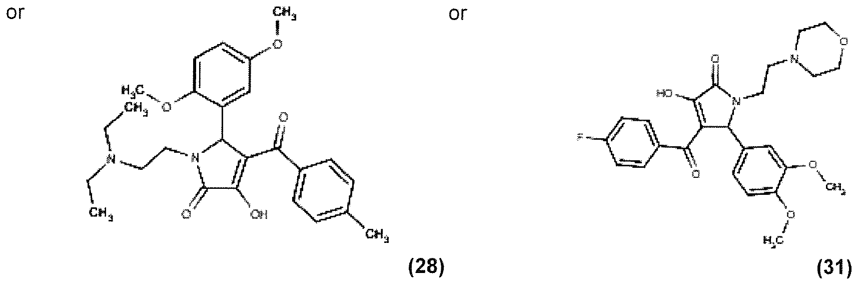

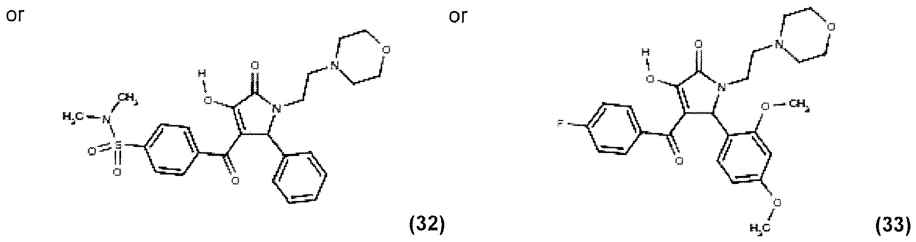

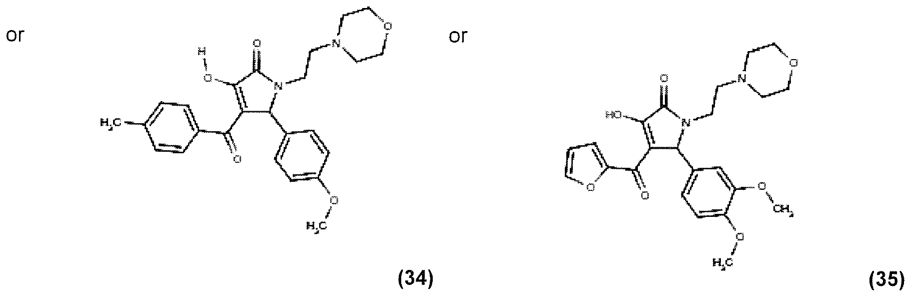

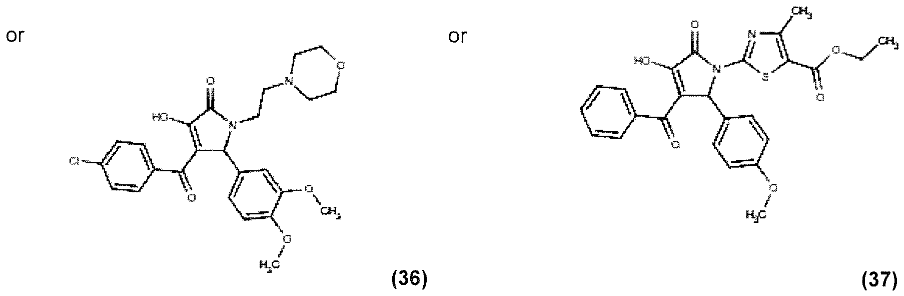

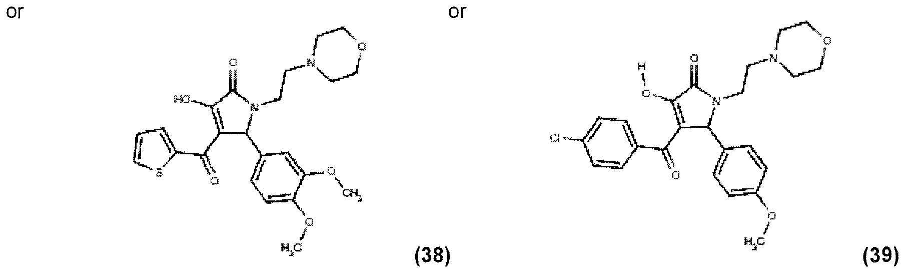

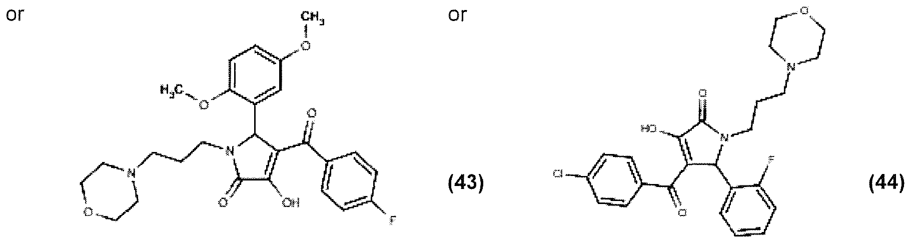

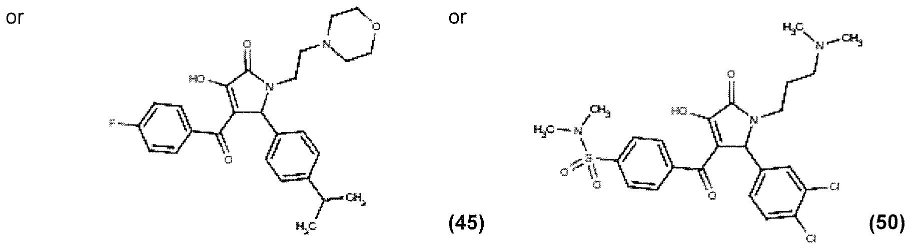

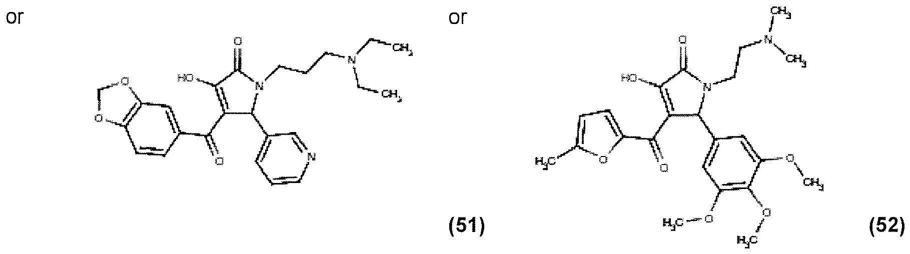

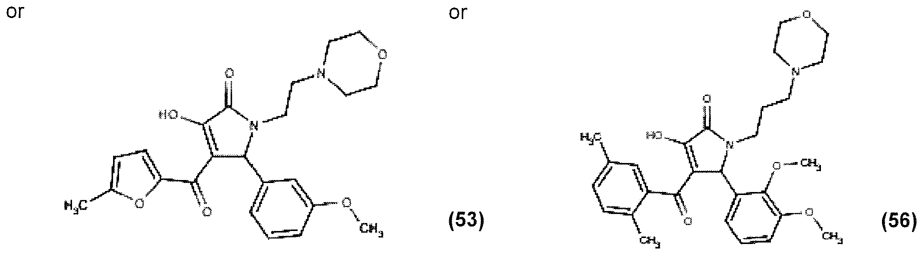

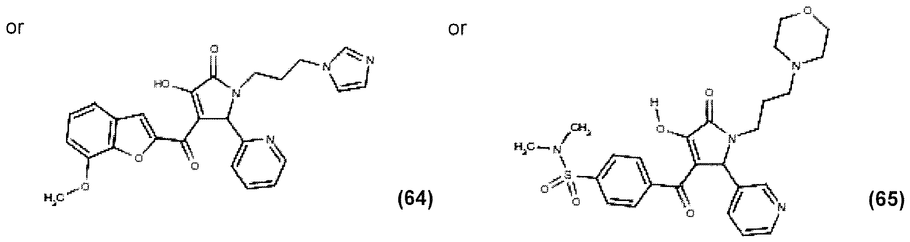

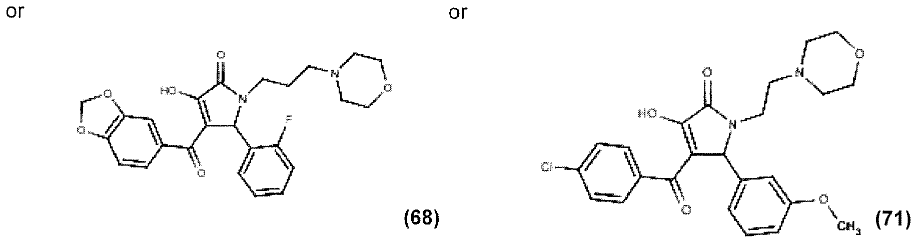

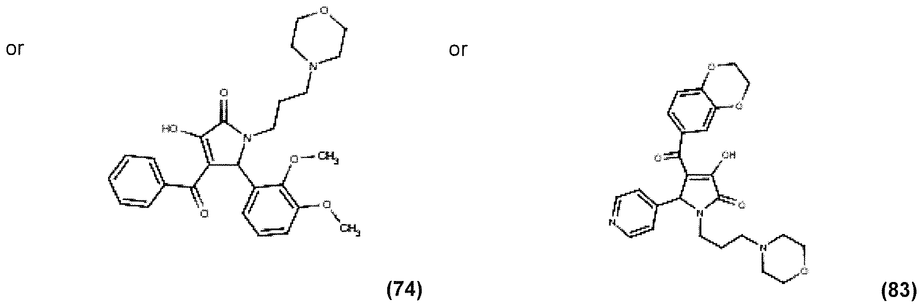

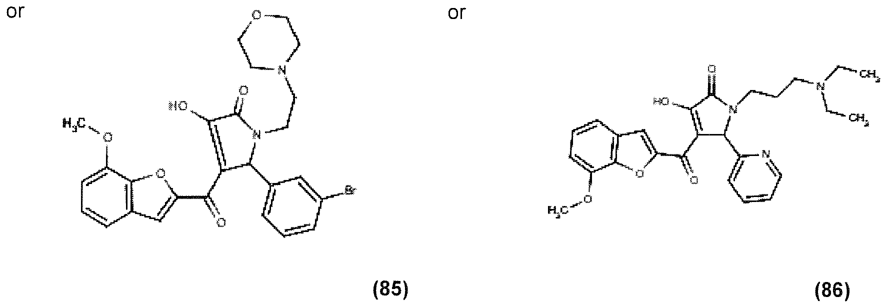

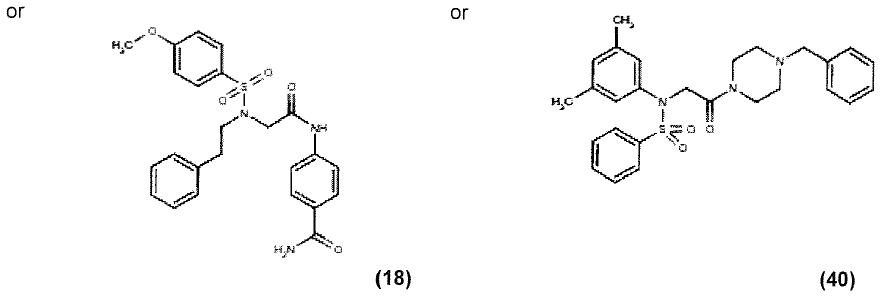

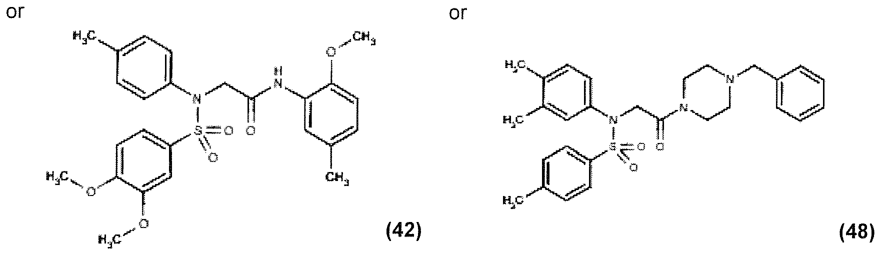

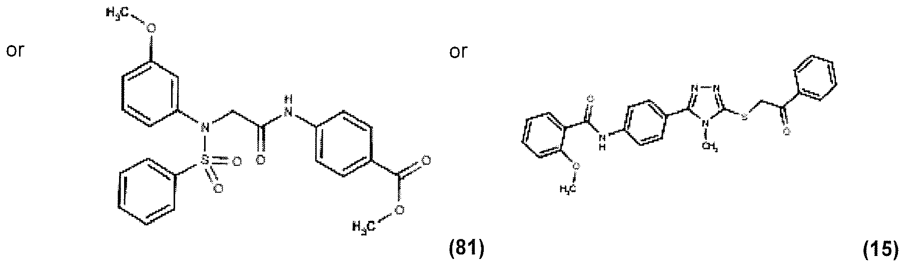

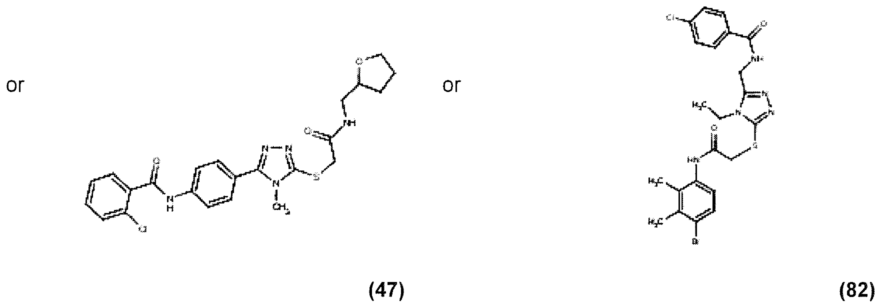

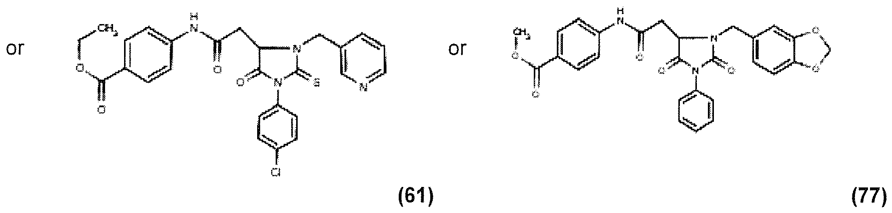

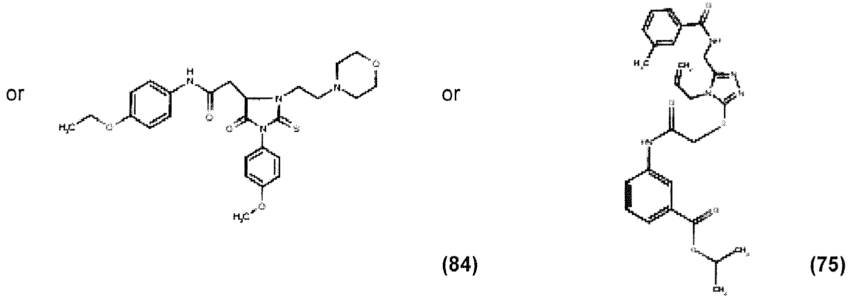

13. Composition comprising a molecule according to any of claims 1 to 12 and a pharmacologically acceptable excipient for use in the prevention or treatment of a condition dependent on a metabolic pathway involving Syk in humans or animals, wherein said condition is caused by type I hypersensitivity, or is rheumatoid arthritis, an autoimmune disease, inflammation or cancer.

14. Composition for its use according to claim 13 wherein said composition also comprises a further therapeutic molecule.

15. Method for identifying an organic molecule having a molecular weight between 50 and 2500 Dalton binding with Syk tyrosine kinase protein and capable of inhibiting by at least 10% *in vitro* binding of
(i) antibody fragment G4G11 (SEQ ID No. 2), or
(ii) an antibody or antibody fragment which binds with human Syk tyrosine kinase protein on an epitope comprising at least one of residues 65 to 74 of the amino acid sequence of human Syk tyrosine kinase protein represented by the sequence SEQ ID No. 1, or
(iii) an antibody or antibody fragment which binds with human Syk tyrosine kinase protein and inhibiting by at least 10% the binding of antibody fragment G4G11 with human Syk tyrosine kinase protein (SEQ ID No. 1),
with human Syk tyrosine kinase protein or with any of the variants thereof in animals, comprising at least the following steps:
a) screening, from a bank of candidate organic molecules having a molecular weight between 50 and 2500 Da, those liable to bind with Syk protein on the three-dimensional binding cavity on the Syk protein of a molecule selected from the molecules having formula C-13, I, II, III, IV or 1 to 87 as illustrated above;
b) selecting from the molecules identified in a) those capable of inhibiting by at least 10% *in vitro* the binding of the antibody or antibody fragment (i), (ii) or (iii) with Syk protein.

16. Method according to claim 15 wherein the molecule in step a) is the molecule **C-13**, molecule (**59**) or molecule (**61**).

17. Method according to claim 15 or 16 comprising an additional step prior to step a) for identifying the three-dimensional binding cavity on the Syk protein of the molecule selected from the molecules having the formula C-13, I, II, II, IV or 1 to 87, wherein said prior step is performed by means of *in silico* docking, and
an additional step c) for selecting from the molecules identified in b) those capable of inhibiting by 50% mast cell degranulation at a concentration (IC50) between 1 ng/ml and 1 mg/ml.


### LISTAGE DES SEQUENCES

<110> CENTRE NATIONAL DE LA RECHERCHE SCIENTIFIQUE
<120> Molécules inhibant une voie métabolique impliquant la protéine tyrosine
   kinase Syk et procédé d'identification de ces molécules
<130> B07504A
<140> PAS ENCORE CONNU
   <141> PAS ENCORE CONNUE
<150> FR 08/01959
   <151> 2008-04-09
<160> 4
<170> PatentIn version 3.1
<210> 1
   <211> 635
   <212> PRT
   <213> Homo sapiens
<400> 1
<210> 2
   <211> 269
   <212> PRT
   <213> Artificiel
<220>
   <223> fragment d'anticorps G4G11
<400> 2
   Met Ala Gln Val Gln Leu Gln Glu Ser Gly Pro Gly Leu Val Lys Pro
   1 5 10 15
   Ser Asp Thr Leu Ser Leu Thr Cys Ala Val Ser Gly Tyr Ser Ile Ser
   20 25 30
   Ser Ser Asn Trp Trp Gly Trp Ile Arg Gln Pro Pro Gly Lys Gly Leu
   35 40 45
   Glu Trp Ile Gly Tyr Ile Tyr His Ser Gly Ser Thr Tyr Tyr Asn Pro
   50 55 60
   Ser Leu Lys Ser Arg Val Thr Ile Ser Val Asp Lys Ser Lys Asn Gln
   65 70 75 80
   Phe Ser Leu Lys Leu Ser Ser Val Thr Ala Ala Asp Thr Ala Val Tyr
   85 90 95
   Tyr Cys Ala Arg Asn Val Gly Phe His Asp Trp Gly Gln Gly Thr Leu
   100 105 110
   Val Thr Val Ser Ser Gly Gly Gly Gly Ser Gly Gly Gly Gly Ser Gly
   115 120 125
   Gly Ser Ala Leu Gln Ser Val Leu Thr Gln Pro Pro Ser Ala Ser Gly
   130 135 140
   Thr Pro Gly Gln Arg Val Thr Ile Ser Cys Ser Gly Ser Ser Ser Asn
   145 150 155 160
   Ile Gly Ser Asn Tyr Val Tyr Trp Tyr Gln Gln Leu Pro Gly Thr Ala
   165 170 175
   Pro Lys Leu Leu Ile Tyr Arg Asn Asn Gln Arg Pro Ser Gly Val Pro
                   180                 185                 190
   Asp Arg Phe Ser Gly Ser Lys Ser Gly Thr Ser Ala Ser Leu Ala Ile
           195                 200                 205
   Ser Gly Leu Arg Ser Glu Asp Glu Ala Asp Tyr Tyr Cys Ala Ala Trp
       210                 215                 220
   Asp Asp Ser Leu Ala Ser Pro Val Phe Gly Gly Gly Thr Lys Leu Thr
   225                 230                 235                 240
   Val Leu Gly Ala Ala Ala His His His His His His Gly Ala Ala Glu
                   245                 250                 255
   Gln Lys Leu Ile Ser Glu Glu Asp Leu Asn Gly Ala Ala
               260                 265
<210> 3
   <211> 269
   <212> PRT
   <213> Artificiel
<220>
   <223> fragment d'anticoprs G4E4
<400> 3
   Met Ala Gln Val Gln Leu Gln Glu Ser Gly Pro Gly Leu Val Lys Pro
   1               5                   10                  15
   Ser Asp Thr Leu Ser Leu Thr Cys Ala Val Ser Gly Tyr Ser Ile Ser
               20                  25                  30
   Ser Ser Asn Trp Trp Gly Trp Ile Arg Gln Pro Pro Gly Lys Gly Leu
           35                  40                  45
   Glu Trp Ile Gly Tyr Ile Tyr His Ser Gly Ser Thr Tyr Tyr Asn Pro
       50                  55                  60
   Ser Leu Lys Ser Arg Val Thr Ile Ser Val Asp Lys Ser Lys Asn Gln
   65                  70                  75                  80
   Phe Ser Leu Lys Leu Ser Ser Val Thr Ala Ala Asp Thr Ala Val Tyr
                   85                  90                  95
   Tyr Cys Ala Arg Asn Val Gly Phe His Asp Trp Gly Gln Gly Thr Leu
               100                 105                 110
   Val Thr Val Ser Ser Gly Gly Gly Gly Ser Gly Gly Gly Gly Ser Gly
           115                 120                 125
   Gly Ser Ala Leu Gln Ser Val Leu Thr Gln Pro Pro Ser Ala Ser Gly
       130                 135                 140
   Thr Pro Gly Gln Arg Val Thr Ile Ser Cys Ser Gly Ser Ser Ser Asn
   145                 150                 155                 160
   Ile Gly Ser Asn Thr Val Asn Trp Tyr Gln Gln Leu Pro Gly Thr Ala
                   165                 170                 175
   Pro Lys Leu Leu Ile Tyr Ser Asn Asn Gln Arg Pro Ser Gly Val Pro
               180                 185                 190
   Asp Arg Phe Ser Gly Ser Lys Ser Gly Thr Ser Ala Ser Leu Ala Ile
           195                 200                 205
   Ser Gly Leu Gln Ser Glu Asp Glu Ala Asp Tyr Tyr Cys Ala Ala Trp
       210                 215                 220
   Asp Asp Ser Leu Phe Gly Ala Val Phe Gly Gly Gly Thr Lys Leu Thr
   225                 230                 235                 240
   Val Leu Gly Ala Ala Ala His His His His His His Gly Ala Ala Glu
                   245                 250                 255
   Gln Lys Leu Ile Ser Glu Glu Asp Leu Asn Gly Ala Ala
               260                 265
<210> 4
   <211> 629
   <212> PRT
   <213> Mus musculus
<400> 4
   Met Ala Gly Ser Ala Val Asp Ser Ala Asn His Leu Thr Tyr Phe Phe
   1               5                   10                  15
   Gly Asn Ile Thr Arg Glu Glu Ala Glu Asp Tyr Leu Val Gln Gly Gly
               20                  25                  30
   Met Thr Asp Gly Leu Tyr Leu Leu Arg Gln Ser Arg Asn Tyr Leu Gly
           35                  40                  45
   Gly Phe Ala Leu Ser Val Ala His Asn Arg Lys Ala His His Tyr Thr
       50                  55                  60
   Ile Glu Arg Glu Leu Asn Gly Thr Tyr Ala Ile Ser Gly Gly Arg Ala
   65                  70                  75                  80
   His Ala Ser Pro Ala Asp Leu Cys His Tyr His Ser Gln Glu Pro Asp
                   85                  90                  95
   Gly Leu Ile Cys Leu Leu Lys Lys Pro Phe Asn Arg Pro Pro Gly Val
               100                 105                 110
   Gln Pro Lys Thr Gly Pro Phe Glu Asp Leu Lys Glu Asn Leu Ile Arg
           115                 120                 125
   Glu Tyr Val Lys Gln Thr Trp Asn Leu Gln Gly Gln Ala Leu Glu Gln
       130                 135                 140
   Ala Ile Ile Ser Gln Lys Pro Gln Leu Glu Lys Leu Ile Ala Thr Thr
   145                 150                 155                 160
   Ala His Glu Lys Met Pro Trp Phe His Gly Asn Ile Ser Arg Asp Glu
                   165                 170                 175
   Ser Glu Gln Thr Val Leu Ile Gly Ser Lys Thr Asn Gly Lys Phe Leu
               180                 185                 190
   Ile Arg Ala Arg Asp Asn Ser Gly Ser Tyr Ala Leu Cys Leu Leu His
           195                 200                 205
   Glu Gly Lys Val Leu His Tyr Arg Ile Asp Arg Asp Lys Thr Gly Lys
       210                 215                 220
   Leu Ser Ile Pro Glu Gly Lys Lys Phe Asp Thr Leu Trp Gln Leu Val
   225                 230                 235                 240
   Glu His Tyr Ser Tyr Lys Pro Asp Gly Leu Leu Arg Val Leu Thr Val
                   245                 250                 255
   Pro Cys Gln Lys Ile Gly Ala Gln Met Gly His Pro Gly Ser Pro Asn
               260                 265                 270
   Ala His Pro Val Thr Trp Ser Pro Gly Gly Ile Ile Ser Arg Ile Lys
           275                 280                 285
   Ser Tyr Ser Phe Pro Lys Pro Gly His Lys Lys Pro Ala Pro Pro Gln
       290                 295                 300
   Gly Ser Arg Pro Glu Ser Thr Val Ser Phe Asn Pro Tyr Glu Pro Thr
   305                 310                 315                 320
   Gly Gly Pro Trp Gly Pro Asp Arg Gly Leu Gln Arg Glu Ala Leu Pro
                   325                 330                 335
   Met Asp Thr Glu Val Tyr Glu Ser Pro Tyr Ala Asp Pro Glu Glu Ile
               340                 345                 350
   Arg Pro Lys Glu Val Tyr Leu Asp Arg Ser Leu Leu Thr Leu Glu Asp
           355                 360                 365
   Asn Glu Leu Gly Ser Gly Asn Phe Gly Thr Val Lys Lys Gly Tyr Tyr
       370                 375                 380
   Gln Met Lys Lys Val Val Lys Thr Val Ala Val Lys Ile Leu Lys Asn
   385                 390                 395                 400
   Glu Ala Asn Asp Pro Ala Leu Lys Asp Glu Leu Leu Ala Glu Ala Asn
                   405                 410                 415
   Val Met Gln Gln Leu Asp Asn Pro Tyr Ile Val Arg Met Ile Gly Ile
               420                 425                 430
   Cys Glu Ala Glu Ser Trp Met Leu Val Met Glu Met Ala Glu Leu Gly
           435                 440                 445
   Pro Leu Asn Lys Tyr Leu Gln Gln Asn Arg His Ile Lys Asp Lys Asn
       450                 455                 460
   Ile Ile Glu Leu Val His Gln Val Ser Met Gly Met Lys Tyr Leu Glu
   465                 470                 475                 480
   Glu Ser Asn Phe Val His Arg Asp Leu Ala Ala Arg Asn Val Leu Leu
                   485                 490                 495
   Val Thr Gln His Tyr Ala Lys Ile Ser Asp Phe Gly Leu Ser Lys Ala
               500                 505                 510
   Leu Arg Ala Asp Glu Asn Tyr Tyr Lys Ala Gln Thr His Gly Lys Trp
           515                 520                 525
   Pro Val Lys Trp Tyr Ala Pro Glu Cys Ile Asn Tyr Tyr Lys Phe Ser
       530                 535                 540
   Ser Lys Ser Asp Val Trp Ser Phe Gly Val Leu Met Trp Glu Ala Phe
   545 550 555 560
   Ser Tyr Gly Gln Lys Pro Tyr Arg Gly Met Lys Gly Ser Glu Val Thr
   565 570 575
   Ala Met Leu Glu Lys Gly Glu Arg Met Gly Cys Pro Ala Gly Cys Pro
   580 585 590
   Arg Glu Met Tyr Asp Leu Met Asn Leu Cys Trp Thr Tyr Asp Val Glu
   595 600 605
   Asn Arg Pro Gly Phe Thr Ala Val Glu Leu Arg Leu Arg Asn Tyr Tyr
   610 615 620
   Tyr Asp Val Val Asn
   625